# EUROPÄISCHE PATENTSCHRIFT

(11) **EP 1 741 000 B1**
(45) Veröffentlichungstag und Bekanntmachung des Hinweises auf die Patenterteilung: **26.12.2012**
(21) Anmeldenummer: 05730277.0
(22) Anmeldetag: 12.04.2005
(51) Int. Cl.: C09K 19/32, C09K 19/34, G02F 1/13

(54) **AS-INDACENDERIVATE MIT NEGATIVER DIELEKTRISCHER ANISOTROPIE**
AS-INDACENE DERIVATIVES HAVING NEGATIVE DIELECTRIC ANISOTROPY
DERIVES DE L'AS-INDACENE AYANT UNE ANISOTROPIE DIELECTRIQUE NÉGATIVE

(30) Priorität: 26.04.2004 DE 102004020249
(43) Veröffentlichungstag der Anmeldung: 10.01.2007
(73) Patentinhaber: Merck Patent GmbH, 64293 Darmstadt (DE)
(72) Erfinder: LIETZAU, Lars, 64295 Darmstadt (DE); BREMER, Matthias, 64295 Darmstadt (DE); KLASEN-MEMMER, Melanie, 67259 Heuchelheim (DE)
(86) Internationale Anmeldenummer: PCT/EP2005/003840
(87) Internationale Veröffentlichungsnummer: WO 2005/103799

(56) Entgegenhaltungen:
- EP-A- 0 305 938
- EP-A- 1 350 780
- WO-A-03/010120
- GB-A- 1 170 672
- US-A- 3 931 239
- SEPIOL, J.; MIREK, J.: "Elimination of the nitrile group from carbocyclic o-hydroxynitriles" SYNTHESIS, 1979, Seiten 290-292, XP002338226
- KOCIOLEK M G ET AL: "Intramolecular Thermal Cyclotrimerization of an Acyclic Triyne: An Uncatalyzed Process" TETRAHEDRON LETTERS, ELSEVIER SCIENCE PUBLISHERS, AMSTERDAM, NL, Bd. 40, Nr. 22, 28. Mai 1999 (1999-05-28), Seiten 4141-4144, XP004164681 ISSN: 0040-4039
- KATZ, T.J. ET AL.: "Synthesis of Bis(as-indacenyliron)" J. AM. CHEM. SOC., Bd. 90, Nr. 3, 1968, Seiten 734-739, XP002338227
- EISENBRAUN E J ET AL: "Polyalkyl Aromatic Hydrocarbons. II. Cyclialkylation of Benzoid Hydrocarbons with Isoprene" JOURNAL OF ORGANIC CHEMISTRY, AMERICAN CHEMICAL SOCIETY. EASTON, US, Bd. 33, 1968, Seiten 2000-2008, XP002242443 ISSN: 0022-3263
- BISELLO A ET AL: "Synthesis, properties and reactivity of mononuclear Rh(I) and Ir(I) complexes of s- and as-hydroindacenide ligands" JOURNAL OF ORGANOMETALLIC CHEMISTRY, ELSEVIER-SEQUOIA S.A. LAUSANNE, CH, Bd. 593-594, 2000, Seiten 315-324, XP004185713 ISSN: 0022-328X
- WISNIEWSKI GRISSOM, J. ET AL.: "High temperature radical cyclization anomalies in the tandem enediyne-Bis-radical cyclization" TETRAHEDRON, Bd. 50, Nr. 16, 1994, Seiten 4635-4650, XP002338228
- BAO, J. ET AL.: "Three-component intramolecular two-alkyne annulations of Fischer carbene complexes: New strategies for steroid synthesis" J. AM. CHEM. SOC., Bd. 116, Nr. 17, 1994, Seiten 7616-7630, XP002338229
- ARNOLD, R.T. ET AL.: "Steric effect of methylene groups. II" J. AM. CHEM. SOC., Bd. 68, Nr. 11, 1946, Seiten 2176-2178, XP002338230
- SEPIOL, J.: "Cyclization of ethyl ylidenecyanoacetates to carbocyclic o-aminoesters. An efficient synthesis of 1,2,3,6,7,8-hexahydro-as-indacene derivatives" TETRAHEDRON, Bd. 42, Nr. 2, 1986, Seiten 609-616, XP002338231
- KELLY, T.R. ET AL.: "A chiral catechol with C2 symmetry" J. ORG. CHEM., Bd. 54, Nr. 4, 1989, Seiten 980-983, XP002338232
- ZHANG Y ET AL: "Selective Remote Functionalization of Alkyl Side Chains in the Coupling of Fischer Carbene Complexes with Conjugated Enediynes" TETRAHEDRON, ELSEVIER SCIENCE PUBLISHERS, AMSTERDAM, NL, Bd. 56, Nr. 15, 2000, Seiten 2175-2182, XP004195553 ISSN: 0040-4020
- VEJDELEK, Z.J. ET AL.: "4-(Aminomethyl)-s-hydrindacenes and 4,8-Bis(aminomethyl)-s-hydrindacenes; synthesis and pharmacological screening" COLLECT. CZECH. CHEM. COMMUN., Bd. 42, 1977, Seiten 3094-3103, XP009051401
- COURTOT, P. ET AL.: "N° 363. - Synthèse et étude de quelques propriétés chimiques de dérivés de l'octahydro-as-indacène et du décahydrophénanthrène" BULL. SOC. CHIM. FR., 1973, Seiten 2121-2125, XP009051402
- LE GUILLANTON, G.: "N° 120. - Utilisation de la cyclopentylidène-2 cyclopentanone à la préparation de dérivés décahydro as-indacéniques et hexahydro as-indacéniques. III. - Préparation de dérivés hexahydro-1,2,3,6,7,8 as-indacéniques" BULL. SOC. CHIM. FR., 1963, Seiten 630-638, XP009051403
- MELMER, M. ET AL.: "Aromatische Spirane, 22. Mitt.: Darstellung von Cyclopenteno-4,5-indan-1-on und 2-Carboxymethyl-bzw. 4-Chlormethyl-indan als Synthone für Synthesen von anellierten 2,2'-Spirobiindanonen" MONATSHEFTE FÜR CHEMIE, Bd. 127, Nr. 3, 1996, Seiten 275-290, XP009051348
- RENÉ, L. ET AL.: "Analogues difurobenzéniques et furochroméniques de l'angélicine et autres furocoumarines angulaires" EUR. J. MED. CHEM., Bd. 13, Nr. 5, 1978, Seiten 435-439, XP009051347
- RODRIGUES D.C. ET AL: "Predicting the Claisen rearrangement regioselectivity of allylindanyl and allyltetrahydronaphthalenyl ether derivatives by 1H NMR experiments", MAGNETIC RESONANCE IN CHEMISTRY, vol. 38, no. 11, 2000, pages 970-974, XP009104837,

## Beschreibung

Die vorliegende Erfindung betrifft as-Indacenderivate, flüssigkristalline Medien enthaltend diese Derivate sowie elektrooptische Anzeigeelemente enthaltend diese flüssigkristallinen Medien. Insbesondere betrifft die Erfindung as-Indacenderivate mit negativer dielektrischer Anisotropie.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete sind insbesondere Anzeigedisplays für Uhren und Taschenrechner sowie große Anzeigetafeln, wie sie in Bahnhöfen, Flughäfen und Sportarenen verwendet werden. Weitere Anwendungsgebiete sind Displays von tragbaren Computern und Navigationssystemen sowie Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, daß viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigkristallinen Mediums ist also für die beiden Orientierungen verschieden groß. Substanzen, deren Dielektrizitätskonstante bei senkrechter Orientierung der Molekül-Längsachsen zu den Kondensatorplatten größer ist als bei paralleler Anordnung, werden als dielektrisch positiv bezeichnet. Mit anderen Worten: Ist die Dielektrizitätskonstante ε_{∥} parallel zu den Moleküllängsachsen größer als die Dielektrizitätskonstante ε_{⊥} senkrecht zu den Moleküllängsachsen, so ist die dielektrische Anisotropie Δε = ε_{∥} - ε_{⊥} größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, daß die größere der dielektrischen Konstanten wirksam wird. Die Stärke der Wechselwirkung mit dem elektrischen Feld hängt dabei von der Differenz der beiden Konstanten ab. Bei kleinen Differenzen sind höhere Schaltspannungen erforderlich als bei großen. Durch den Einbau geeigneter polarer Gruppen, wie z.B. von Nitrilgruppen oder Fluor, in die Flüssigkristallmoleküle läßt sich ein weiter Bereich von Arbeitsspannungen realisieren.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment. Bei den am weitesten verbreiteten TN-Zellen (abgeleitet aus dem Englischen: "twisted nematic", verdrillt nematisch) ist eine nur etwa 5 bis 10 µm dicke flüssigkristalline Schicht zwischen zwei planparallelen Glasplatten angeordnet, auf die jeweils eine elektrisch leitende, transparente Schicht aus Zinnoxid oder Indium-Zinnoxid (ITO) als Elektrode aufgedampft ist. Zwischen diesen Filmen und der flüssigkristallinen Schicht befindet sich eine ebenfalls transparente Orientierungsschicht, die meist aus einem Kunststoff (z.B. Polyimiden) besteht. Sie dient dazu, durch Oberflächenkräfte die Längsachsen der benachbarten flüssigkristallinen Moleküle in eine Vorzugsrichtung zu bringen, so daß sie im spannungsfreien Zustand einheitlich mit der gleichen Orientierung flach oder mit demselben kleinen Anstellwinkel (englisch: "tilt angle") auf der Innenseite der Displayfläche aufliegen. Auf der Außenseite des Displays sind zwei Polarisationsfolien, die nur linear polarisiertes Licht ein- und austreten lassen, in einer bestimmten Anordnung aufgebracht.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen zu erreichen. Schwierigkeiten bereitet jedoch noch die starke Blickwinkelabhängigkeit bei Flüssigkristallanzeigen, wie sie beispielsweise für Laptops verwendet werden. Die beste Abbildungsqualität läßt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters ohne wesentliche Minderung der Abbildungsqualität verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie Δε ist negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Durch Realisierung mehrer Domänen konnte unter Verwendung von flüssigkristallinen Medien mit negativer dielektrischer Anisotropie eine Verbesserung der Blickwinkelabhängigkeit erreicht werden. Auch können mit dieser Technologie kürzere Schaltzeiten in Displays und bessere Kontraste erzielt werden. Derartige Displays werden als VA-TFT-Displays bezeichnet (abgeleitet aus dem Englischen: "vertically aligned").

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

In der DE 25 14 389 A werden unter anderem in 7-Position substituierte 6-Oxo-6H-indeno[5,4-b]furancarbonsäuren und ihre Verwendung als Arzneimittel offenbart; weder mesogene Eigenschaften noch der Einsatz in flüssigkristallinen Medien dieser Verbindungen werden beschrieben.

In J. Heterocycl. Chem., 15 (1978) 43-48, werden 2,7-Dimethylbenzo[1,2-b:4,3-b']difuran und 4-Chlor-2,7-dimethylbenzo[1,2-b:4,3-b']difuran offenbart; weder mesogene Eigenschaften noch der Einsatz in flüssigkristallinen Medien dieser Verbindungen werden beschrieben.

In der EP 1 350 780 A1 werden allgemein 1,7-Dihydroindacenderivate mit negativer dielektrischer Anisotropie beschrieben, ohne daß physikalische oder elektrooptische Eigenschaften präzisiert werden. Gleichermaßen werden in der WO 03/010120 A 2,6-substituierte 1,2,3,5,6,7-Hexahydro-s-indacene offenbart.

Eine Aufgabe der vorliegenden Erfindung ist es, Verbindungen mit vorteilhaften Eigenschaften für den Einsatz in flüssigkristallinen Medien zur Verfügung zu stellen. Insbesondere sollten sie über eine negative dielektrische Anisotropie verfügen, was sie besonders geeignet macht für den Einsatz in flüssigkristallinen Medien für VA-Displays.

Diese Aufgabe wird erfindungsgemäß gelöst durch Verbindungen der allgemeinen Formel I 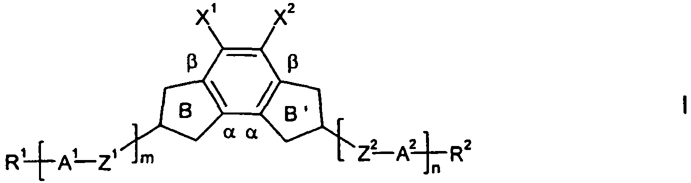 worin: 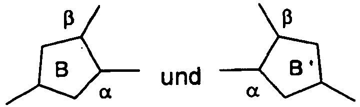 unabhängig voneinander für 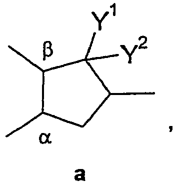 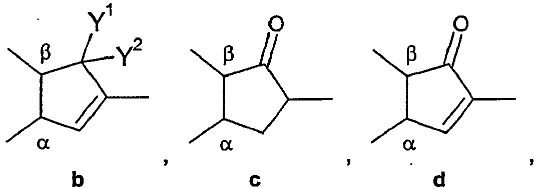 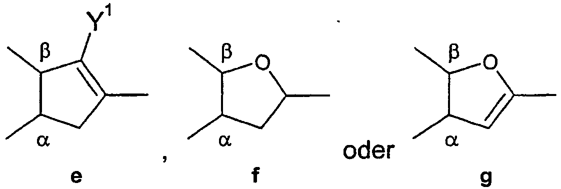 stehen;
- A¹ und A²: jeweils unabhängig voneinander 1 ,4-Phenylen, worin =CH- ein- oder zweimal durch =N- ersetzt sein kann und das unsubstituiert oder ein- bis viermal unabhängig voneinander mit -CN, F, Cl, Br und/oder I, unsubstituiertem oder mit Fluor und/oder Chlor ein- oder mehrfach substituiertem C₁-C₆-Alkanyl, unsubstituiertem oder mit Fluor und/oder Chlor ein- oder mehrfach substituiertem C₁-C₆-Alkoxy substituiert sein kann, 1,4-Cyclohexylen, 1,4-Cyclohexenylen oder 1,4-Cyclohexadienylen, worin -CH₂- ein- oder zweimal unabhängig voneinander durch -O- oder -S- so ersetzt sein kann, daß Heteroatome nicht direkt verknüpft sind, und die unsubstituiert oder ein- oder mehrfach durch F, Cl, Br und/oder I substituiert sein können, bedeuten;
- Z¹ und Z²: jeweils unabhängig voneinander eine Einfachbindung, eine Doppelbindung, -CF₂O-, -OCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CF=CH-, -CH=CF-, -CF=CF-, -CH=CH- oder -C≡Cbedeuten;
- R¹ und R²: Wasserstoff, einen unsubstituierten, einen einfach mit -CN oder -CF₃ oder einen einfach oder mehrfach mit F, Cl, Br und/oder I substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂- oder -OCO-O-so ersetzt sein können, daß Heteroatome in der Kette nicht direkt verknüpft sind, F, Cl, Br, I, -CN, -SCN, -NCS oder -SF₅ bedeuten;
- m und n: unabhängig voneinander 0, 1, 2 oder 3 sind;
- Y²: Wasserstoff, einen unsubstituierten oder einfach oder mehrfach durch F, Cl, Br und/oder I substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂-, -CO-, -COO-, -OCO- oder -OCO-O- so ersetzt sein können, daß Heteroatome in der Kette nicht direkt verknüpft sind, F, Cl, Br, I, -CN, -SF₅, -SCN oder -NCS bedeuten;
- Y¹: wie Y² definiert ist oder für -[-Z³-A³-]ₚ-R³ steht, wobei Z³ wie Z¹ und Z², A³ wie A¹ und A², R³ wie R¹ und R² sowie p wie n und m definiert sind;
- X¹ und X²: jeweils unabhängig voneinander -OCF₃, -CF₃-, F oder Cl bedeuten,
wobei
- A¹, A², A³; Z¹, Z², Z³; R¹, R², R³: jeweils die gleiche oder unterschiedliche Bedeutungen haben können, wenn m, n beziehungsweise p größer als 1 sind;
- Y¹ und Y²: für die Ringe B und B' jeweils die gleichen oder verschiedene Bedeutungen haben können.

Die Verbindungen besitzen ein negatives Δε und eignen sich daher insbesondere für eine Verwendung in VA-TFT-Displays. Vorzugsweise besitzen die erfindungsgemäßen Verbindungen ein Δε < -2 und besonders bevorzugt ein Δε < -5 . Sie zeigen eine sehr gute Verträglichkeit mit den üblichen, in Flüssigkristallmischungen für Displays verwendeten Substanzen.

Ferner weisen die erfindungsgemäßen Verbindungen der Formel I insbesondere für die Verwendung in VA-TFT-Displays geeignete Werte der optischen Anisotropie Δn auf. Bevorzugt besitzen die erfindungsgemäßen Verbindungen ein Δn von größer als 0,02 und kleiner als 0,25.

Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die Verbindungen weisen insbesondere eine ausreichende Breite der nematischen Phase und eine gute Tieftemperatur- und Langzeitstabilität sowie ausreichend hohe Klärpunkte und gute Viskositäten und Schaltzeiten auf.

Ferner ist es bevorzugt, daß wenigstens einer der Ringe B und B' wenigstens einen elektronegativen Rest (z.B. -F, -Cl oder =O oder mit Halogen substituierte Alkylreste) aufweist. Besonders bevorzugt weisen beide Ringe B und B' elektronegative Reste auf.

Durch die Reste X¹ und X², nämlich -CF₃, -OCF₃, Fluor- und/oder Chlorsubstituenten, insbesondere Fluorsubstituenten, am zentralen aromatischen Sechsring sowie die vorzugsweise elektronegativen Reste am Ring B beziehungsweise B' wird ein Dipolmoment senkrecht zur Moleküllängsachse erzeugt, das gegebenenfalls durch geeignete Substituenten in den Flügeleinheiten -(Z¹-A¹-)ₘ-R¹ und -(Z²-A²-)ₙ-R² weiter verstärkt werden kann. Im feldfreien Zustand richten sich die Verbindungen der Formel I mit ihrer Moleküllängsachse senkrecht zur behandelten oder beschichteten Glasfläche des Displays aus.

Bevorzugte erfindungsgemäße Verbindungen der Formel I sind solche, in denen die Ringe B und B' jeweils unabhängig voneinander ausgewählt sind aus 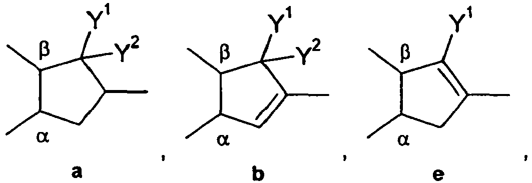 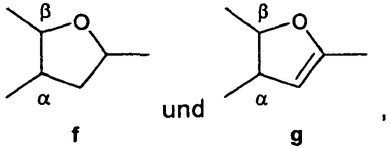 wobei Y¹ und Y² wie für Formel I oben definiert sind. Dabei ist es besonders bevorzugt, daß die Ringe B und B' gleich sind, wobei der Rest Y¹ in beiden Ringen die gleiche oder unterschiedliche Bedeutungen und der Rest Y² in beiden Ringen gleiche oder unterschiedliche Bedeutungen haben können.

Besonders bevorzugt sind Y¹ und Y² unabhängig voneinander F, Cl oder ein (per-)fluorierter Alkanyl- oder Alkoxyrest mit bis zu 6 Kohlenstoffatomen, insbesondere -CF₃ oder -OCF₃. Ferner ist Y¹ besonders bevorzugt auch -[-Z³-A³-]p-R³. Ganz besonders bevorzugt sind Y¹ und Y² in mindestens einem der Ringe B und B' F, insbesondere in beiden Ringen.

Ferner ist bevorzugt, daß die erfindungsgemäßen Verbindungen der Formel I ausgewählt sind aus der Gruppe von Verbindungen der folgenden Formeln: 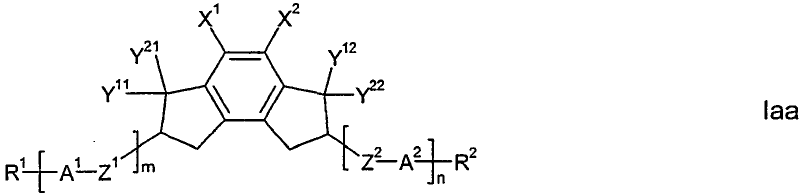 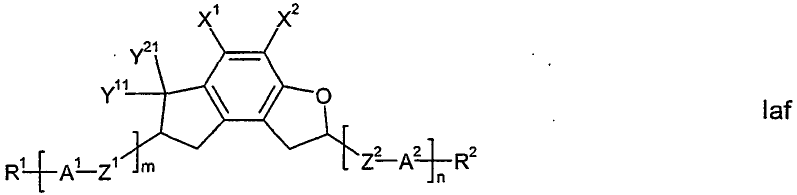 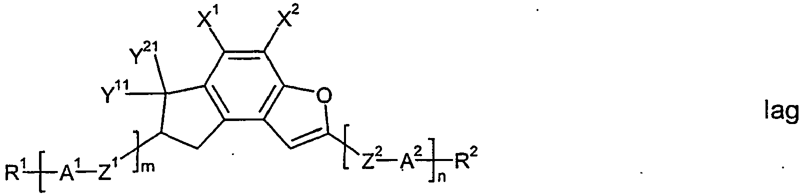 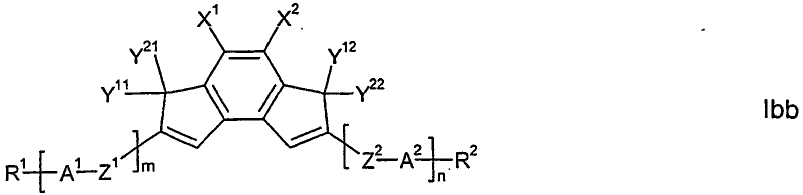 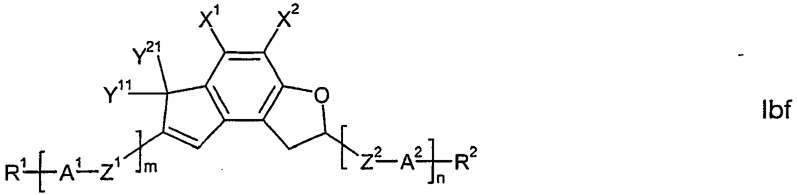 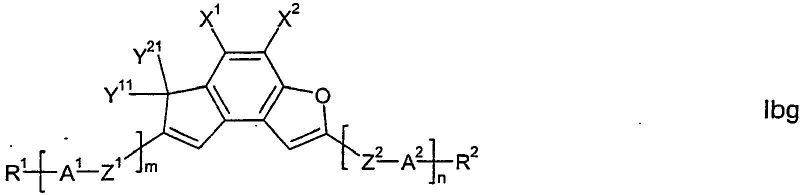 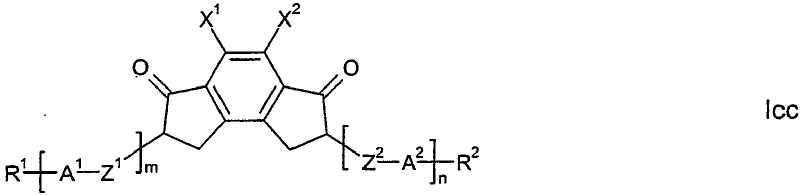 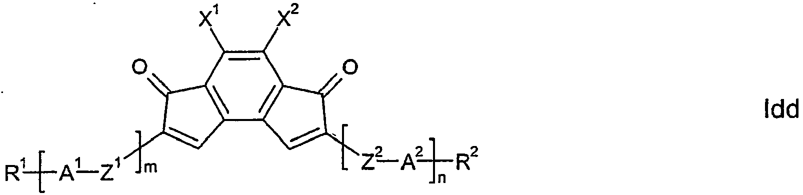 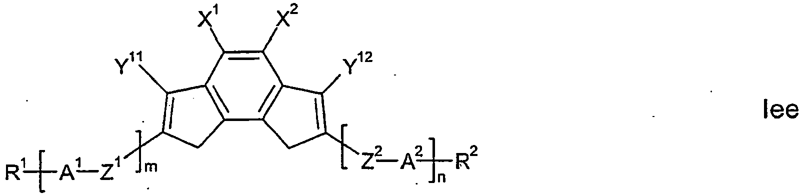 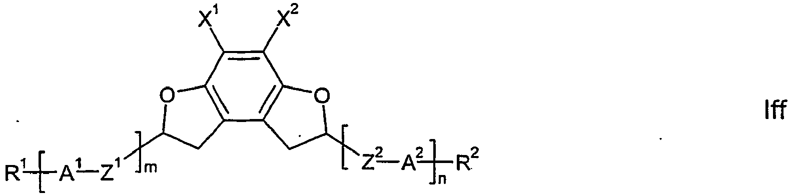 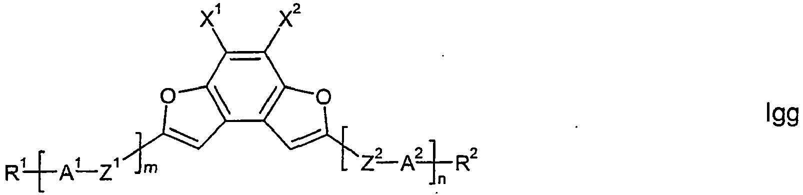 wobei
- X¹, X², R¹, R², A¹, A², Z¹, Z², m und n: wie in Anspruch 1 und wie für Formel I oben definiert sind;
- Y¹¹ und Y¹²: wie Y¹ in Anspruch 1 und wie für Formel I oben definiert sind und die gleiche oder verschiedene Bedeutungen haben können; und
- Y²¹ und Y²²: wie Y² in Anspruch 1 und wie für Formel I oben definiert sind und die gleiche oder verschiedene Bedeutungen haben können.

Besonders bevorzugt sind Y¹¹, Y¹², Y²¹ und Y²² unabhängig voneinander H, F, Cl oder ein (per-)fluorierter Alkanyl- oder Alkoxyrest mit bis zu 6 Kohlenstoffatomen, insbesondere -CF₃ oder -OCF₃. Ferner stehen Y¹¹ beziehungsweise Y¹² besonders bevorzugt auch für -[-Z³-A³-]ₚ-R³. Ganz besonders bevorzugt sind Y¹¹ und Y²¹ beziehungsweise Y¹² und Y²² gleich und insbesondere Fluor. Insbesondere sind Y¹¹, Y²¹, Y¹² und Y²² alle zugleich Fluor.

Besonders bevorzugt sind Verbindungen der Formeln laa, Ibb, lee, Iff und Igg.

In den erfindungsgemäßen Verbindungen der allgemeinen Formel I und der Formeln laa, laf, lag, Ibb, Ibf, Ibg, Icc, Idd, lee, Iff und Igg stehen Z¹ und Z² sowie - falls vorhanden - Z³ bevorzugt unabhängig voneinander für eine Einfachbindung, -CF₂O-, -OCF₂-,-CF₂CF₂-, -CH=CH-, -CF=CH-, -CH=CF- oder -CF=CF-, besonders bevorzugt für eine Einfachbindung, -CF₂O- oder -OCF₂-, insbesondere eine Einfachbindung.

In den erfindungsgemäßen Verbindungen der Formel I und der Formeln laa, laf, lag, Ibb, Ibf, Ibg, Icc, Idd, lee, Iff und Igg sind A¹, A² und - sofern vorhanden - A³ bevorzugt unabhängig voneinander gegebenenfalls substituiertes 1,4-Phenylen, gegebenenfalls substituiertes 1,4-Cyclohexylen, worin -CH₂- ein- oder zweimal durch -O- ersetzt sein kann, oder gegebenenfalls substituiertes 1,4-Cyclohexenylen. Bei m > 1 können die Ringe A¹ die gleichen oder unterschiedliche Bedeutungen annehmen. Bei n > 1 können die Ringe A² die gleichen oder unterschiedliche Bedeutungen annehmen. Bei p > 1 können die Ringe A³ die gleichen oder unterschiedliche Bedeutungen annehmen.

Besonders bevorzugt sind A¹, A² und - sofern vorhanden - A³ unabhängig voneinander

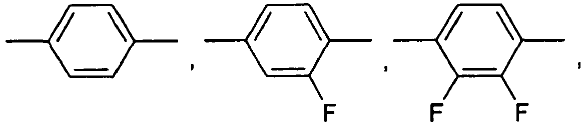

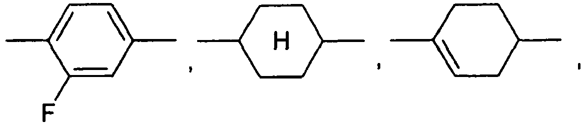

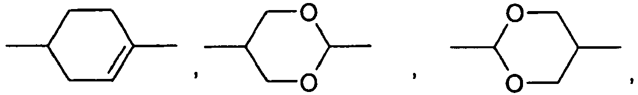

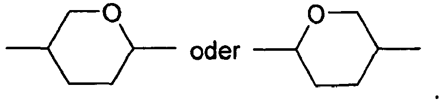

Ganz besonders bevorzugt sind A¹, A² und - sofern vorhanden - A³ 1,4-Cyclohexylenringe und/oder gegebenenfalls mit Fluor substituierte 1,4-Phenylenringe.

R¹, R², R³, Y¹ und Y² in Formel I können jeweils unabhängig voneinander ein Alkanylrest und/oder ein Alkoxyrest (Alkyloxyrest) mit 1 bis 15 C-Atomen sein, der geradkettig oder verzweigt ist. Vorzugsweise ist er geradkettig, hat 1, 2, 3, 4, 5, 6 oder 7 C-Atome und ist demnach vorzugsweise Methyl, Ethyl, Propyl, Butyl, Pentyl, Hexyl, Heptyl, Methoxy, Ethoxy, Propoxy, Butoxy, Pentoxy, Hexoxy oder Heptoxy.

R¹, R², R³, Y¹ und Y² in Formel I können jeweils unabhängig voneinander Oxaalkyl - d.h. eine der nichtterminalen CH₂-Gruppen des Alkanylrests ist durch -O- ersetzt - sein, vorzugsweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4- 5- oder 6-Oxaheptyl. In entsprechender Weise können R¹, R², R³, Y¹ und Y² in Formel I auch unabhängig voneinander Thioalkanyl- bzw. Sulfonalkanylreste sein, d.h. Alkanylreste, in denen eine CH₂-Gruppe durch -S- beziehungsweise -SO₂- ersetzt ist.

R¹, R², R³, Y¹ und Y² in Formel I können ferner jeweils unabhängig voneinander ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im allgemeinen sind die jeweiligen E-Isomeren bevorzugt.

R¹, R², R³, Y¹ und Y² in Formel I können unabhängig voneinander auch ein Alkinylrest mit 2 bis 15 C-Atomen sein, der geradkettig oder verzweigt ist und wenigstens eine C-C-Dreifachbindung aufweist.

R¹, R², R³, Y¹ und Y² in Formel I können jeweils unabhängig voneinander ein Alkanylrest mit 1 bis 15 C-Atomen sein, in dem eine CH₂-Gruppe durch -O- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser Rest geradkettig und hat 2 bis 6 C-Atome. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxy-methyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl oder 4-(Methoxycarbonyl)butyl. Ferner kann ein Alkanylrest auch eine -O-CO-O-Einheit aufweisen. Auch der Ersatz einer CH₂-Gruppe durch lediglich eine -CO-Gruppe (Carbonylfunktion) ist möglich.

Y¹ und Y² in Formel I können jeweils unabhängig voneinander ein Alkenylrest mit 2 bis 15 C-Atomen sein, in dem eine CH₂-Gruppe in Nachbarschaft zu einer unsubstituierten oder substituierten -C=C-Einheit eine CH₂-Gruppe durch -CO- oder -CO-O- oder -O-CO- ersetzt ist, wobei dieser Rest geradkettig oder verzweigt sein kann. Vorzugsweise ist der Rest geradkettig und hat 4 bis 13 C-Atome. Besonders bevorzugt sind dabei Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl oder 8-Methacryloyloxyoctyl. Entsprechend kann auch in einem Alkinylrest in Nachbarschaft zu einer substituierten -C≡C-Einheit eine CH₂-Gruppe durch -CO-, -CO-O-, -O-CO- oder -O-CO-O- ersetzt sein.

R¹, R² und R³ in Formel I können jeweils unabhängig voneinander ein einfach durch -CN oder -CF₃ substituierter Alkanylrest mit 1 bis 15 C-Atomen oder Alkenylrest mit 2 bis 15 C-Atomen sein, wobei diese vorzugsweise geradkettig sind. Die Substitution durch -CN oder -CF₃ ist in beliebiger Position möglich.

R¹, R², R³, Y¹ und Y² in Formel I können jeweils unabhängig voneinander ein Alkanylrest sein, in dem zwei oder mehr CH₂-Gruppen durch -O- und/oder -CO-O- ersetzt sind, wobei dieser geradkettig oder verzweigt sein kann. Vorzugsweise ist er verzweigt und hat 3 bis 12 C-Atome.

R¹, R², R³, Y¹ und Y² in Formel I können jeweils unabhängig voneinander ein einfach oder mehrfach mit F, Cl, Br und/oder I substituierter Alkanylrest mit 1 bis 15 C-Atomen oder Alkenylrest mit 2 bis 15 C-Atomen sein, wobei diese Reste vorzugsweise geradkettig sind und Halogen vorzugsweise-F und/oder -Cl ist. Bei Mehrfachsubstitution ist Halogen vorzugsweise -F. Die resultierenden Reste schließen auch perfluorierte Reste wie -CF₃ ein. Bei Einfachsubstitution kann der Fluor- oder Chlorsubstituent in beliebiger Position sein, vorzugsweise ist er in ω-Position.

R¹, R², R³, Y¹ und Y² in Formel I können auch jeweils unabhängig voneinander -F, -Cl, -Br, -I, -CN, -SCN, -NCS oder -SF₅ sein.

Besonders bevorzugt sind R¹, R² und R³ unabhängig voneinander in der allgemeinen Formel I ein Alkanylrest, Alkoxyrest oder Alkenylrest mit 1 bis 7 bzw. 2 bis 7 C-Atomen.

Bevorzugt sind X¹ und X² beide Fluor.

Bevorzugte Verbindungen der allgemeinen Formel I weisen insgesamt keine, eine, zwei oder drei Flügeleinheiten -Z¹-A¹- und -Z²-A²- auf, das heißt n+m = 0, 1, 2 oder 3 mit n und m jeweils 0, 1, 2 oder 3. Sind zwei oder drei Flügeleinheiten vorhanden, können sie an nur eine Molekülseite, d.h. entweder an Ring B oder an Ring B', oder auch an beide Molekülseiten gebunden sein. Besonders bevorzugt ist n+m = 0 oder 1.

Halogen bedeutet im Zusammenhang der vorliegenden Erfindung Fluor, Chlor, Brom und Iod.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen.

Falls in einem Alkylrest eine oder mehrere CH₂-Gruppen durch -O- ersetzt sein können, umfaßt der Ausdruck "Alkyl" auch "Alkoxy"- beziehungsweise "Oxaalkyl"-Reste. Unter Alkoxy ist im Zusammenhang mit der vorliegenden Erfindung - sofern der Ausdruck nicht an anderer Stelle in der Beschreibung oder in den Ansprüchen nicht abweichend definiert ist - ein O-Alkyl-Rest zu verstehen, in dem das Sauerstoffatom direkt mit der durch den Alkoxyrest substituierten Gruppe oder dem substituierten Ring verbunden ist und Alkyl wie oben definiert ist

Bevorzugte Alkoxyreste sind Methoxy, Ethoxy, Propoxy, Butoxy, Pentaxy, Hexoxy, Heptoxy und Octoxy. Besonders bevorzugt ist Alkoxy -OCH₃, - OC₂H₅, -O-n-C₃H₇, -O-n-C₄H₉, oder -O-t-C₄H₉. Im Zusammenhang mit der vorliegenden Erfindung bedeutet der Ausdruck "Oxaalkyl" - sofern der Ausdruck nicht an anderer Stelle in der Beschreibung oder in den Ansprüchen nicht abweichend definiert ist - Alkylreste, in denen wenigstens eine nicht-terminale CH₂-Gruppe durch -O- derart ersetzt ist, daß keine benachbarten Heteroatome (O, S) vorliegen. Vorzugsweise umfaßt Oxaalkyl geradkettige Reste mit 1, 2, 3, 4, 5, 6, 7, 8, 9 oder 10 Kohlenstoffatomen.

Im Zusammenhang der vorliegenden Erfindung bedeutet der Ausdruck "Alkenyl" - sofern er nicht in den Ansprüchen oder an anderer Stelle in dieser Beschreibung abweichend definiert ist - einen wie oben definierten Alkylrest, in dem eine oder mehrere -CH=CH-Gruppen vorhanden sind. Sofern zwei -CH=CH-Gruppen in dem Rest vorhanden sind, kann dieser auch als "Alkadienyl" bezeichnet werden. Ein Alkenylrest kann 2 bis 15 (d.h. 2, 3, 4, 5, 6, 7, 8, 9, 10, 11, 12, 13, 14 oder 15) Kohlenstoffatome enthalten und ist verzweigtkettig oder vorzugsweise geradkettig. Falls die CH=CH-Gruppe an beiden Kohlenstoffatomen einen anderen Rest als Wasserstoff trägt, etwa wenn sie eine nicht-terminale Gruppe ist, kann die CH=CH-Gruppe in zwei Konfigurationen vorliegen, nämlich als E-Isomer und als Z-Isomer. Im allgemeinen ist das E-Isomer (trans) bevorzugt. Vorzugsweise enthält der Alkenylrest 2, 3, 4, 5, 6 oder 7 Kohlenstoffatome und bedeutet Vinyl, 1 E-Propenyl, 1 E-Butenyl, 1 E-Pentenyl, 1 E-Hexenyl, 1 E-Heptenyl, 2-Propenyl, 2E-Butenyl, 2E-Pentenyl, 2E-Hexenyl, 2E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl.

Falls in einem Alkylrest eine oder mehrere CH₂-Gruppen durch -C≡Cersetzt sind, liegt ein Alkinylrest vor. Auch die Ersetzung von einer oder mehreren CH₂-Gruppen durch -CO-O- oder -O-CO- ist möglich. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)propyl, 3-(Ethoxy-carbonyl)-propyl oder 4-(Methoxycarbonyl)butyl.

Falls in einem Alkylrest eine CH₂-Gruppe durch unsubstituiertes oder substituiertes -CH=CH- und eine benachbarte CH₂-Gruppe durch CO, CO-O oder O-CO- ersetzt sind, so kann dieser Rest geradkettig oder verzweigt sein. Vorzugsweise ist er geradkettig und hat 4 bis 12 C-Atome. Er bedeutet demnach besonders bevorzugt Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl oder 8-Methacryloyloxyoctyl.

Sofern Reste oder Substituenten der erfindungsgemäßen Verbindungen beziehungsweise die erfindungsgemäßen Verbindungen selbst als optisch aktive oder stereoisomere Reste, Substituenten beziehungsweise Verbindungen vorliegen können, weil sie beispielsweise ein asymmetrisches Zentrum aufweisen, so sind diese von der vorliegenden Erfindung mit umfaßt. Dabei ist es selbstverständlich, daß die erfindungsgemäßen Verbindungen der allgemeinen Formel I in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Die Verbindungen der allgemeinen Formel 1 werden nach an sich bekannten Methoden dargestellt, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch in situ gebildet werden, derart, daß man sie aus dem Reaktionsgemisch nicht isoliert, sondern sofort weiter zu den Verbindungen der allgemeinen Formel I umsetzt.

Die Synthesen verschiedener mehrfachsubstituierter Phenylderivate, die zum Aufbau der erfindungsgemäßen as-Indacenderivate mit den anellierten Fünfringen B und B' verwendet werden, werden in den Beispielen exemplarisch beschrieben. Die Ausgangssubstanzen sind nach allgemein zugänglichen Literaturvorschriften oder käuflich zu erhalten. Die beschriebenen Reaktionen sind ebenfalls als literaturbekannt anzusehen.

Eine beispielhafte Synthese zum Aufbau des as-Indacengerüsts ist im folgenden dargestellt. Die Synthese kann durch die Wahl geeigneter Ausgangsprodukte an die jeweils gewünschten Verbindungen der allgemeinen Formel I angepasst werden.

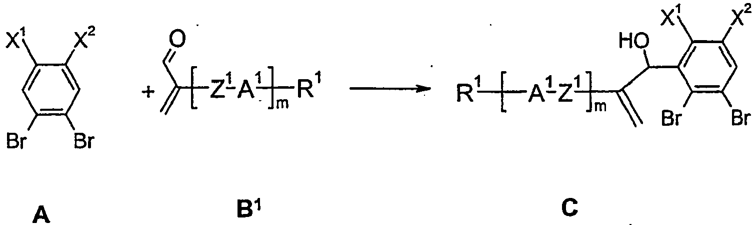

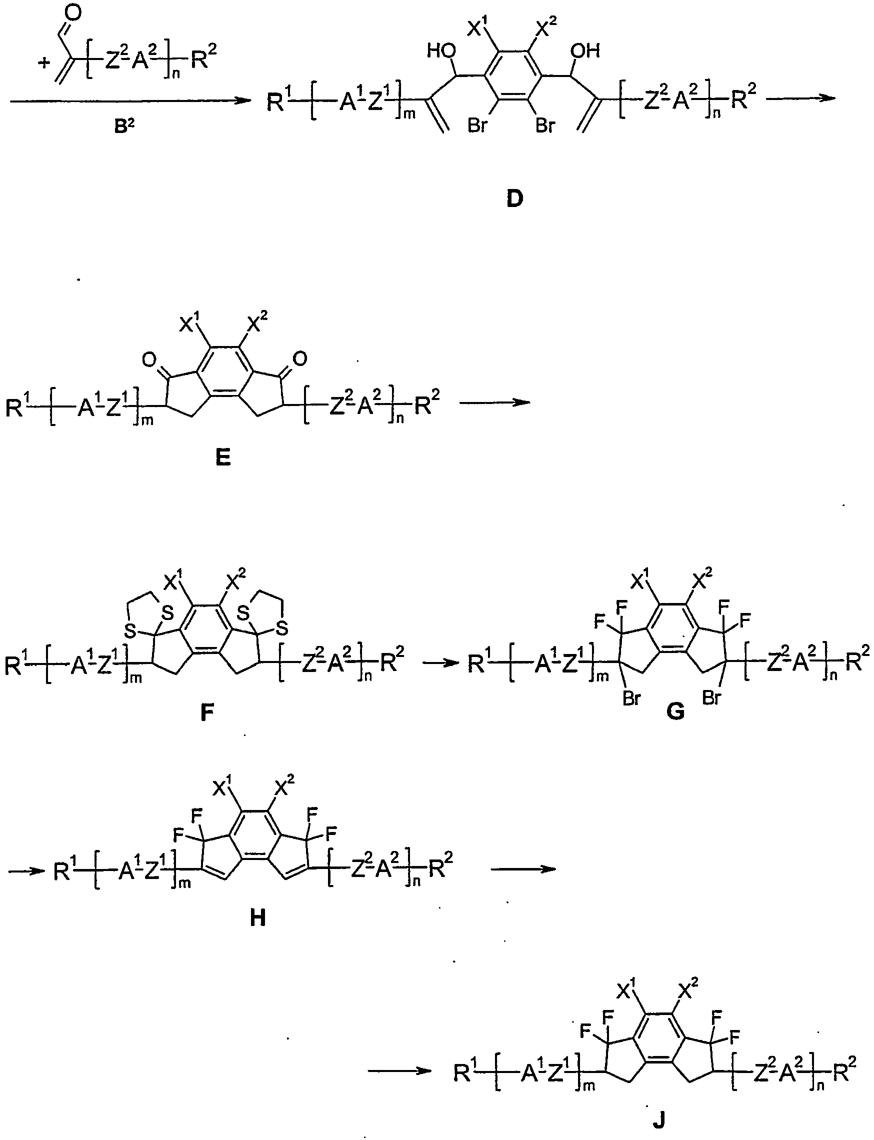

Ausgehend von dem 1,2-Dibromphenolderivat **A** wird durch Umsetzung mit dem α,β-ungesättigten Aldehyd **B**¹ in Gegenwart von Lithiumdiisopropylamid (LDA) die Verbindung **C** erhalten. Durch Wiederholung dieser Reaktion mit dem Aldehyd **B²** wird **D** erhalten. **D** reagiert unter Palladiumkatalyse in Gegenwart von Triethylamin unter Ringschluss zum Diketon **E** (= Verbindung Icc). Aus dem Diketon **E** und 1,2-Ethandithiol wird in Gegenwart von BF₃-Diethylether das entsprechende Bis-dithian **F** erhalten. Dieses wird mit 1,3-Dibrom-5,5-dimethylhydantoin (DBH) und HF in Pyridin zu G umgesetzt. Eliminierung von HBr in Gegenwart von Diazabicycloundecen (DBU) ergibt das as-Indacenderivat **H** (= Verbindung Ibb mit Y¹¹ = Y¹² = Y²¹ = Y²² = F). Das as-Indacenderivat H wird an Palladium/Kohle-Katalysator in Wasserstoffatmosphäre zum as-Indacenderivat **J** (= Verbindung Iaa mit Y¹¹ = Y¹² = Y²¹ = Y²² = F) hydriert. 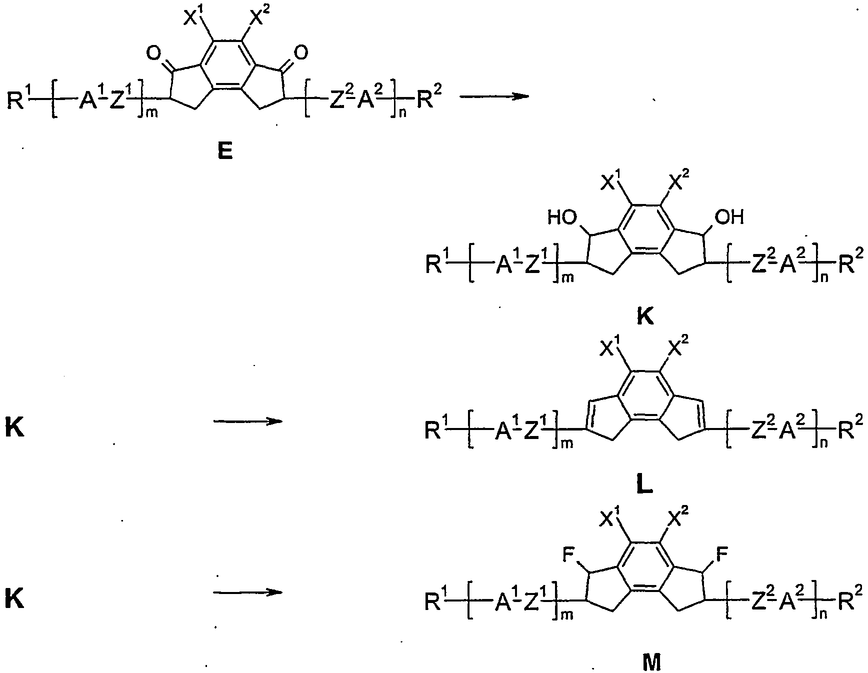 Aus dem Diketon **E** ist zunächst durch Reduktion beispielsweise mit LiAIH₄ der Alkohol **K** erhältlich, aus welchem zum einen durch anschließende Eliminierung mit Säure das as-Indacenderivat **L** (= Verbindung lee mit Y¹¹ = Y²¹ = H) und zum anderen mit DAST (Diaminoschwefeltrifluorid; vgl. M. Hudlicky, Organic Reactions, 35, 1988, 513) das as-Indacenderivat **M** (= Verbindung laa mit Y¹¹ = Y¹² = F und Y²¹ = Y²² = H) analog zu dem in DE 44 34 975 A1 beschriebenen Verfahren zugänglich ist.

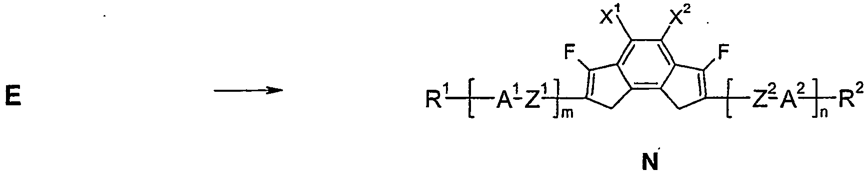

Aus dem Diketon **E** ist ferner analog zu dem in DE 44 34 975 A1 offenbarten Verfahren das as-Indacenderivat **N** (= Verbindung lee mit Y¹ = Y² = F) durch Umsetzung mit DAST zugänglich.

Auch die Einführung von Chloratomen (anstelle von Fluoratomen) ist ausgehend vom Diketon **E** mit Phosphorylchlorid unter Erwärmung in Dimethylformamid möglich. Die Einführung von Trifluormethylresten erfolgt analog zu dem in DE 10135499 A1 offenbarten Verfahren ausgehend vom Diketon **E.**

Eine weitere beispielhafte Synthese erfindungsgemäßer Verbindungen der Formel I zeigt das folgende Schema:

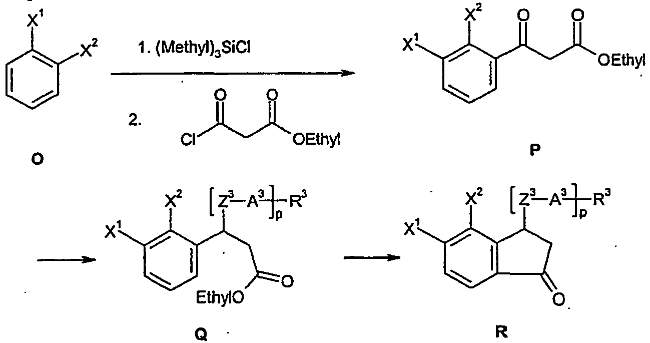

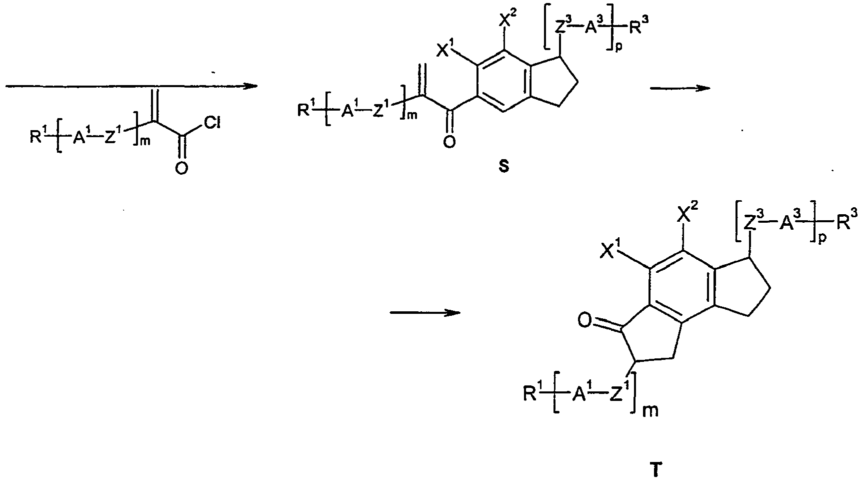

Ausgehend von **O** kann mit n-Butyllithium und Trimethylsilylchlorid eine Trimethylsilylgruppe an dem Phenylring eingeführt werden; anschließende Umsetzung mit dem gezeigten Säurechlorid unter Lewis-Säure-Katalyse ergibt **P.** Je nach Bedeutung des Restes -[-Z³-A³-]ₚ-R³ erfolgt seine Einführung unter Bildung von **Q** durch Wittig-Reaktion und anschließende katalytische Hydrierung (p = 0, R³ = Alkyl) oder durch Umsetzung mit einer geeigneten metallorganischen Verbindung, die -[-Z³-A³-]ₚ-R³ als Strukturelement enthält, anschließende Eliminierung der gebildeten OH-Gruppe und abschließende katalytische Reduktion. Die Cyclisierung zu **R** erfolgt nach Verseifung der Esterfunktion und Überführung in ein Säurechlorid unter Lewis-Säure-Katalyse. Die gebildete Keto-Funktion von **R** kann mittels Wolff-Kishner-Reduktion reduziert werden, woran sich eine Silylierung mit Trimethylsilylchlorid und Umsetzung mit dem gezeigten Säurechlorid zu **S** anschließt. Die Cyclisierung zu **T** erfolgt unter Säurekatalyse. Ausgehend von **T** sind dann in der weiter oben dargelegten Weise weitere erfindungsgemäße Verbindungen der Formel I zugänglich.

Erfindungsgemäße Verbindungen mit einem oder zwei sauerstoffhaltigen Fünfringen lassen sich gemäß dem folgenden Schema beziehungsweise unter Anpassung dieser und der für die Bildung der carbocyclischen Fünfringe beschriebenen Synthesen herstellen:

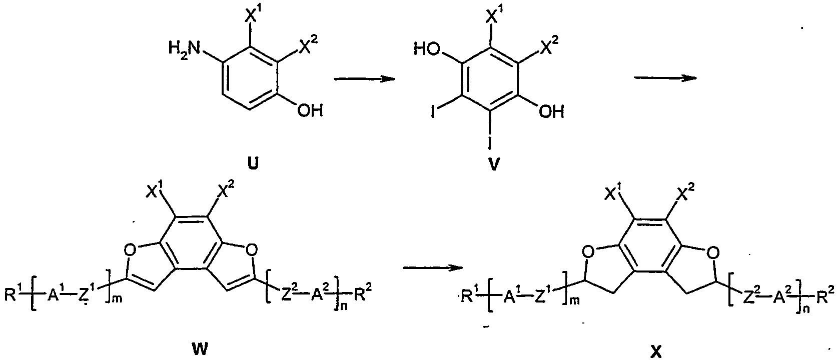

Der Aromat **U** wird nach Diazotierung und Einführung der zweiten OH-Gruppe mit Iod/Kaliumcarbonat in den diiodierten Aromaten **V** überführt. Unter Katalyse mit Bistriphenylphosphinpalladium(II)chlorid und Kupfer(I)iodid wird mit dem Alkin R¹-[-A¹-Z¹-]ₘ-≡ bzw. R²-[-A²-Z²-]ₙ-≡ das Benzodifuran **W** erhalten, aus dem mittels katalytischer Hydrierung auch **X** zugänglich ist.

Die nachfolgende Synthesesequenz veranschaulicht die Herstellung erfindungsgemäßer Verbindungen mit einem Sauerstoff-haltigen Fünfring, wobei für die einzelnen Syntheseschritte auf die für die obigen Schemata erläuterten Herstellungsmethoden zurückgegriffen werden kann.

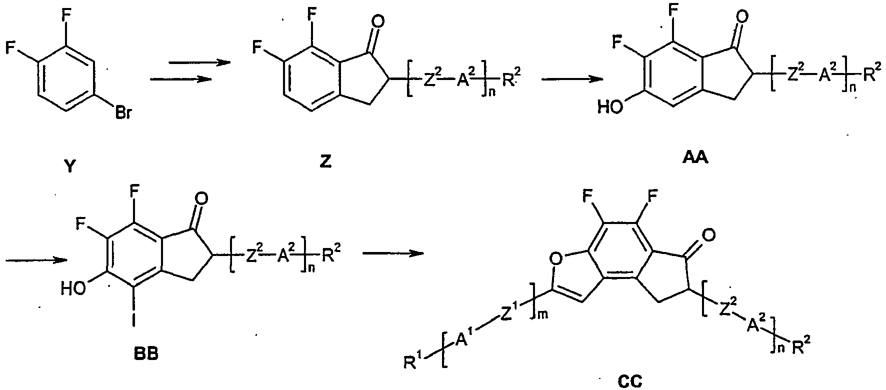

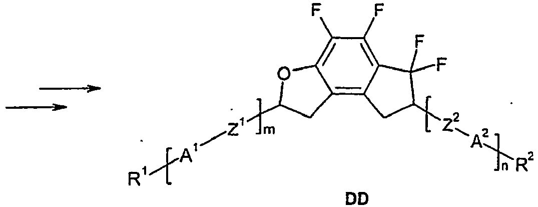

Die dargestellten Reaktionen sind nur als beispielhaft aufzufassen. Der Fachmann kann entsprechende Variationen der vorgestellten Synthesen vornehmen sowie auch andere geeignete Synthesewege beschreiten, um Verbindungen der Formel I zu erhalten.

Wie bereits erwähnt, können die Verbindungen der allgemeinen Formel I in flüssigkristallinen Medien verwendet werden.

Gegenstand der vorliegenden Erfindung ist daher auch ein flüssigkristallines Medium mit mindestens zwei flüssigkristallinen Verbindungen, enthaltend mindestens eine Verbindung der allgemeinen Formel I.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydropyrane-, Phenyl- oder Cyclohexylbenzoate, Cyclohexancarbonsäurephenyl- oder-cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylen-gruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-CH₂CH₂-E-R" (V)

R'-L-CF₂O-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (IIIa), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet E

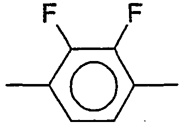

In den Verbindungen der Gruppe B, die mit den Teilformeln (IIb), (IIIb), (IVb), (Vb) und (VIb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN; diese Untergruppe wird im folgenden als Gruppe C bezeichnet und die Verbindungen dieser Untergruppe werden entsprechend mit Teilformeln (IIc), (IIIc), (IVc), (Vc) und (VIc) beschrieben. In den Verbindungen der Teilformeln (IIc), (IIIc), (IVc), (Vc) und (Vlc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:
Gruppe A: 0 bis 90%, vorzugsweise 20 bis 90%, insbesondere 30 bis 90%
Gruppe B: 0 bis 80%, vorzugsweise 10 bis 80%, insbesondere 10 bis 70%
Gruppe C: 0 bis 80%, vorzugsweise 5 bis 80%, insbesondere 5 bis 50%.

Die erfindungsgemäßen Medien enthalten vorzugsweise 1 bis 40%, besonders bevorzugt 5 bis 30% an den erfindungsgemäßen Verbindungen der Formel I. Weiterhin bevorzugt sind Medien, enthaltend mehr als 40%, insbesondere 45 bis 90% an erfindungsgemäßen Verbindungen der Formeln I, II, III, IV und/oder V. Die Medien enthalten vorzugsweise eine, zwei, drei, vier oder fünf erfindungsgemäße Verbindungen der Formel I.

Beispiele für die Verbindungen der Formeln (II), (III), (IV), (V) und (VI) sind die nachstehend aufgeführten Verbindungen: 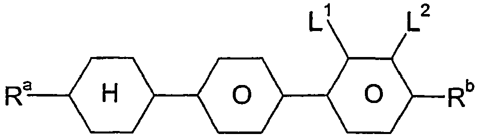 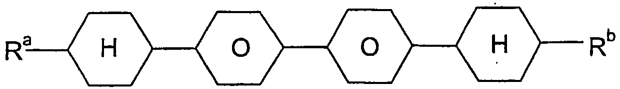 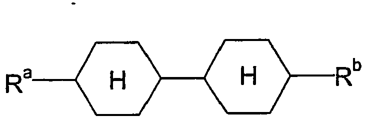 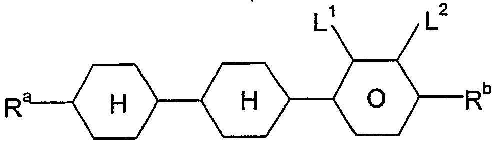 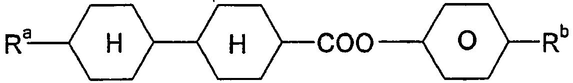 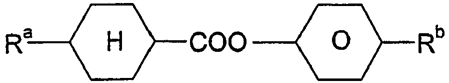 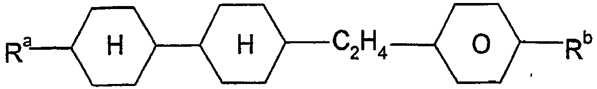 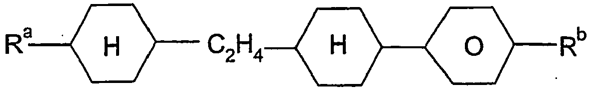 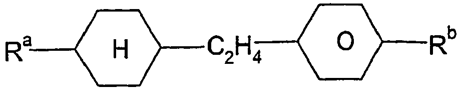 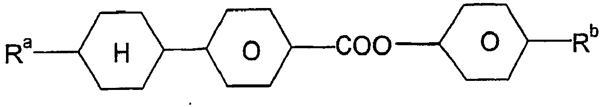 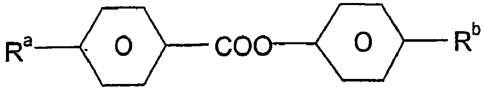 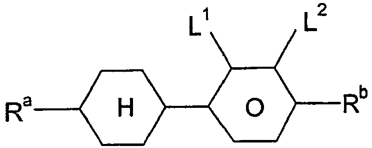 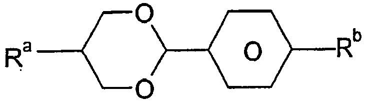 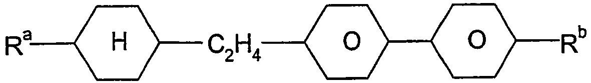 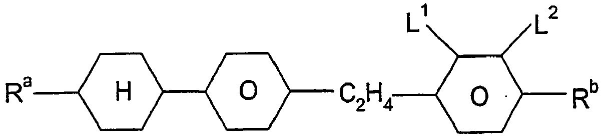 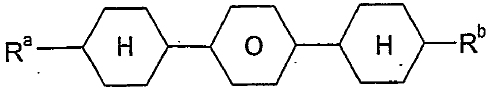 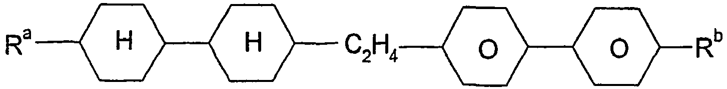 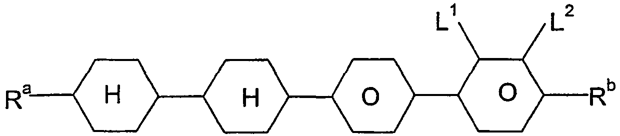 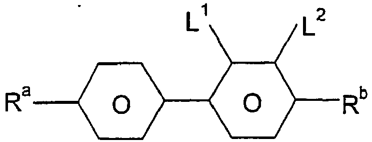 mit R^{a}, R^{b} unabhängig voneinander -CₙH₂ₙ₊₁ oder -OCₙH₂ₙ₊₁ und n = 1, 2, 3, 4, 5, 6, 7, 8 sowie L¹, L² unabhängig voneinander -H oder -F, 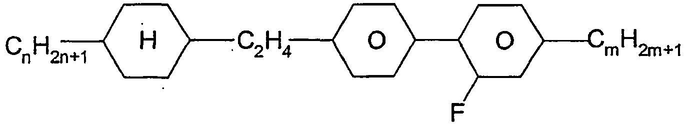 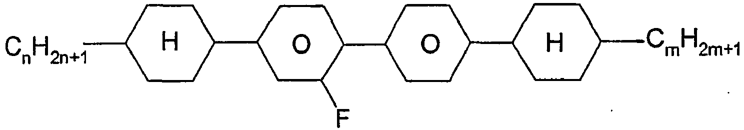 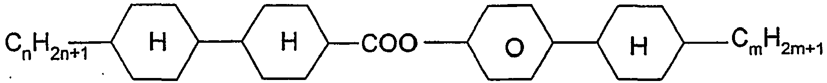 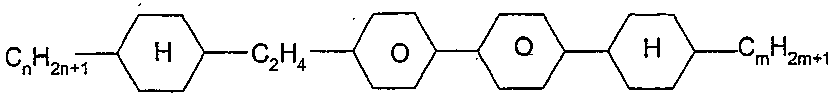 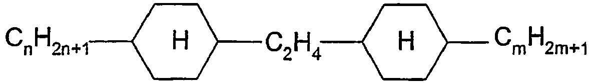 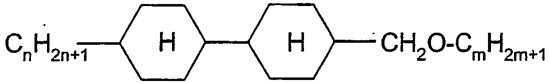 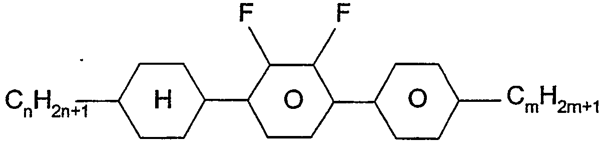 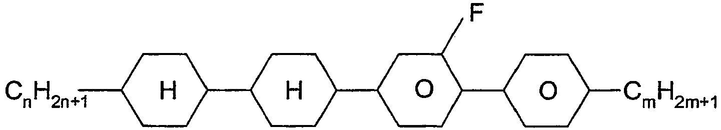 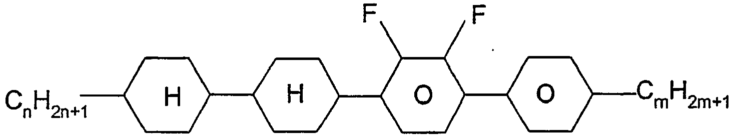 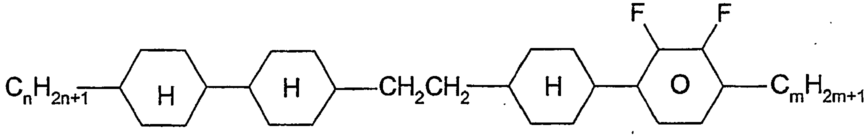 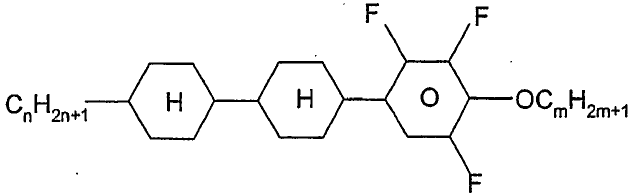 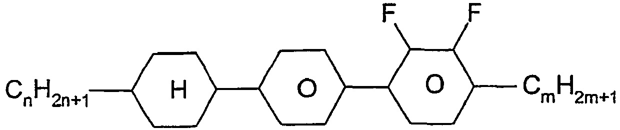 mit m, n unabhängig voneinander 1, 2, 3, 4, 5, 6, 7, 8.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen der vorliegenden Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen Δε insbesondere für eine Verwendung in VA-TFT-Displays.

Gegenstand der vorliegenden Erfindung sind daher auch elektrooptische Flüssigkristallanzeigeelemente, enthaltend ein erfindungsgemäßes flüssigkristallines Medium.

Die Erfindung wird im folgenden anhand von Ausführungsbeispielen näher erläutert.

### Beispiele

Die Ausgangssubstanzen können nach allgemein zugänglichen Literaturvorschriften oder käuflich erhalten werden. Die beschriebenen Reaktionen sind literaturbekannt.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

### Beispiel A

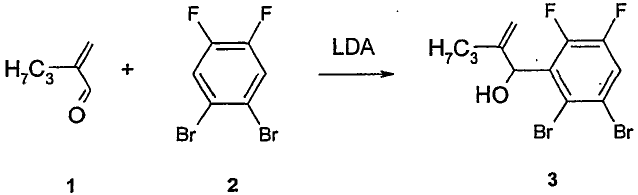

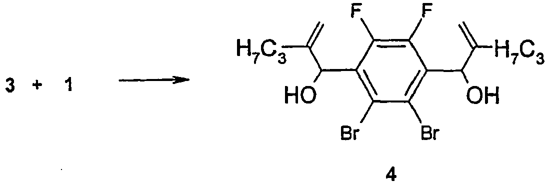

Bei -75°C werden 27,0 ml einer mit 100 ml verdünnten Lösung von 2 N Lithiumdiisopropylamid (LDA) in Cyclohexan/Ethylbenzol/THF (54.0 mmol) mit einer Lösung von 13,5 g (50,0 mmol) des 1,2-Dibrom-4,5-difluorbenzols **2** in 10 ml THF versetzt. Nach 2 Stunden bei der tiefen Temperatur werden 4,6 g (47,0 mmol) des Aldehyds 1 in 10 ml THF hinzugefügt. Nach 30 Minuten wird die Kühlung entfernt, und der Ansatz bei 20°C mit 100 ml 1 N HCl versetzt. Nach Extraktion der wässrigen Phase, Trocknen der organischen Phase, Einengen und Chromatographie erhält man den Allylalkohol **3**.

Durch Wiederholung dieser Reaktion ausgehend von **3** wird der Bisallylalkohol **4** erhalten.

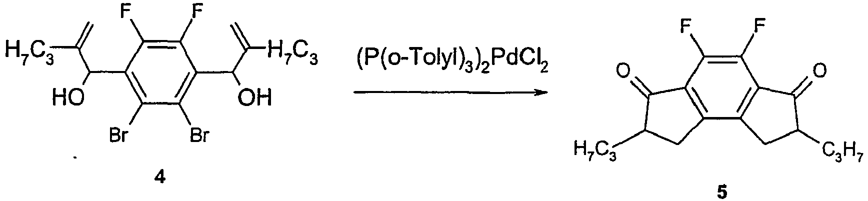

35,0 g (74,8 mmol) des Bis-allylalkohols **4**, 5,5 g Bis(tri-o-tolylphosphin)palladiumdichlorid und 50 ml Triethylamin werden in 390 ml Acetonitril gelöst und bis zur vollständigen Umsetzung des Allylalkohols auf 90°C erwärmt. Der erkaltete Ansatz wird auf Wasser gegeben. Nach Extraktion, Trocknen, Einengen und Chromatographie erhält man das Diketon **5**.

### Beispiel B

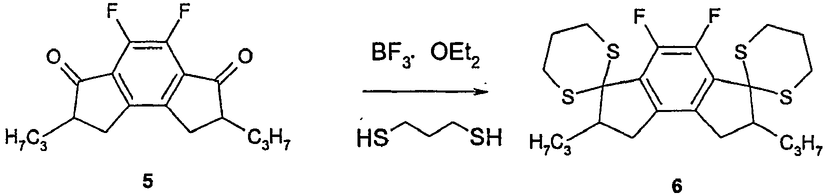

10,0 g (32,6 mmol) des Diketons **5** und 7,0 ml (69.9 mmol) Propandithiol werden in 50 ml Dichlormethan gelöst und bei 6 bis 7°C mit 7,0 ml Bortrifluorid-Diethylether-Komplex versetzt und anschließend über Nacht bei Raumtemperatur gerührt. Der Ansatz wird auf 10 ml gesättigte Natriumhydrogencarbonat-Lösung gegeben und bis zur Beendigung der Gasentwicklung gerührt. Nach Extraktion der wässrigen Phase, Trocknen der organischen Phase, Einengen und Filtration über Kieselgel wird der erhaltene Rückstand ohne weitere Reinigung in der nächsten Stufe eingesetzt.

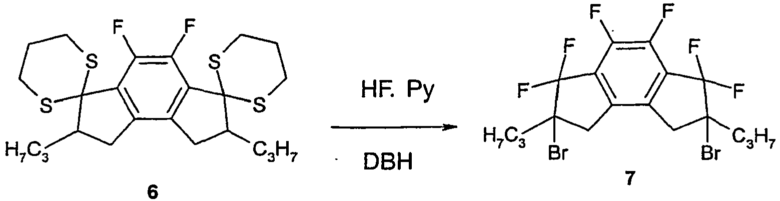

10,0 g des rohen Thioketals **6**, gelöst in 30 ml Dichlormethan, werden langsam bei -75°C in ein Gemisch aus 45,8 g (160 mmol) 1,3-Dibrom-5,5-dimethylhydantoin (DBH), 80 ml einer 65 %igen Lösung von Fluorwasserstoff in Pyridin und 50 ml Dichlormethan gegeben. Anschließend wird der Ansatz über Nacht bei Raumtemperatur gerührt. Das Reaktionsgemisch wird in eisgekühlte Hydrogensulfit-Lösung gegeben und mit gesättigter Natriumhydrogencarbonat-Lösung und Natronlauge entsäuert. Nach Extraktion, Trocknen, Einengen, erneutem Waschen mit Wasser, Chromatographie und Kristallisation aus Hexan erhält man das as-Indacenderivat **7**.

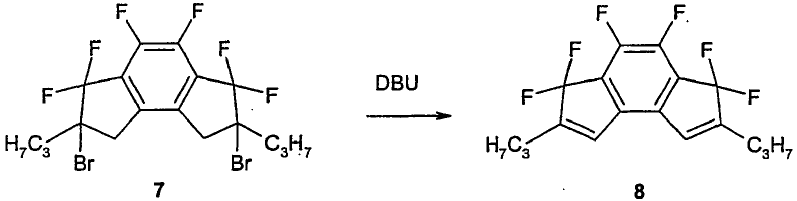

6,0 g (11.8 mmol) des as-Indacenderivat **7** werden in 50 ml Dichlormethan gelöst, mit 4,5 ml (30,0 mmol) 1,8-Diazabicyclo[5.4.0]-undec-7-en (DBU) versetzt und bei Raumtemperatur gerührt, bis das Edukt vollständig umgesetzt ist. Der Ansatz wird mit Wasser und gesättigter NatriumchloridLösung gewaschen, eingeengt und chromatographiert. Es wird das as-Indacenderivat **8** isoliert.

### Beispiel C

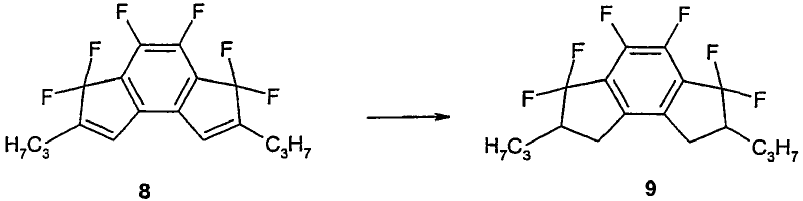

4,0 g (11,6 mmol) des as-Indacenderivats **8** werden in 50 ml THF gelöst und bei Raumtemperatur und Normaldruck am Palladiumkatalysator hydriert. Nach Einengen, Chromatographie an Kieselgel und Kristallisation erhält man das as-Indacenderivat **9**.

### Beispiel D

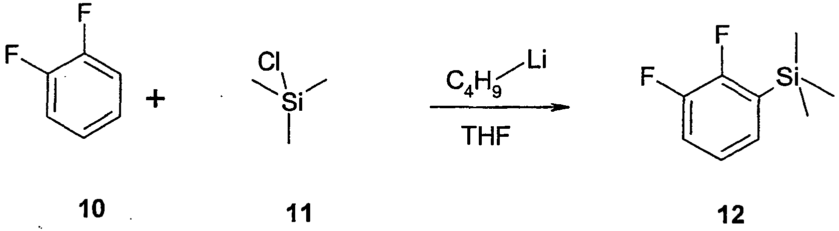

Unter Stickstoff werden 116,5 g (1,00 mol) 1,2-Difluorbenzol (**10**) in 600 ml Tetrahydrofuran gelöst und bei -70°C mit 656 ml einer 15%igen Lösung von Butyllithium in *n*-Hexan (1,05 mol) versetzt. Nach 1 h bei dieser Temperatur wird eine Lösung von 132,6 ml (1,05 mol) Trimethylsilylchlorid (**11**) in 100 ml Tetrahydrofuran langsam in das Reaktionsgemisch gegeben. Der Ansatz taut über Nacht auf und wird auf 1,5 I ges. Ammoniumchloridlösung gegeben. Die organische Phase wird über Natriumsulfat getrocknet und eingeengt: Der erhaltene Rückstand wird bei 8 mbar destilliert. Man erhält 152 g (81 %, Sdp. 88°C) der Silylverbindung **12**.

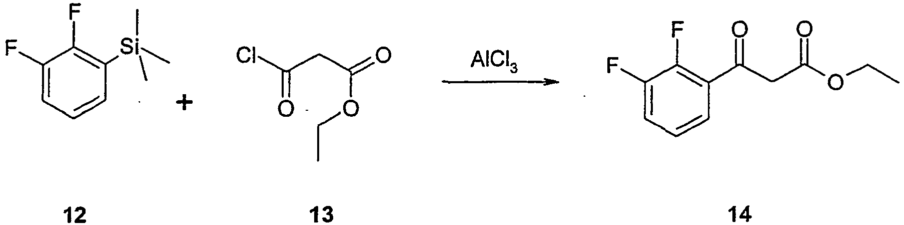

Unter Stickstoff werden 37,6 g (250 mmol) des Säurechlorids **13** in 200 ml Cyclohexan gelöst. Bei 0-2 °C werden 46,6 g (250 mmol) der Silylverbindung **12** in die Lösung gegeben. Anschließend werden 37,3 g (280 mmol) Aluminiumchlorid portionsweise in das Reaktionsgemisch gegeben, wobei die Temperatur zwischen -2 °C und 2 °C gehalten wird. Nach 1 h bei tiefer Temperatur wird die Kühlung entfernt. Der Ansatz erwärmt sich auf Raumtemperatur und wird auf Eis gegeben. Die wäßrige Phase wird mit Methyl-tert.-butylether extrahiert, die organische Phase wird über Natriumsulfat getrocknet und eingeengt. Der Rückstand wird an Kieselgel gereinigt. Man erhält 31,4 g (55%) des Ketoesters **14**.

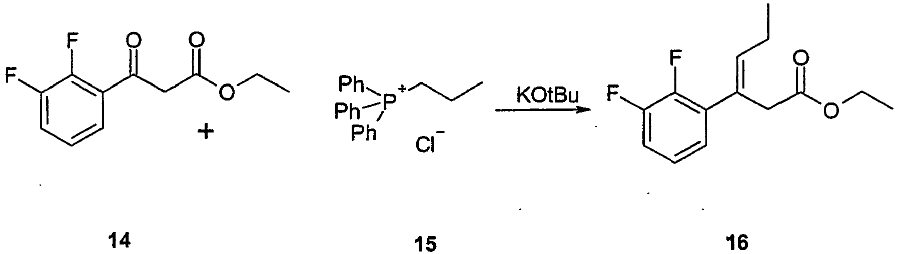

Unter Stickstoff werden 51,1 g (150 mmol) des Wittigsalzes **15** in 200 ml THF suspendiert und bei 5-10 °C mit einer Lösung von 15,7 g (140 mmol) Kalium-*tert*.-butylat in 75 ml THF versetzt. Nach einer Stunde wird der Ketoester **14** gelöst in 75 ml THF hinzugefügt. Anschließend wird die Kühlung entfernt. Nach 20 h bei Raumtemp. wird der Ansatz mit Wasser versetzt. Die wäßrige Phase wird mit MTB-Ether extrahiert. Die organische Phase wird mit ges. Natriumchloridlösung gewaschen, über Natriumsulfat getrocknet und eingeengt. Der Rückstand wird über Kieselgel gegeben (MTB-Ether/ n-Hexan 1:10). Man erhält 29,4 g (88%) des Esters **16**.

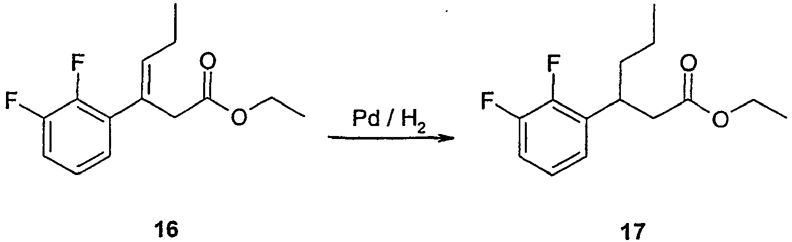

29,0 g (21,6 mmol) des Esters **16** werden in 500 ml Tetrahydrofuran gelöst und am Palladiumkatalysator hydriert. Die Hydrierlösung wird eingeengt und der Rückstand an Kieselgel chromatographiert. Man erhält 27,8 g (95%) der Substanz **17**.

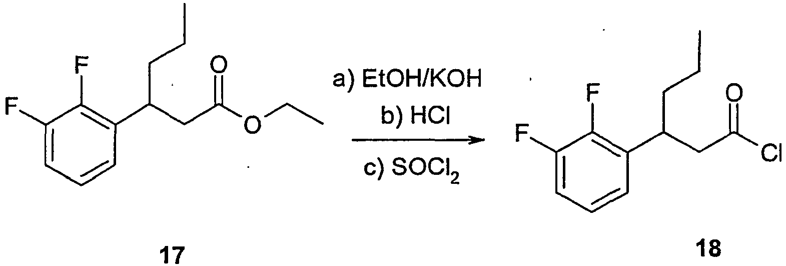

27,0 g (105 mmol) des Esters **17** werden 5h in ethanolischer Kaliumhydroxidlösung unter Rückfluß erhitzt. Anschließend wird der Alkohol abdestilliert, der Rückstand mit Wasser aufgenommen, mit Salzsäure angesäuert und mit MTB-Ether extrahiert. Die organische Phase wird über Natriumsulfat getrocknet und eingeengt. Der erhaltene Rückstand wird mit 50 ml Thionylchlorid und einem Tropfen DMF versetzt und bis zum Ende der Gasentwicklung unter Rückfluss erhitzt. Überschüssiges Thionylchlorid wird abdestilliert. Der Rückstand (25 g) wird ohne weitere Reinigung in der nächsten Stufe eingesetzt.

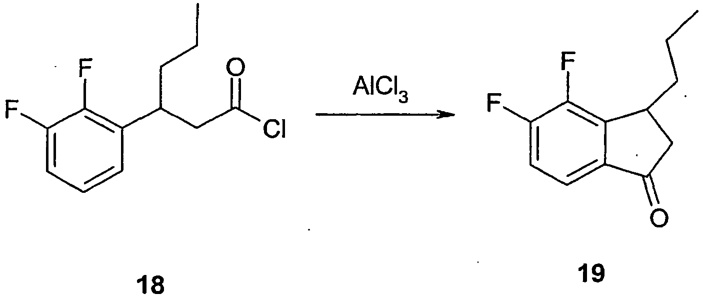

Eine Lösung von 25 g (ca. 105 mmol) des rohen Säurechlorids **18** in 120 ml Dichlormethan werden unter Stickstoff und bei -20 bis -15 °C in eine Suspension von 16,4 g (119 mmol) Aluminiumchlorid in 80 ml Dichlormethan gegeben. Nach 4,5 h wird die Reaktion durch Zugabe von Eis abgebrochen und mit Salzsäure angesäuert. Die wäßrige Phase wird mit Methylenchlorid extrahiert. Die organische Phase wird über Natriumsulfat getrocknet und eingeengt. Der Rückstand wird an Kieselgel (MTB/*n*-Heptan 1:5) chromatographiert. Man erhält 18,5 g (84%) des Indanons **19**.

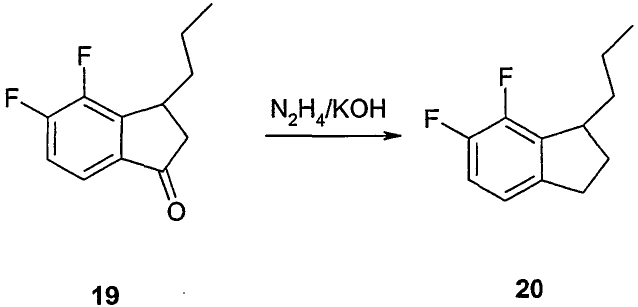

23,9 g (361 mmol) Kaliumhydroxid werden in 90 ml Diethylenglycol gelöst. Die Lösung wird mit 13,0 ml (268 mmol) Hydraziniumhydroxid und 18,0 g (85,6 mmol) des Indanons **19** versetzt und 2 h unter Rückfluss erhitzt. Anschließend wird die Temperatur des Heizbades auf 200 °C erhöht und bis zum Ende der Gasentwicklung gehalten. Das abgekühlte Reaktionsgemisch wird auf Wasser gegossen und angesäuert. Die wäßrige Phase wird mit *n-*Pentan extrahiert. Die organische Phase wird über Natriumsulfat getrocknet und eingeengt. Der Rückstand wird durch Chromatographie an Kieselgel (n-Pentan) gereinigt. Man erhält 11,4 g (68%) des Indans **20**.

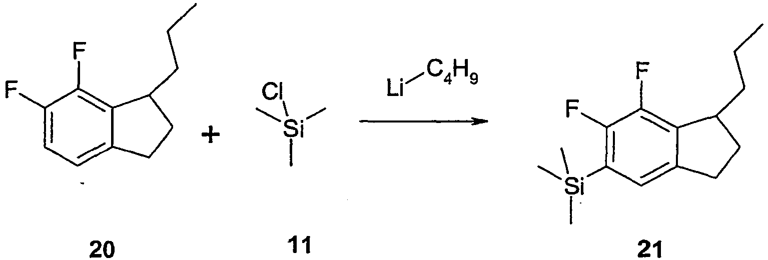

Die Silylierung des Indans **20** erfolgt nach der obigen Vorschrift. Die Aufreinigung erfolgt über Chromatographie an Kieselgel (*n-*Pentan). Man erhält aus 11,0 g (56,5 mmol) Indan **20** 12,6 g (84%) der Silylverbindung **21**.

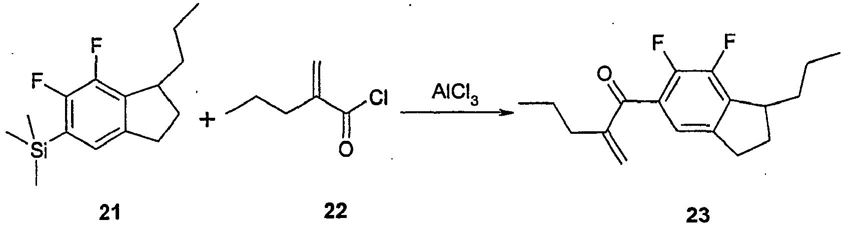

Die Acylierung der Silylverbindung **21** erfolgt nach der obigen Vorschrift. Man erhält aus 12,0 g (44,7 mmol) **21** 6,8 g (52 %) des Ketons **23**.

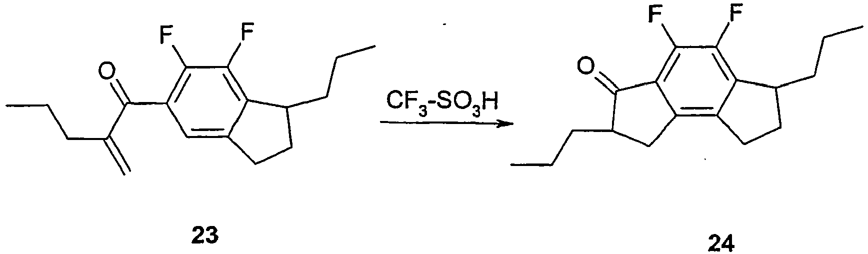

Unter Eiskühlung und Rühren werden 6,5 g (22,2 mmol) des Ketons **23** in 30 I Trifluormethansulfonsäure eingetragen. Nach 24 h wird der Ansatz auf Eis gegeben und mit MTB-Ether extrahiert. Die organische Phase wird mit ges. Natriumhydrogencarbonatlösung gewaschen, über Natriumsulfat getrocknet und eingeengt. Der Rückstand wird an Kieselgel (MTB-Ether/*n-*Heptan 1:8) gereinigt. Man erhält 2,6 g (40%) des Indacens **24**.

Die weitere Derivatisierung von **24** erfolgt analog den Beispielen B und C.

### Beispiel E

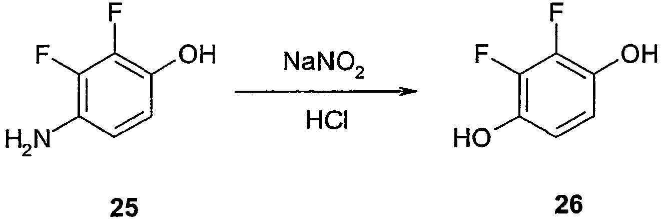

25,0 g (172 mmol) des Aromaten **1** werden in 10 ml halbkonz. Salzsäure gelöst und bei einer Temperatur von unter 5°C mit 11,9 g (172 mmol) Natriumnitrit in 70 ml Wasser versetzt. Nach Beendigung der Nitritzugabe wird der Ansatz mit 100 ml Wasser verdünnt und zum Sieden erhitzt. Nachdem die Stickstoffentwicklung (Blasenzähler) abgeklungen ist, wird der Ansatz noch eine weitere Stunde unter Rückfluss erhitzt. Die abgekühlte Reaktionslösung wird mit MTB-Ether extrahiert. Die organische Phase wird über Natriumsulfat getrocknet und eingeengt. Man erhält 13,7 g (54%) des Diols **26**.

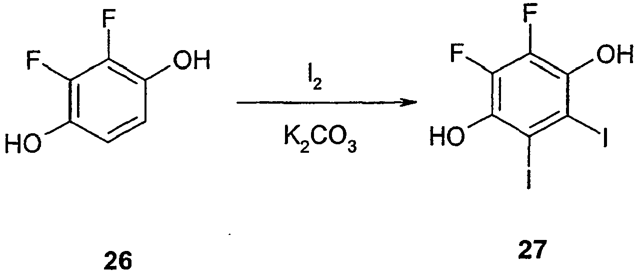

13,0 g (90,0 mmol) des Phenols **26** und 48 g (348 mmol) Kaliumcarbonat werden in 160 ml Wasser gelöst und bei 5°C portionsweise mit 50 g (200 mmol) Iod versetzt. Der Ansatz wird über Nacht gerührt. Die Reaktionslösung wird vom entstandenen Niederschlag abdekantiert und mit Salzsäure angesäuert. Die wäßrige Phase wird mit MTB-Ether extrahiert, mit ges. Natriumchloridlösung gewaschen, über Natriumsulfat getrocknet und eingeengt. Der Rückstand wird über Kieselgel gegeben (MTB-Ether/ Heptan 2:1). Man erhält 20,1 g (57%) des diiodierten Aromaten **27**.

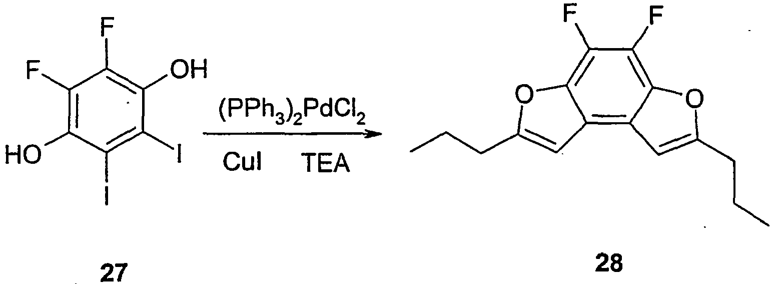

Unter Stickstoff werden 20,0 g (50,3 mmol) des Phenols **27** in 120 ml Dimethylformamid und 28 ml Triethylamin gelöst und mit 1,0 g Bistriphenylphosphinpalladium(II)chlorid und 580 mg Kupfer(I)iodid versetzt. Anschließend wird eine Lösung von 10,3 ml (105 mmol) 1-Pentin in 30 ml Dimethylformamid langsam in den Ansatz gegeben. Der Ansatz wird bis zum vollständigen Umsatz (DC) bei Raumtemp. gerührt. Das Reaktionsgemisch wird mit Wasser versetzt und mit MTB-Ether extrahiert. Die organische Phase wird über Natriumsulfat getrocknet und eingeengt. Nach der Chromatographie an Kieselgel erhält man 6.5 g (46%) des Benzodifurans **28**.

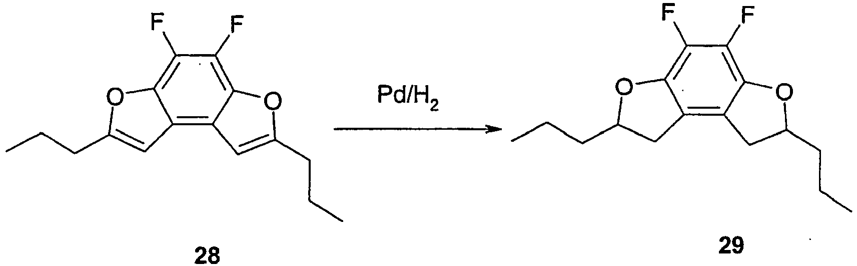

6,0 g (21,6 mmol) des Benzodifurans **28** werden in 100 ml Tetrahydrofuran gelöst und am Palladiumkatalysator hydriert. Die Hydrierlösung wird eingeengt und der Rückstand an Kieselgel chromatographiert. Man erhält 5,5 g (90%) der Substanz **29**.

Die folgenden Verbindungen wurden gemäß beziehungsweise analog den Beispielen A bis E hergestellt. Dabei steht "Bdg." für eine Einfachbindung:

### Beispiele 1-259

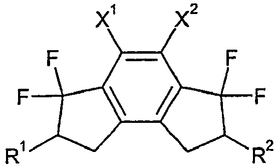

### Beispiele 260-518

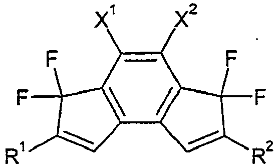

### Beispiele 519-777

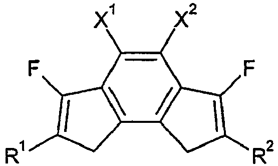

| **Beispiel-Nr.** | | | **X¹** | **X²** | **R¹** | **R²** |
|---|---|---|---|---|---|---|
| 1, | 260, | 519, | H | H | H | CH₃ |
| 2, | 261, | 520, | H | H | H | C₂H₅ |
| 3, | 262, | 521, | H | H | H | n-C₃H₇ |
| 4, | 263, | 522, | H | H | H | n-C₄H₉ |
| 5, | 264, | 523, | H | H | H | n-C₅H₁₁ |
| 6, | 265, | 524, | H | H | H | n-C₆H₁₃ |
| 7, | 266, | 525, | H | H | H | n-C₇H₁₅ |
| 8, | 267, | 526, | F | H | H | CH₃ |
| 9, | 268, | 527, | F | H | H | C₂H₅ |
| 10, | 269, | 528, | F | H | H | n-C₃H₇ |
| 11, | 270, | 529, | F | H | H | n-C₄H₉ |
| 12, | 271, | 530, | F | H | H | n-C₅H₁₁ |
| 13, | 272, | 531, | F | H | H | n-C₆H₁₃ |
| 14, | 273, | 532, | F | H | H | n-C₇H₁₅ |
| 15, | 274, | 533, | H | F | H | CH₃ |
| 16, | 275, | 534, | H | F | H | C₂H₅ |
| 17, | 276, | 535, | H | F | H | n-C₃H₇ |
| 18, | 277, | 536, | H | F | H | n-C₄H₉ |
| 19, | 278, | 537, | H | F | H | n-C₅H₁₁ |
| 20, | 279, | 538, | H | F | H | n-C₆H₁₃ |
| 21, | 280, | 539, | H | F | H | n-C₇H₁₅ |
| 22, | 281, | 540, | F | F | H | CH₃ |
| 23, | 282, | 541, | F | F | H | C₂H₅ |
| 24, | 283, | 542, | F | F | H | n-C₃H₇ |
| 25, | 284, | 543, | F | F | H | n-C₄H₉ |
| 26, | 285, | 544, | F | F | H | n-C₅H₁₁ |
| 27, | 286, | 545, | F | F | H | n-C₆H₁₃ |
| 28, | 287, | 546, | F | F | H | n-C₇H₁₅ |
| 29, | 288, | 547, | CF₃ | H | H | CH₃ |
| 30, | 289, | 548, | CF₃ | H | H | C₂H₅ |
| 31, | 290, | 549, | CF₃ | H | H | n-C₃H₇ |
| 32, | 291, | 550, | CF₃ | H | H | n-C₄H₉ |
| 33, | 292, | 551, | CF₃ | H | H | n-C₅H₁₁ |
| 34, | 293, | 552, | CF₃ | H | H | n-C₆H₁₃ |
| 35, | 294, | 553, | CF₃ | H | H | n-C₇H₁₅ |
| 36, | 295, | 554, | H | CF₃ | H | CH₃ |
| 37, | 296, | 555, | H | CF₃ | H | C₂H₅ |
| 38, | 297, | 556, | H | CF₃ | H | n-C₃H₇ |
| 39, | 298, | 557, | H | CF₃ | H | n-C₄H₉ |
| 40, | 299, | 558, | H | CF₃ | H | n-C₅H₁₁ |
| 41, | 300, | 559, | H | CF₃ | H | n-C₆H₁₃ |
| 42, | 301, | 560, | H | CF₃ | H | n-C₇H₁₅ |
| 43, | 302, | 561, | -OCF₃ | H | H | CH₃ |
| 44, | 303, | 562, | -OCF₃ | H | H | C₂H₅ |
| 45, | 304, | 563, | -OCF₃ | H | H | n-C₃H₇ |
| 46, | 305, | 564, | -OCF₃ | H | H | n-C₄H₉ |
| 47, | 306, | 565, | -OCF₃ | H | H | n-C₅H₁₁ |
| 48, | 307, | 566, | -OCF₃ | H | H | n-C₆H₁₃ |
| 49, | 308, | 567, | -OCF₃ | H | H | n-C₇H₁₅ |
| 50, | 309, | 568, | H | -OCF₃ | H | CH₃ |
| 51, | 310, | 569, | H | -OCF₃ | H | C₂H₅ |
| 52, | 311, | 570, | H | -OCF₃ | H | n-C₃H₇ |
| 53, | 312, | 571, | H | -OCF₃ | H | n-C₄H₉ |
| 54, | 313, | 572, | H | -OCF₃ | H | n-C₅H₁₁ |
| 55, | 314, | 573, | H | -OCF₃ | H | n-C₆H₁₃ |
| 56, | 315, | 574, | H | -OCF₃ | H | n-C₇H₁₅ |
| 57, | 316, | 575, | H | H | CH₃ | CH₃ |
| 58, | 317, | 576, | H | H | CH₃ | C₂H₅ |
| 59, | 318, | 577, | H | H | CH₃ | n-C₃H₇ |
| 60, | 319, | 578, | H | H | CH₃ | n-C₄H₉ |
| 61, | 320, | 579, | H | H | CH₃ | n-C₅H₁₁ |
| 62, | 321, | 580, | H | H | CH₃ | n-C₆H₁₃ |
| 63, | 322, | 581, | H | H | CH₃ | n-C₇H₁₅ |
| 64, | 323, | 582, | F | H | CH₃ | CH₃ |
| 65, | 324, | 583, | F | H | CH₃ | C₂H₅ |
| 66, | 325, | 584, | F | H | CH₃ | n-C₃H₇ |
| 67, | 326, | 585, | F | H | CH₃ | n-C₄H₉ |
| 68, | 327, | 586, | F | H | CH₃ | n-C₅H₁₁ |
| 69, | 328, | 587, | F | H | CH₃ | n-C₆H₁₃ |
| 70, | 329, | 588, | F | H | CH₃ | n-C₇H₁₅ |
| 71, | 330, | 589, | F | F | CH₃ | CH₃ |
| 72, | 331, | 590, | F | F | CH₃ | C₂H₅ |
| 73, | 332, | 591, | F | F | CH₃ | n-C₃H₇ |
| 74, | 333, | 592, | F | F | CH₃ | n-C₄H₉ |
| 75, | 334, | 593, | F | F | CH₃ | n-C₅H₁₁ |
| 76, | 335, | 594, | F | F | CH₃ | n-C₆H₁₃ |
| 77, | 336, | 595, | F | F | CH₃ | n-C₇H₁₅ |
| 78, | 337, | 596, | CF₃ | H | CH₃ | CH₃ |
| 79, | 338, | 597, | CF₃ | H | CH₃ | C₂H₅ |
| 80, | 339, | 598, | CF₃ | H | CH₃ | n-C₃H₇ |
| 81, | 340, | 599, | CF₃ | H | CH₃ | n-C₄H₉ |
| 82, | 341, | 600, | CF₃ | H | CH₃ | n-C₅H₁₁ |
| 83, | 342, | 601, | CF₃ | H | CH₃ | n-C₆H₁₃ |
| 84, | 343, | 602, | CF₃ | H | CH₃ | n-C₇H₁₅ |
| 85, | 344, | 603, | -OCF₃ | H | CH₃ | CH₃ |
| 86, | 345, | 604, | -OCF₃ | H | CH₃ | C₂H₅ |
| 87, | 346, | 605, | -OCF₃ | H | CH₃ | n-C₃H₇ |
| 88, | 347, | 606, | -OCF₃ | H | CH₃ | n-C₄H₉ |
| 89, | 348, | 607, | -OCF₃ | H | CH₃ | n-C₅H₁₁ |
| 90, | 349, | 608, | -OCF₃ | H | CH₃ | n-C₆H,₃ |
| 91, | 350, | 609, | -OCF₃ | H | CH₃ | n-C₇H,₅ |
| 92, | 351, | 610, | H | H | C₂H₅ | C₂H₅ |
| 93, | 352, | 611, | H | H | C₂H₅ | n-C₃H₇ |
| 94, | 353, | 612, | H | H | C₂H₅ | n-C₄H₉ |
| 95, | 354, | 613, | H | H | C₂H₅ | n-C₅H₁₁ |
| 96, | 355, | 614, | H | H | C₂H₅ | n-C₆H₁₃ |
| 97, | 356, | 615, | H | H | C₂H₅ | n-C₇H₁₅ |
| 98, | 357, | 616, | F | H | C₂H₅ | CH₃ |
| 99, | 358, | 617, | F | H | C₂H₅ | C₂H₅ |
| 100, | 359, | 618, | F | H | C₂H₅ | n-C₃H₇ |
| 101, | 360, | 619, | F | H | C₂H₅ | n-C₄H₉ |
| 102, | 361, | 620, | F | H | C₂H₅ | n-C₅H₁₁ |
| 103, | 362, | 621, | F | H | C₂H₅ | n-C₆H₁₃ |
| 104, | 363, | 622, | F | H | C₂H₅ | n-C₇H₁₅ |
| 105, | 364, | 623, | F | F | C₂H₅ | C₂H₅ |
| 106, | 365, | 624, | F | F | C₂H₅ | n-C₃H₇ |
| 107, | 366, | 625, | F | F | C₂H₅ | n-C₄H₉ |
| 108, | 367, | 626, | F | F | C₂H₅ | n-C₅H₁₁ |
| 109, | 368, | 627, | F | F | C₂H₅ | n-C₆H₁₃ |
| 110, | 369, | 628, | F | F | C₂H₅ | n-C₇H₁₅ |
| 111, | 370, | 629, | CF₃ | H | C₂H₅ | CH₃ |
| 112, | 371, | 630, | CF₃ | H | C₂N₅ | C₂H₅ |
| 113, | 372, | 631, | CF₃ | H | C₂H₅ | n-C₃H₇ |
| 114, | 373, | 632, | CF₃ | H | C₂H₅ | n-C₄H₉ |
| 115, | 374, | 633, | CF₃ | H | C₂H₅ | n-C₅H₁₁ |
| 116, | 375, | 634, | CF₃ | H | C₂H₅ | n-C₆H₁₃ |
| 117, | 376, | 635, | CF₃ | H | C₂H₅ | n-C₇H₁₅ |
| 118, | 377, | 636, | -OCF₃ | H | C₂H₅ | CH₃ |
| 119, | 378, | 637, | -OCF₃ | H | C₂H₅ | C₂H₅ |
| 120, | 379, | 638, | -OCF₃ | H | C₂H₅ | n-C₃H₇ |
| 121, | 380, | 639, | -OCF₃ | H | C₂H₅ | n-C₄H₉ |
| 122, | 381, | 640, | -OCF₃ | H | C₂H₅ | n-C₅H₁₁ |
| 123, | 382, | 641, | -OCF₃ | H | C₂H₅ | n-C₆H₁₃ |
| 124, | 383, | 642, | -OCF₃ | H | C₂H₅ | n-C₇H₁₅ |
| 125, | 384, | 643, | H | H | n-C₃H₇ | n-C₃H₇ |
| 126, | 385, | 644, | H | H | n-C₃H₇ | n-C₄H₉ |
| 127, | 386, | 645, | H | H | n-C₃H₇ | n-C₅H₁₁ |
| 128, | 387, | 646, | H | H | n-C₃H₇ | n-C₆H₁₃ |
| 129, | 388, | 647, | H | H | n-C₃H₇ | n-C₇H₁₅ |
| 130, | 389, | 648, | F | H | n-C₃H₇ | CH₃ |
| 131, | 390, | 649, | F | H | n-C₃H₇ | C₂H₅ |
| 132, | 391, | 650, | F | H | n-C₃H₇ | n-C₃H₇ |
| 133, | 392, | 651, | F | H | n-C₃H₇ | n-C₄H₉ |
| 134, | 393, | 652, | F | H | n-C₃H₇ | n-C₅H₁₁ |
| 135, | 394, | 653, | F | H | n-C₃H₇ | n-C₆H₁₃ |
| 136, | 395, | 654, | F | H | n-C₃H₇ | n-C₇H₁₅ |
| 137, | 396, | 655, | F | F | n-C₃H₇ | n-C₃H₇ |
| 138, | 397, | 656, | F | F | n-C₃H₇ | n-C₄H₉ |
| 139, | 398, | 657, | F | F | n-C₃H₇ | n-C₅H₁₁ |
| 140, | 399, | 658, | F | F | n-C₃H₇ | n-C₆H₁₃ |
| 141, | 400, | 659, | F | F | n-C₃H₇ | n-C₇H₁₅ |
| 142, | 401, | 660, | CF₃ | H | n-C₃H₇ | CH₃ |
| 143, | 402, | 661, | CF₃ | H | n-C₃H₇ | C₂H₅ |
| 144, | 403, | 662, | CF₃ | H | n-C₃H₇ | n-C₃H₇ |
| 145, | 404, | 663, | CF₃ | H | n-C₃H₇ | n-C₄H₉ |
| 146, | 405, | 664, | CF₃ | H | n-C₃H₇ | n-C₅H₁₁ |
| 147, | 406, | 665, | CF₃ | H | n-C₃H₇ | n-C₆H₁₃ |
| 148, | 407, | 666, | CF₃ | H | n-C₃H₇ | n-C₇H₁₅ |
| 149, | 408, | 667, | -OCF₃ | H | n-C₃H₇ | CH₃ |
| 150, | 409, | 668, | -OCF₃ | H | n-C₃H₇ | C₂H₅ |
| 151, | 410, | 669, | -OCF₃ | H | n-C₃H₇ | n-C₃H₇ |
| 152, | 411, | 670, | -OCF₃ | H | n-C₃H₇ | n-C₄H₉ |
| 153, | 412, | 671, | -OCF₃ | H | n-C₃H₇ | n-C₅H₁₁ |
| 154, | 413, | 672, | -OCF₃ | H | n-C₃H₇ | n-C₆H₁₃ |
| 155, | 414, | 673, | -OCF₃ | H | n-C₃H₇ | n-C₇H₁₅ |
| 156, | 415, | 674, | H | H | n-C₄H₉ | n-C₄H₉ |
| 157, | 416, | 675, | H | H | n-C₄H₉ | n-C₅H₁₁ |
| 158, | 417, | 676, | H | H | n-C₄H₉ | n-C₆H₁₃ |
| 159, | 418, | 677, | H | H | n-C₄H₉ | n-C₇H₁₅ |
| 160, | 419, | 678, | F | H | n-C₄H₉ | CH₃ |
| 161, | 420, | 679, | F | H | n-C₄H₉ | C₂H₅ |
| 162, | 421, | 680, | F | H | n-C₄H₉ | n-C₃H₇ |
| 163, | 422, | 681, | F | H | n-C₄H₉ | n-C₄H₉ |
| 164, | 423, | 682, | F | H | n-C₄H₉ | n-C₅H₁₁ |
| 165, | 424, | 683, | F | H | n-C₄H₉ | n-C₆H₁₃ |
| 166, | 425, | 684, | F | H | n-C₄H₉ | n-C₇H₁₅ |
| 167, | 426, | 685, | F | F | n-C₄H₉ | n-C₄H₉ |
| 168, | 427, | 686, | F | F | n-C₄H₉ | n-C₅H₁₁ |
| 169, | 428, | 687, | F | F | n-C₄H₉ | n-C₆H₁₃ |
| 170, | 429, | 688, | F | F | n-C₄H₉ | n-C₇H₁₅ |
| 171, | 430, | 689, | CF₃ | H | n-C₄H₉ | CH₃ |
| 172, | 431, | 690, | CF₃ | H | n-C₄H₉ | C₂H₅ |
| 173, | 432, | 691, | CF₃ | H | n-C₄H₉ | n-C₃H₇ |
| 174, | 433, | 692, | CF₃ | H | n-C₄H₉ | n-C₄H₉ |
| 175, | 434, | 693, | CF₃ | H | n-C₄H₉ | n-C₅H₁₁ |
| 176, | 435, | 694, | CF₃ | H | n-C₄H₉ | n-C₆H₁₃ |
| 177, | 436, | 695, | CF₃ | H | n-C₄H₉ | n-C₇H₁₅ |
| 178, | 437, | 696, | -OCF₃ | H | n-C₄H₉ | CH₃ |
| 179, | 438, | 697, | -OCF₃ | H | n-C₄H₉ | C₂H₅ |
| 180, | 439, | 698, | -OCF₃ | H | n-C₄H₉ | n-C₃H₇ |
| 181, | 440, | 699, | -OCF₃ | H | n-C₄H₉ | n-C₄H₉ |
| 182, | 441, | 700, | -OCF₃ | H | n-C₄H₉ | n-C₅H₁₁ |
| 183, | 442, | 701, | -OCF₃ | H | n-C₄H₉ | n-C₆H₁₃ |
| 184, | 443, | 702, | -OCF₃ | H | n-C₄H₉ | n-C₇H₁₅ |
| 185, | 444, | 703, | H | H | n-C₅H₁₁ | n-C₅H₁₁ |
| 186, | 445, | 704, | H | H | n-C₅H₁₁ | n-C₆H₁₃ |
| 187, | 446, | 705, | H | H | n-C₅H₁₁ | n-C₇H₁₅ |
| 188, | 447, | 706, | F | H | n-C₅H₁₁ | CH₃ |
| 189, | 448, | 707, | F | H | n-C₅H₁₁ | C₂H₅ |
| 190, | 449, | 708, | F | H | n-C₅H₁₁ | n-C₃H₇ |
| 191, | 450, | 709, | F | H | n-C₅H₁₁ | n-C₄H₉ |
| 192, | 451, | 710, | F | H | n-C₅H₁₁ | n-C₅H₁₁ |
| 193, | 452, | 711, | F | H | n-C₅H₁₁ | n-C₆H₁₃ |
| 194, | 453, | 712, | F | H | n-C₅H₁₁ | n-C₇H₁₅ |
| 195, | 454, | 713, | F | F | n-C₅H₁₁ | n-C₅H₁₁ |
| 196, | 455, | 714, | F | F | n-C₅H₁₁ | n-C₆H₁₃ |
| 197, | 456, | 715, | F | F | n-C₅H₁₁ | n-C₇H₁₅ |
| 198, | 457, | 716, | CF₃ | H | n-C₅H₁₁ | CH₃ |
| 199, | 458, | 717, | CF₃ | H | n-C₅H₁₁ | C₂H₅ |
| 200, | 459, | 718, | CF₃ | H | n-C₅H₁₁ | n-C₃H₇ |
| 201, | 460, | 719, | CF₃ | H | n-C₅H₁₁ | n-C₄H₉ |
| 202, | 461, | 720, | CF₃ | H | n-C₅H₁₁ | n-C₅H₁₁ |
| 203, | 462, | 721, | CF₃ | H | n-C₅H₁₁ | n-C₆H₁₃ |
| 204, | 463, | 722, | CF₃ | H | n-C₅H₁₁ | n-C₇H₁₅ |
| 205, | 464, | 723, | -OCF₃ | H | n-C₅H₁₁ | CH₃ |
| 206, | 465, | 724, | -OCF₃ | H | n-C₅H₁₁ | C₂H₅ |
| 207, | 466, | 725, | -OCF₃ | H | n-C₅H₁₁ | n-C₃H₇ |
| 208, | 467, | 726, | -OCF₃ | H | n-C₅H₁₁ | n-C₄H₉ |
| 209, | 468, | 727, | -OCF₃ | H | n-C₅H₁₁ | n-C₅H₁₁ |
| 210, | 469, | 728, | -OCF₃ | H | n-C₅H₁₁ | n-C₆H₁₃ |
| 211, | 470, | 729, | -OCF₃ | H | n-C₅H₁₁ | n-C₇H₁₅ |
| 212, | 471, | 730, | H | H | n-C₆H₁₃ | n-C₆H₁₃ |
| 213, | 472, | 731, | H | H | n-C₆H₁₃ | n-C₇H₁₅ |
| 214, | 473, | 732, | F | H | n-C₆H₁₃ | CH₃ |
| 215, | 474, | 733, | F | H | n-C₆H₁₃ | C₂H₅ |
| 216, | 475, | 734, | F | H | n-C₆H₁₃ | n-C₃H₇ |
| 217, | 476, | 735, | F | H | n-C₆H₁₃ | n-C₄H₉ |
| 218, | 477, | 736, | F | H | n-C₆H₁₃ | n-C₅H₁₁ |
| 219, | 478, | 737, | F | H | n-C₆H₁₃ | n-C₆H₁₃ |
| 220, | 479, | 738, | F | H | n-C₆H₁₃ | n-C₇H₁₅ |
| 221, | 480, | 739, | F | F | n-C₆H₁₃ | n-C₆H₁₃ |
| 222, | 481, | 740, | F | F | n-C₆H₁₃ | n-C₇H₁₅ |
| 223, | 482, | 741, | CF₃ | H | n-C₆H₁₃ | CH₃ |
| 224, | 483, | 742, | CF₃ | H | n-C₆H₃ | C₂H₅ |
| 225, | 484, | 743, | CF₃ | H | n-C₆H₁₃ | n-C₃H₇ |
| 226, | 485, | 744, | CF₃ | H | n-C₆H₁₃ | n-C₄H₉ |
| 227, | 486, | 745, | CF₃ | H | n-C₆H₁₃ | n-C₅H₁₁ |
| 228, | 487, | 746, | CF₃ | H | n-C₆H₁₃ | n-C₅H₁₃ |
| 229, | 488, | 747, | CF₃ | H | n-C₆H₁₃ | n-C₇H₁₅ |
| 230, | 489, | 748, | -OCF₃ | H | n-C₆H₁₃ | CH₃ |
| 231, | 490, | 749, | -OCF₃ | H | n-C₆H₁₃ | C₂H₅ |
| 232, | 491, | 750, | -OCF₃ | H | n-C₆H₁₃ | n-C₃H₇ |
| 233, | 492, | 751, | -OCF₃ | H | n-C₆H₁₃ | n-C₄H₉ |
| 234, | 493, | 752, | -OCF₃ | H | n-C₆H₁₃ | n-C₅H₁₁ |
| 235, | 494, | 753, | -OCF₃ | H | n-C₆H₁₃ | n-C₆H₁₃ |
| 236, | 495, | 754, | -OCF₃ | H | n-C₆H₁₃ | n-C₇H₁₅ |
| 237, | 496, | 755, | H | H | n-C₇H₁₅ | n-C₇H₁₅ |
| 238, | 497, | 756, | F | H | n-C₇H₁₅ | CH₃ |
| 239, | 498, | 757, | F | H | n-C₇H₁₅ | C₂H₅ |
| 240, | 499, | 758, | F | H | n-C₇H₁₅ | n-C₃H₇ |
| 241, | 500, | 759, | F | H | n-C₇H₁₅ | n-C₄H₉ |
| 242, | 501, | 760, | F | H | n-C₇H₁₅ | n-C₅H₁₁ |
| 243, | 502, | 761, | F | H | n-C₇H₁₅ | n-C₅H₁₃ |
| 244, | 503, | 762, | F | H | n-C₇H₁₅ | n-C₇H₁₅ |
| 245, | 504, | 763, | F | F | n-C₇H₁₅ | n-C₇H₁₅ |
| 246, | 505, | 764, | CF₃ | H | n-C₇H₁₅ | CH₃ |
| 247, | 506, | 765, | CF₃ | H | n-C₇H₁₅ | C₂H₅ |
| 248, | 507, | 766, | CF₃ | H | n-C₇H₁₅ | n-C₃H₇ |
| 249, | 508, | 767, | CF₃ | H | n-C₇H₁₅ | n-C₄H₉ |
| 250, | 509, | 768, | CF₃ | H | n-C₇H₁₅ | n-C₅H₁₁ |
| 251, | 510, | 769, | CF₃ | H | n-C₇H₁₅ | n-C₆H₁₃ |
| 252, | 511, | 770, | CF₃ | H | n-C₇H₁₅ | n-C₇H₁₅ |
| 253, | 512, | 771, | -OCF₃ | H | n-C₇H₁₅ | CH₃ |
| 254, | 513, | 772, | -OCF₃ | H | n-C₇H₁₅ | C₂H₅ |
| 255, | 514, | 773, | -OCF₃ | H | n-C₇H₁₅ | n-C₃H₇ |
| 256, | 515, | 774, | -OCF₃ | H | n-C₇H₁₅ | n-C₄H₉ |
| 257, | 516, | 775, | -OCF₃ | H | n-C₇H₁₅ | n-C₅H₁₁ |
| 258, | 517, | 776, | -OCF₃ | H | n-C₇H₁₅ | n-C₆H₁₃ |
| 259, | 518, | 777, | -OCF₃ | H | n-C₇H₁₅ | n-C₇H₁₅ |

### Beispiele 778-840

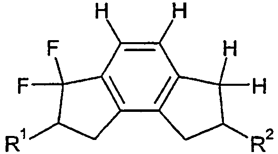

### Beispiele 841-903

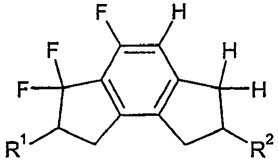

### Beispiele 904-966

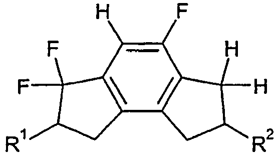

### Beispiele 967-1029

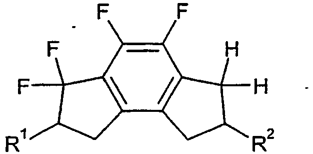

| **Beispiel-Nr.** | | | | **R¹** | **R²** |
|---|---|---|---|---|---|
| 778, | 841, | 904, | 967, | H | CH₃ |
| 779, | 842, | 905, | 968, | H | C₂H₅ |
| 780, | 843, | 906, | 969, | H | n-C₃H₇ |
| 781, | 844, | 907, | 970, | H | n-C₄H₉ |
| 782, | 845, | 908, | 971, | H | n-C₅H₁₁ |
| 783, | 846, | 909, | 972, | H | n-C₆H₁₃ |
| 784, | 847, | 910, | 973, | H | n-C₇H₁₅ |
| 785, | 848, | 911, | 974, | CH₃ | H |
| 786, | 849, | 912, | 975, | CH₃ | CH₃ |
| 787, | 850, | 913, | 976, | CH₃ | C₂H₅ |
| 788, | 851, | 914, | 977, | CH₃ | n-C₃H₇ |
| 789, | 852, | 915, | 978, | CH₃ | n-C₄H₉ |
| 790, | 853, | 916, | 979, | CH₃ | n-C₅H₁₁ |
| 791, | 854, | 917, | 980, | CH₃ | n-C₆H₁₃ |
| 792, | 855, | 918, | 981, | CH₃ | n-C₇H₁₅ |
| 793, | 856, | 919, | 982, | C₂H₅ | H |
| 794, | 857, | 920, | 983, | C₂H₅ | CH₃ |
| 795, | 858, | 921, | 984, | C₂H₅ | C₂H₅ |
| 796, | 859, | 922, | 985, | C₂H₅ | n-C₃H₇ |
| 797, | 860, | 923, | 986, | C₂H₅ | n-C₄H₉ |
| 798, | 861, | 924, | 987, | C₂H₅ | n-C₅H₁₁ |
| 799, | 862, | 925, | 988, | C₂H₅ | n-C₆H₁₃ |
| 800, | 863, | 926, | 989, | C₂H₅ | n-C₇H₁₅ |
| 801, | 864, | 927, | 990, | n-C₃H₇ | H |
| 802, | 865, | 928, | 991, | n-C₃H₇ | CH₃ |
| 803, | 866, | 929, | 992, | n-C₃H₇ | C₂H₅ |
| 804, . | 867, | 930, | 993, | n-C₃H₇ | n-C₃H₇ |
| 805, | 868, | 931, | 994, | n-C₃H₇ | n-C₄H₉ |
| 806, | 869, | 932, | 995, | n-C₃H₇ | n-C₅H₁₁ |
| 807, | 870, | 933, | 996, | n-C₃H₇ | n-C₆H₁₃ |
| 808, | 871, | 934, | 997, | n-C₃H₇ | n-C₇H₁₅ |
| 809, | 872, | 935, | 998, | n-C₄H₉ | H |
| 810, | 873, | 936, | 999, | n-C₄H₉ | CH₃ |
| 811, | 874, | 937, | 1000, | n-C₄H₉ | C₂H₅ |
| 812, | 875, | 938, | 1001, | n-C₄H₉ | n-C₃H₇ |
| 813, | 876, | 939, | 1002, | n-C₄H₉ | n-C₄H₉ |
| 814, | 877, | 940, | 1003, | n-C₄H₉ | n-C₅H₁₁ |
| 815, | 878, | 941, | 1004, | n-C₄H₉ | n-C₆H₁₃ |
| 816, | 879, | 942, | 1005, | n-C₄H₉ | n-C₇H₁₅ |
| 817, | 880, | 943, | 1006, | n-C₅H₁₁ | H |
| 818, | 881, | 944, | 1007, | n-C₅H₁₁ | CH₃ |
| 819, | 882, | 945, | 1008, | n-C₅H₁₁ | C₂H₅ |
| 820, | 883, | 946, | 1009, | n-C₅H₁₁ | n-C₃H₇ |
| 821, | 884, | 947, | 1010, | n-C₅H₁₁ | n-C₄H₉ |
| 822, | 885, | 948, | 1011, | n-C₅H₁₁ | n-C₅H₁₁ |
| 823, | 886, | 949, | 1012, | n-C₅H₁₁ | n-C₆H₁₃ |
| 824, | 887, | 950, | 1013, | n-C₅H₁₁ | n-C₇H₁₅ |
| 825, | 888, | 951, | 1014, | n-C₆H₁₃ | H |
| 826, | 889, | 952, | 1015, | n-C₅H₁₃ | CH₃ |
| 827, | 890, | 953, | 1016, | n-C₆H₁₃ | C₂H₅ |
| 828, | 891, | 954, | 1017, | n-C₆H₁₃ | n-C₃H₇ |
| 829, | 892, | 955, | 1018, | n-C₆H₁₃ | n-C₄H₉ |
| 830, | 893, | 956, | 1019, | n-C₆H₁₃ | n-C₅H₁₁ |
| 831, | 894, | 957, | 1020, | n-C₆H₁₃ | n-C₆H₁₃ |
| 832, | 895, | 958, | 1021, | n-C₆H₁₃ | n-C₇H₁₅ |
| 833, | 896, | 959, | 1022, | n-C₇H₁₅ | H |
| 834, | 897, | 960, | 1023, | n-C₇H₁₅ | CH₃ |
| 835, | 898, | 961, | 1024, | n-C₇H₁₅ | C₂H₅ |
| 836, | 899, | 962, | 1025, | n-C₇H₁₅ | n-C₃H₇ |
| 837, | 900, | 963, | 1026, | n-C₇H₁₅ | n-C₄H₉ |
| 838, | 901, | 964, | 1027, | n-C₇H₁₅ | n-C₅H₁₁ |
| 839, | 902, | 965, | 1028, | n-C₇H₁₅ | n-C₆H₁₃ |
| 840, | 903, | 966, | 1029, | n-C₇H₁₅ | n-C₇H₁₅ |

### Beispiele 1030-1142

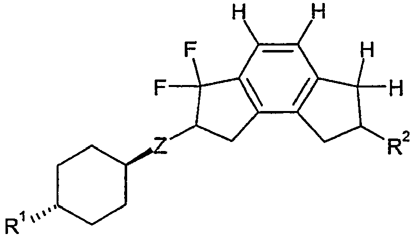

### Beispiele 1143-1255

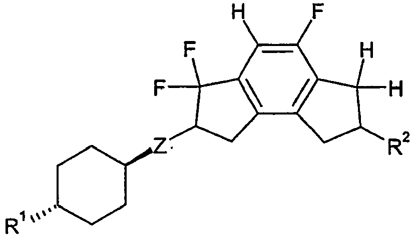

### Beispiele 1256-1368

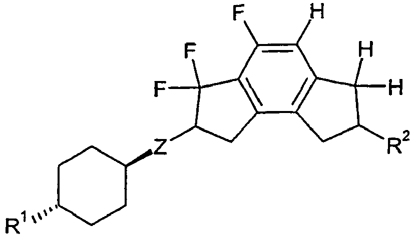

### Beispiele 1369-1481

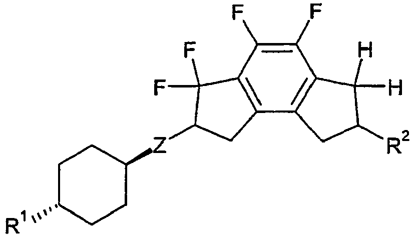

| **Beispiel-Nr.** | | | | **R¹** | **R²** | **Z** |
|---|---|---|---|---|---|---|
| 1030, | 1143, | 1256, | 1369, | CH₃ | H | Bdg. |
| 1031, | 1144, | 1257, | 1370, | CH₃ | CH₃ | Bdg. |
| 1032, | 1145, | 1258, | 1371, | CH₃ | C₂H₅ | Bdg. |
| 1033, | 1146, | 1259, | 1372, | CH₃ | n-C₃H₇ | Bdg. |
| 1034, | 1147, | 1260, | 1373, | CH₃ | n-C₄H₉ | Bdg. |
| 1035, | 1148, | 1261, | 1374, | CH₃ | n-C₅H₁₁ | Bdg. |
| 1036, | 1149, | 1262, | 1375, | CH₃ | n-C₆H₁₃ | Bdg. |
| 1037, | 1150, | 1263, | 1376, | CH₃ | n-C₇H₁₅ | Bdg. |
| 1038, | 1151, | 1264, | 1377, | CH₃ | H | CF₂CF₂ |
| 1039, | 1152, | 1265, | 1378, | CH₃ | CH₃ | CF₂CF₂ |
| 1040, | 1153, | 1266, | 1379, | CH₃ | C₂H₅ | CF₂CF₂ |
| 1041, | 1154, | 1267, | 1380, | CH₃ | n-C₃H₇ | CF₂CF₂ |
| 1042, | 1155, | 1268, | 1381, | CH₃ | n-C₄H₉ | CF₂CF₂ |
| 1043, | 1156, | 1269, | 1382, | CH₃ | n-C₅H₁₁ | CF₂CF₂ |
| 1044, | 1157, | 1270, | 1383, | CH₃ | n-C₆H₁₃ | CF₂CF₂ |
| 1045, | 1158, | 1271, | 1384, | CH₃ | n-C₇H₁₅ | CF₂CF₂ |
| 1046, | 1159, | 1272, | 1385, | C₂H₅ | H | Bdg. |
| 1047, | 1160, | 1273, | 1386, | C₂H₅ | CH₃ | Bdg. |
| 1048, | 1161, | 1274, | 1387, | C₂H₅ | C₂H₅ | Bdg. |
| 1049, | 1162, | 1275, | 1388, | C₂H₅ | n-C₃H₇ | Bdg. |
| 1050, | 1163, | 1276, | 1389, | C₂H₅ | n-C₄H₉ | Bdg. |
| 1051, | 1164, | 1277, | 1390, | C₂H₅ | n-C₅H₁₁ | Bdg. |
| 1052, | 1165, | 1278, | 1391, | C₂H₅ | n-C₆H₁₃ | Bdg. |
| 1053, | 1166, | 1279, | 1392, | C₂H₅ | n-C₇H₁₅ | Bdg. |
| 1054, | 1167, | 1280, | 1393, | C₂H₅ | H | CF₂CF₂ |
| 1055, | 1168, | 1281, | 1394, | C₂H₅ | CH₃ | CF₂CF₂ |
| 1056, | 1169, | 1282, | 1395, | C₂H₅ | C₂H₅ | CF₂CF₂ |
| 1057, | 1170, | 1283, | 1396, | C₂H₅ | n-C₃H₇ | CF₂CF₂ |
| 1058, | 1171, | 1284, | 1397, | C₂H₅ | n-C₄H₉ | CF₂CF₂ |
| 1059, | 1172, | 1285, | 1398, | C₂H₅ | n-C₅H₁₁ | CF₂CF₂ |
| 1060, | 1173, | 1286, | 1399, | C₂H₅ | n-C₆H₁₃ | CF₂CF₂ |
| 1061, | 1174, | 1287, | 1400, | C₂H₅ | n-C₇H₁₅ | CF₂CF₂ |
| 1062, | 1175, | 1288, | 1401, | n-C₃H₇ | H | Bdg. |
| 1063, | 1176, | 1289, | 1402, | n-C₃H₇ | CH₃ | Bdg. |
| 1064, | 1177, | 1290, | 1403, | n-C₃H₇ | C₂H₅ | Bdg. |
| 1065, | 1178, | 1291, | 1404, | n-C₃H₇ | n-C₃H₇ | Bdg. |
| 1066, | 1179, | 1292, | 1405, | n-C₃H₇ | n-C₄H₉ | Bdg. |
| 1067, | 1180, | 1293, | 1406, | n-C₃H₇ | n-C₅H₁₁ | Bdg. |
| 1068, | 1181, | 1294, | 1407, | n-C₃H₇ | n-C₆H₁₃ | Bdg. |
| 1069, | 1182, | 1295, | 1408, | n-C₃H₇ | n-C₇H₁₅ | Bdg. |
| 1070, | 1183, | 1296, | 1409, | n-C₃H₇ | H | CF₂CF₂ |
| 1071, | 1184, | 1297, | 1410, | n-C₃H₇ | CH₃ | CF₂CF₂ |
| 1072, | 1185, | 1298, | 1411, | n-C₃H₇ | C₂H₅ | CF₂CF₂ |
| 1073, | 1186, | 1299, | 1412, | n-C₃H₇ | n-C₃H₇ | CF₂CF₂ |
| 1074, | 1187, | 1300, | 1413, | n-C₃H₇ | n-C₄H₉ | CF₂CF₂ |
| 1075, | 1188, | 1301, | 1414, | n-C₃H₇ | n-C₅H₁₁ | CF₂CF₂ |
| 1076, | 1189, | 1302, | 1415, | n-C₃H₇ | n-C₆H₁₃ | CF₂CF₂ |
| 1077, | 1190, | 1303, | 1416, | n-C₃H₇ | n-C₇H₁₅ | CF₂CF₂ |
| 1078, | 1191, | 1304, | 1417, | n-C₄H₉ | H | Bdg. |
| 1079, | 1192, | 1305, | 1418, | n-C₄H₉ | CH₃ | Bdg. |
| 1080, | 1193, | 1306, | 1419, | n-C₄H₉ | C₂H₅ | Bdg. |
| 1081, | 1194, | 1307, | 1420, | n-C₄H₉ | n-C₃H₇ | Bdg. |
| 1082, | 1195, | 1308, | 1421, | n-C₄H₉ | n-C₄H₉ | Bdg. |
| 1083, | 1196, | 1309, | 1422, | n-C₄H₉ | n-C₅H₁₁ | Bdg. |
| 1084, | 1197, | 1310, | 1423, | n-C₄H₉ | n-C₆H₁₃ | Bdg. |
| 1085, | 1198, | 1311, | 1424, | n-C₄H₉ | n-C₇H₁₅ | Bdg. |
| 1086, | 1199, | 1312, | 1425, | n-C₄H₉ | H | CF₂CF₂ |
| 1087, | 1200, | 1313, | 1426, | n-C₄H₉ | CH₃ | CF₂CF₂ |
| 1088, | 1201, | 1314, | 1427, | n-C₄H₉ | C₂H₅ | CF₂CF₂ |
| 1089, | 1202, | 1315, | 1428, | n-C₄H₉ | n-C₃H₇ | CF₂CF₂ |
| 1090, | 1203, | 1316, | 1429, | n-C₄H₉ | n-C₄H₉ | CF₂CF₂ |
| 1091, | 1204, | 1317, | 1430, | n-C₄H₉ | n-C₅H₁₁ | CF₂CF₂ |
| 1092, | 1205, | 1318, | 1431, | n-C₄H₉ | n-C₆H₁₃ | CF₂CF₂ |
| 1093, | 1206, | 1319, | 1432, | n-C₄H₉ | n-C₇H₁₅ | CF₂CF₂ |
| 1094, | 1207, | 1320, | 1433, | n-C₅H₁₁ | n-C₇H₁₅ | Bdg. |
| 1095, | 1208, | 1321, | 1434, | n-C₅H₁₁ | H | Bdg. |
| 1096, | 1209, | 1322, | 1435, | n-C₅H₁₁ | CH₃ | Bdg. |
| 1097, | 1210, | 1323, | 1436, | n-C₅H₁₁ | C₂H₅ | Bdg. |
| 1098, | 1211, | 1324, | 1437, | n-C₅H₁₁ | n-C₃H₇ | Bdg. |
| 1099, | 1212, | 1325, | 1438, | n-C₅H₁₁ | n-C₄H₉ | Bdg. |
| 1100, | 1213, | 1326, | 1439, | n-C₅H₁₁ | n-C₅H₁₁ | Bdq. |
| 1101, | 1214, | 1327, | 1440, | n-C₅H₁₁ | n-C₆H₁₃ | Bdq. |
| 1102, | 1215, | 1328, | 1441, | n-C₅H₁₁ | n-C₇H₁₅ | Bdg. |
| 1103, | 1216, | 1329, | 1442, | n-C₅H₁₁ | H | CF₂CF₂ |
| 1104, | 1217, | 1330, | 1443, | n-C₅H₁₁ | CH₃ | CF₂CF₂ |
| 1105, | 1218, | 1331, | 1444, | n-C₅H₁₁ | C₂H₅ | CF₂CF₂ |
| 1106, | 1219, | 1332, | 1445, | n-C₅H₁₁ | n-C₃H₇ | CF₂CF₂ |
| 1107, | 1220, | 1333, | 1446, | n-C₅H₁₁ | n-C₄H₉ | CF₂CF₂ |
| 1108, | 1221, | 1334, | 1447, | n-C₅H₁₁ | n-C₅H₁₁ | CF₂CF₂ |
| 1109, | 1222, | 1335, | 1448, | n-C₅H₁₁ | n-C₆H₁₃ | CF₂CF₂ |
| 1110, | 1223, | 1336, | 1449, | n-C₅H₁₁ | n-C₇H₁₅ | CF₂CF₂ |
| 1111, | 1224, | 1337, | 1450, | n-C₆H₁₃ | H | Bdg. |
| 1112, | 1225, | 1338, | 1451, | n-C₆H₁₃ | CH₃ | Bdg. |
| 1113, | 1226, | 1339, | 1452, | n-C₆H₁₃ | C₂H₅ | Bdg. |
| 1114, | 1227, | 1340, | 1453, | n-C₆H₁₃ | n-C₃H₇ | Bdg. |
| 1115, | 1228, | 1341, | 1454, | n-C₆H₁₃ | n-C₄H₉ | Bdg. |
| 1116, | 1229, | 1342, | 1455, | n-C₆H₁₃ | n-C₅H₁₁ | Bdg. |
| 1117, | 1230, | 1343, | 1456, | n-C₆H₁₃ | n-C₆H₁₃ | Bdg. |
| 1118, | 1231, | 1344, | 1457, | n-C₆H₁₃ | n-C₇H₁₅ | Bdg. |
| 1119, | 1232, | 1345, | 1458, | n-C₆H₁₃ | H | CF₂CF₂ |
| 1120, | 1233, | 1346, | 1459, | n-C₆H₁₃ | CH₃ | CF₂CF₂ |
| 1121, | 1234, | 1347, | 1460, | n-C₆H₁₃ | C₂H₅ | CF₂CF₂ |
| 1122, | 1235, | 1348, | 1461, | n-C₆H₁₃ | n-C₃H₇ | CF₂CF₂ |
| 1123, | 1236, | 1349, | 1462, | n-C₆H₁₃ | n-C₄H₉ | CF₂CF₂ |
| 1124, | 1237, | 1350, | 1463, | n-C₆H₁₃ | n-C₅H₁₁ | CF₂CF₂ |
| 1125, | 1238, | 1351, | 1464, | n-C₆H₁₃ | n-C₆H₁₃ | CF₂CF₂ |
| 1126, | 1239, | 1352, | 1465, | n-C₆H₁₃ | n-C₇H₁₅ | CF₂CF₂ |
| 1127, | 1240, | 1353, | 1466, | n-C₇H₁₅ | H | Bdg. |
| 1128, | 1241, | 1354, | 1467, | n-C₇H₁₅ | CH₃ | Bdg. |
| 1129, | 1242, | 1355, | 1468, | n-C₇H₁₅ | C₂H₅ | Bdg. |
| 1130, | 1243, | 1356, | 1469, | n-C₇H₁₅ | n-C₃H₇ | Bdg. |
| 1131, | 1244, | 1357, | 1470, | n-C₇H₁₅ | n-C₄H₉ | Bdg. |
| 1132, | 1245, | 1358, | 1471, | n-C₇H₁₅ | n-C₅H₁₁ | Bdg. |
| 1133, | 1246, | 1359, | 1472, | n-C₇H₁₅ | n-C₆H₁₃ | Bdg. |
| 1134, | 1247, | 1360, | 1473, | n-C₇H₁₅ | n-C₇H₁₅ | Bdg. |
| 1135, | 1248, | 1361, | 1474, | n-C₇H₁₅ | H | CF₂CF₂ |
| 1136, | 1249, | 1362, | 1475, | n-C₇H₁₅ | CH₃ | CF₂CF₂ |
| 1137, | 1250, | 1363, | 1476, | n-C₇H₁₅ | C₂H₅ | CF₂CF₂ |
| 1138, | 1251, | 1364, | 1477, | n-C₇H₁₅ | n-C₃H₇ | CF₂CF₂ |
| 1139, | 1252, | 1365, | 1478, | n-C₇H₁₅ | n-C₄H₉ | CF₂CF₂ |
| 1140, | 1253, | 1366, | 1479, | n-C₇H₁₅ | n-C₅H₁₁ | CF₂CF₂ |
| 1141, | 1254, | 1367, | 1480, | n-C₇H₁₅ | n-C₆H₁₃ | CF₂CF₂ |
| 1142, | 1255, | 1368, | 1481, | n-C₇H₁₅ | n-C₇H₁₅ | CF₂CF₂ |

### Beispiele 1482-1594

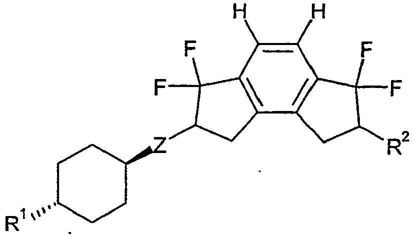

### Beispiele 1595-1707

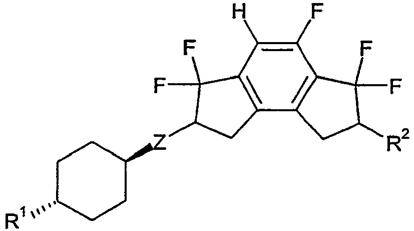

### Beispiele 1708-1820

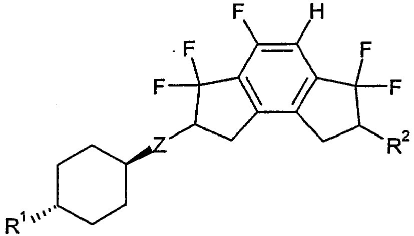

### Beispiele 1821-1933

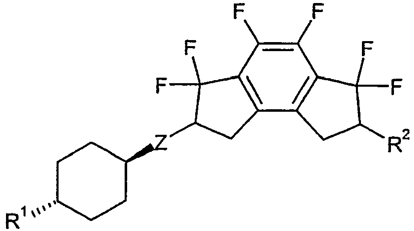

| **Beispiel-Nr.** | | | | **R¹** | **R²** | **Z** |
|---|---|---|---|---|---|---|
| 1482, | 1595, | 1708, | 1821, | CH₃ | H | Bdg. |
| 1483, | 1596, | 1709, | 1822, | CH₃ | CH₃ | Bdg. |
| 1484, | 1597, | 1710, | 1823, | CH₃ | C₂H₅ | Bdg. |
| 1485, | 1598, | 1711, | 1824, | CH₃ | n-C₃H₇ | Bdg. |
| 1486, | 1599, | 1712, | 1825, | CH₃ | n-C₄H₉ | Bdg. |
| 1487, | 1600, | 1713, | 1826, | CH₃ | n-C₅H₁₁ | Bdg. |
| 1488, | 1601, | 1714, | 1827, | CH₃ | n-C₆H₁₃ | Bdg. |
| 1489, | 1602, | 1715, | 1828, | CH₃ | n-C₇H₁₅ | Bdg. |
| 1490, | 1603, | 1716, | 1829, | CH₃ | H | **CF₂CF₂** |
| 1491, | 1604, | 1717, | 1830, | CH₃ | CH₃ | CF₂CF₂ |
| 1492, | 1605, | 1718, | 1831, | CH₃ | C₂H₅ | CF₂CF₂ |
| 1493, | 1606, | 1719, | 1832, | CH₃ | n-C₃H₇ | CF₂CF₂ |
| 1494, | 1607, | 1720, | 1833, | CH₃ | n-C₄H₉ | CF₂CF₂ |
| 1495, | 1608, | 1721, | 1834, | CH₃ | n-C₅H₁₁ | CF₂CF₂ |
| 1496, | 1609, | 1722, | 1835, | CH₃ | n-C₆H₁₃ | CF₂CF₂ |
| 1497, | 1610, | 1723, | 1836, | CH₃ | n-C₇H₁₅ | CF₂CF₂ |
| 1498, | 1611, | 1724, | 1837, | C₂H₅ | H | Bdq. |
| 1499, | 1612, | 1725, | 1838, | C₂H₅ | CH₃ | Bdg. |
| 1500, | 1613, | 1726, | 1839, | C₂H₅ | C₂H₅ | Bdg. |
| 1501, | 1614, | 1727, | 1840, | C₂H₅ | n-C₃H₇ | Bdg. |
| 1502, | 1615, | 1728, | 1841, | C₂H₅ | n-C₄H₉ | Bdg. |
| 1503, | 1616, | 1729, | 1842, | C₂H₅ | n-C₅H₁₁ | Bdg. |
| 1504, | 1617, | 1730, | 1843, | C₂H₅ | n-C₆H₁₃ | Bdg. |
| 1505, | 1618, | 1731, | 1844, | C₂H₅ | n-C₇H₁₅ | Bdg. |
| 1506, | 1619, | 1732, | 1845, | C₂H₅ | H | CF₂CF₂ |
| 1507, | 1620, | 1733, | 1846, | C₂H₅ | CH₃ | CF₂CF₂ |
| 1508, | 1621, | 1734, | 1847, | C₂H₅ | C₂H₅ | CF₂CF₂ |
| 1509, | 1622, | 1735, | 1848, | C₂H₅ | n-C₃H₇ | CF₂CF₂ |
| 1510, | 1623, | 1736, | 1849, | C₂H₅ | n-C₄H₉ | CF₂CF₂ |
| 1511, | 1624, | 1737, | 1850, | C₂H₅ | n-C₅H₁₁ | CF₂CF₂ |
| 1512, | 1625, | 1738, | 1851, | C₂H₅ | n-C₆H₁₃ | CF₂CF₂ |
| 1513, | 1626, | 1739, | 1852, | C₂H₅ | n-C₇H₁₅ | CF₂CF₂ |
| 1514, | 1627, | 1740, | 1853, | n-C₃H₇ | H | Bdg. |
| 1515, | 1628, | 1741, | 1854, | n-C₃H₇ | CH₃ | Bdg. |
| 1516, | 1629, | 1742, | 1855, | n-C₃H₇ | C₂H₅ | Bdg. |
| 1517, | 1630, | 1743, | 1856, | n-C₃H₇ | n-C₃H₇ | Bdg. |
| 1518, | 1631, | 1744, | 1857, | n-C₃H₇ | n-C₄H₉ | Bdg. |
| 1519, | 1632, | 1745, | 1858, | n-C₃H₇ | n-C₅H₁₁ | Bdg. |
| 1520, | 1633, | 1746, | 1859, | n-C₃H₇ | n-C₆H₁₃ | Bdg. |
| 1521, | 1634, | 1747, | 1860, | n-C₃H₇ | n-C₇H₁₅ | Bdg. |
| 1522, | 1635, | 1748, | 1861, | n-C₃H₇ | H | CF₂CF₂ |
| 1523, | 1636, | 1749, | 1862, | n-C₃H₇ | CH₃ | CF₂CF₂ |
| 1524, | 1637, | 1750, | 1863, | n-C₃H₇ | C₂H₅ | CF₂CF₂ |
| 1525, | 1638, | 1751, | 1864, | n-C₃H₇ | n-C₃H₇ | CF₂CF₂ |
| 1526, | 1639, | 1752, | 1865, | n-C₃H₇ | n-C₄H₉ | CF₂CF₂ |
| 1527, | 1640, | 1753, | 1866, | n-C₃H₇ | n-C₅H₁₁ | CF₂CF₂ |
| 1528, | 1641, | 1754, | 1867, | n-C₃H₇ | n-C₆H₁₃ | CF₂CF₂ |
| 1529, | 1642, | 1755, | 1868, | n-C₃H₇ | n-C₇H₁₅ | CF₂CF₂ |
| 1530, | 1643, | 1756, | 1869, | n-C₄H₉ | H | Bdg. |
| 1531, | 1644, | 1757, | 1870, | n-C₄H₉ | CH₃ | Bdg. |
| 1532, | 1645, | 1758, | 1871, | n-C₄H₉ | C₂H₅ | Bdg. |
| 1533, | 1646, | 1759, | 1872, | n-C₄H₉ | n-C₃H₇ | Bdg. |
| 1534, | 1647, | 1760, | 1873, | n-C₄H₉ | n-C₄H₉ | Bdg. |
| 1535, | 1648, | 1761, | 1874, | n-C₄H₉ | n-C₅H₁₁ | Bdg. |
| 1536, | 1649, | 1762, | 1875, | n-C₄H₉ | n-C₆H₁₃ | Bdg. |
| 1537, | 1650, | 1763, | 1876, | n-C₄H₉ | n-C₇H₁₅ | Bdg. |
| 1538, | 1651, | 1764, | 1877, | n-C₄H₉ | H | CF₂CF₂ |
| 1539, | 1652, | 1765, | 1878, | n-C₄H₉ | CH₃ | CF₂CF₂ |
| 1540, | 1653, | 1766, | 1879, | n-C₄H₉ | C₂H₅ | CF₂CF₂ |
| 1541, | 1654, | 1767, | 1880, | n-C₄H₉ | n-C₃H₇ | CF₂CF₂ |
| 1542, | 1655, | 1768, | 1881, | n-C₄H₉ | n-C₄H₉ | CF₂CF₂ |
| 1543, | 1656, | 1769, | 1882, | n-C₄H₉ | n-C₅H₁₁ | CF₂CF₂ |
| 1544, | 1657, | 1770, | 1883, | n-C₄H₉ | n-C₆H₁₃ | CF₂CF₂ |
| 1545, | 1658, | 1771, | 1884, | n-C₄H₉ | n-C₇H₁₅ | CF₂CF₂ |
| 1546, | 1659, | 1772, | 1885, | n-C₅H₁₁ | n-C₇H₁₅ | Bdg. |
| 1547, | 1660, | 1773, | 1886, | n-C₅H₁₁ | H | Bdq. |
| 1548, | 1661, | 1774, | 1887, | n-C₅H₁₁ | CH₃ | Bdg. |
| 1549, | 1662, | 1775, | 1888, | n-C₅H₁₁ | C₂H₅ | Bdg. |
| 1550, | 1663, | 1776, | 1889, | n-C₅H₁₁ | n-C₃H₇ | Bdg. |
| 1551, | 1664, | 1777, | 1890, | n-C₅H₁₁ | n-C₄H₉ | Bdg. |
| 1552, | 1665, | 1778, | 1891, | n-C₅H₁₁ | n-C₅H₁₁ | Bdg. |
| 1553, | 1666, | 1779, | 1892, | n-C₅H₁₁ | n-C₆H₁₃ | Bdg. |
| 1554, | 1667, | 1780, | 1893, | n-C₅H₁₁ | n-C₇H₁₅ | Bdg. |
| 1555, | 1668, | 1781, | 1894, | n-C₅H₁₁ | H | CF₂CF₂ |
| 1556, | 1669, | 1782, | 1895, | n-C₅H₁₁ | CH₃ | CF₂CF₂ |
| 1557, | 1670, | 1783, | 1896, | n-C₅H₁₁ | C₂H₅ | CF₂CF₂ |
| 1558, | 1671, | 1784, | 1897, | n-C₅H₁₁ | n-C₃H₇ | CF₂CF₂ |
| 1559, | 1672, | 1785, | 1898, | n-C₅H₁₁ | n-C₄H₉ | CF₂CF₂ |
| 1560, | 1673, | 1786, | 1899, | n-C₅H₁₁ | n-C₅H₁₁ | CF₂CF₂ |
| 1561, | 1674, | 1787, | 1900, | n-C₅H₁₁ | n-C₆H₁₃ | CF₂CF₂ |
| 1562, | 1675, | 1788, | 1901, | n-C₅H₁₁ | n-C₇H₁₅ | CF₂CF₂ |
| 1563, | 1676, | 1789, | 1902, | n-C₆H₁₃ | H | Bdg. |
| 1564, | 1677, | 1790, | 1903, | n-C₆H₁₃ | CH₃ | Bdg. |
| 1565, | 1678, | 1791, | 1904, | n-C₆H₁₃ | C₂H₅ | Bdg. |
| 1566, | 1679, | 1792, | 1905, | n-C₆H₁₃ | n-C₃H₇ | Bdg. |
| 1567, | 1680, | 1793, | 1906, | n-C₆H₁₃ | n-C₄H₉ | Bdq. |
| 1568, | 1681, | 1794, | 1907, | n-C₆H₁₃ | n-C₅H₁₁ | Bdg. |
| 1569, | 1682, | 1795, | 1908, | n-C₆H₁₃ | n-C₆H₁₃ | Bdg. |
| 1570, | 1683, | 1796, | 1909, | n-C₆H₁₃ | n-C₇H₁₅ | Bdg. |
| 1571, | 1684, | 1797, | 1910, | n-C₆H₁₃ | H | CF₂CF₂ |
| 1572, | 1685, | 1798, | 1911, | n-C₆H₁₃ | CH₃ | CF₂CF₂ |
| 1573, | 1686, | 1799, | 1912, | n-C₆H₁₃ | C₂H₅ | CF₂CF₂ |
| 1574, | 1687, | 1800, | 1913, | n-C₆H₁₃ | n-C₃H₇ | CF₂CF₂ |
| 1575, | 1688, | 1801, | 1914, | n-C₆H₁₃ | n-C₄H₉ | CF₂CF₂ |
| 1576, | 1689, | 1802, | 1915, | n-C₆H₁₃ | n-C₅H₁₁ | CF₂CF₂ |
| 1577, | 1690, | 1803, | 1916, | n-C₆H₁₃ | n-C₆H₁₃ | CF₂CF₂ |
| 1578, | 1691, | 1804, | 1917, | n-C₆H₁₃ | n-C₇H₁₅ | CF₂CF₂ |
| 1579, | 1692, | 1805, | 1918, | n-C₇H₁₅ | H | Bdg. |
| 1580, | 1693, | 1806, | 1919, | n-C₇H₁₅ | CH₃ | Bdg. |
| 1581, | 1694, | 1807, | 1920, | n-C₇H₁₅ | C₂H₅ | Bdg. |
| 1582, | 1695, | 1808, | 1921, | n-C₇H₁₅ | n-C₃H₇ | Bdg. |
| 1583, | 1696, | 1809, | 1922, | n-C₇H₁₅ | n-C₄H₉ | Bdg. |
| 1584, | 1697, | 1810, | 1923, | n-C₇H₁₅ | r1-C₅H₁₁ | Bdg. |
| 1585, | 1698, | 1811, | 1924, | n-C₇H₁₅ | n-C₆H₁₃ | Bdg. |
| 1586, | 1699, | 1812, | 1925, | n-C₇H₁₅ | n-C₇H₁₅ | Bdg. |
| 1587, | 1700, | 1813, | 1926, | n-C₇H₁₅ | H | CF₂CF₂ |
| 1588, | 1701, | 1814, | 1927, | n-C₇H₁₅ | CH₃ | CF₂CF₂ |
| 1589, | 1702, | 1815, | 1928, | n-C₇H₁₅ | C₂H₅ | CF₂CF₂ |
| 1590, | 1703, | 1816, | 1929, | n-C₇H₁₅ | n-C₃H₇ | CF₂CF₂ |
| 1591, | 1704, | 1817, | 1930, | n-C₇H₁₅ | n-C₄H₉ | CF₂CF₂ |
| 1592, | 1705, | 1818, | 1931, | n-C₇H₁₅ | n-C₅H₁₁ | CF₂CF₂ |
| 1593, | 1706, | 1819, | 1932, | n-C₇H₁₅ | n-C₆H₁₃ | CF₂CF₂ |
| 1594, | 1707, | 1820, | 1933, | n-C₇H₁₅ | n-C₇H₁₅ | CF₂CF₂ |

### Beispiele 1934-1958

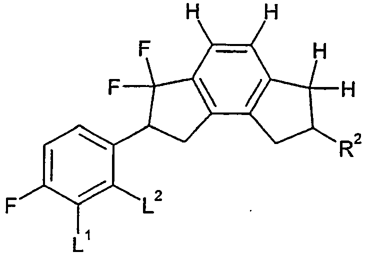

### Beispiele 1959-1983

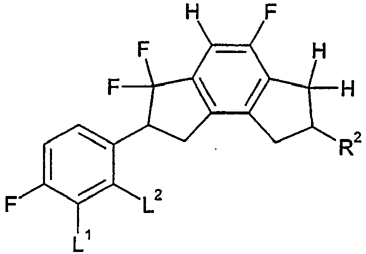

### Beispiele 1984-2008

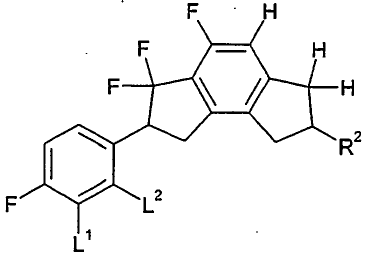

### Beispiele 2009-2033

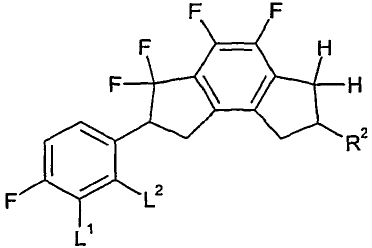

| **Beispiel-Nr.** | | | | **L¹** | **L²** | **R²** |
|---|---|---|---|---|---|---|
| 1934, | 1959, | 1984, | 2009, | H | H | H |
| 1935, | 1960, | 1985, | 2010, | F | H | H |
| 1936, | 1961, | 1986, | 2011, | F | F | H |
| 1937, | 1962, | 1987, | 2012, | F | F | H |
| 1938, | 1963, | 1988, | 2013, | H | H | CH₃ |
| 1939, | 1964, | 1989, | 2014, | F | H | CH₃ |
| 1940, | 1965, | 1990, | 2015, | F | F | CH₃ |
| 1941, | 1966, | 1991, | 2016, | H | H | C₂H₅ |
| 1942, | 1967, | 1992, | 2017, | F | H | C₂H₅ |
| 1943, | 1968, | 1993, | 2018, | F | F | C₂H₅ |
| 1944, | 1969, | 1994, | 2019, | H | H | n-C₃H₇ |
| 1945, | 1970, | 1995, | 2020, | F | H | n-C₃H₇ |
| 1946, | 1971, | 1996, | 2021, | F | F | n-C₃H₇ |
| 1947, | 1972, | 1997, | 2022, | H | H | n-C₄H₉ |
| 1948, | 1973, | 1998, | 2023, | F | H | n-C₄H₉ |
| 1949, | 1974, | 1999, | 2024, | F | F | n-C₄H₉ |
| 1950, | 1975, | 2000, | 2025, | H | H | n-C₅H₁₁ |
| 1951, | 1976, | 2001, | 2026, | F | H | n-C₅H₁, |
| 1952, | 1977, | 2002, | 2027, | F | F | n-C₅H₁₁ |
| 1953, | 1978, | 2003, | 2028, | H | H | n-C₆H₁₃ |
| 1954, | 1979, | 2004, | 2029, | F | H | n-C₆H₁₃ |
| 1955, | 1980, | 2005, | 2030, | F | F | n-C₆H₁₃ |
| 1956, | 1981, | 2006, | 2031, | H | H | n-C₇H₁₅ |
| 1957, | 1982, | 2007, | 2032, | F | H | n-C₇H₁₅ |
| 1958, | 1983, | 2008, | 2033, | F | F | n-C₇H₁₅ |

### Beispiele 2034-2058

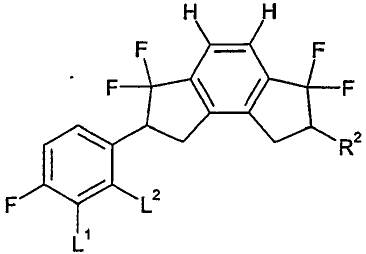

### Beispiele 2059-2083

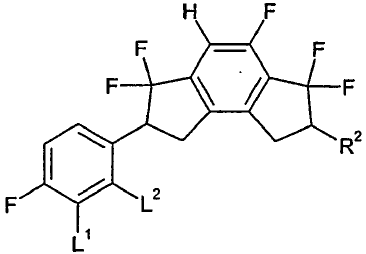

### Beispiele 2084-2108

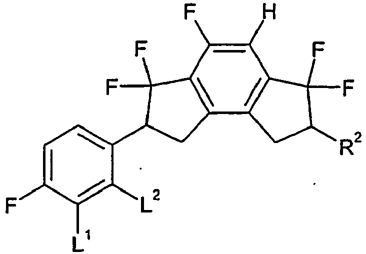

### Beispiele 2109-2133

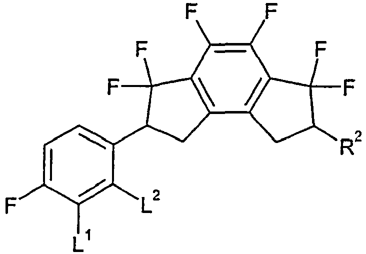

| **Beispiel-Nr.** | | | | **L¹** | **L²** | **R²** |
|---|---|---|---|---|---|---|
| 2034, | 2059, | 2084, | 2109, | H | H | H |
| 2035, | 2060, | 2085, | 2110, | F | H | H |
| 2036, | 2061, | 2086, | 2111, | F | F | H |
| 2037, | 2062, | 2087, | 2112, | F | F | H |
| 2038, | 2063, | 2088, | 2113, | H | H | CH₃ |
| 2039, | 2064, | 2089, | 2114, | F | H | CH₃ |
| 2040, | 2065, | 2090, | 2115, | F | F | CH₃ |
| 2041, | 2066, | 2091, | 2116, | H | H | C₂H₅ |
| 2042, | 2067, | 2092, | 2117, | F | H | C₂H₅ |
| 2043, | 2068, | 2093, | 2118, | F | F | C₂H₅ |
| 2044, | 2069, | 2094, | 2119, | H | H | n-C₃H₇ |
| 2045, | 2070, | 2095, | 2120, | F | H | n-C₃H₇ |
| 2046, | 2071, | 2096, | 2121, | F | F | n-C₃H₇ |
| 2047, | 2072, | 2097, | 2122, | H | H | n-C₄H₉ |
| 2048, | 2073, | 2098, | 2123, | F | H | n-C₄H₉ |
| 2049, | 2074, | 2099, | 2124, | F | F | n-C₄H₉ |
| 2050, | 2075, | 2100, | 2125, | H | H | n-C₅H₁₁ |
| 2051, | 2076, | 2101, | 2126, | F | H | n-C₅H₁₁ |
| 2052, | 2077, | 2102, | 2127, | F | F | n-C₅H₁₁ |
| 2053, | 2078, | 2103, | 2128, | H | H | n-C₆H₁₃ |
| 2054, | 2079, | 2104, | 2129, | F | H | n-C₆H₁₃ |
| 2055, | 2080, | 2105, | 2130, | F | F | n-C₆H₁₃ |
| 2056, | 2081, | 2106, | 2131, | H | H | n-C₇H₁₅ |
| 2057, | 2082, | 2107, | 2132, | F | H | n-C₇H₁₅ |
| 2058, | 2083, | 2108, | 2133, | F | F | n-C₇H₁₅ |

### Beispiele 2134-2189

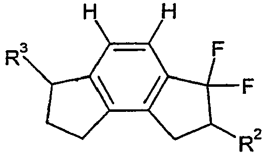

### Beispiele 2190-2245

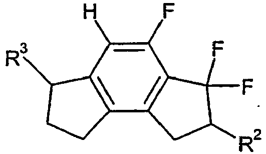

### Beispiele 2246-2301

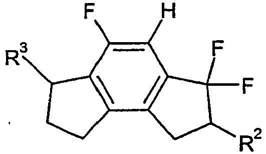

### Beispiele 2302-2357

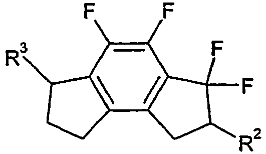

| **Beispiel-Nr.** | | | | **R²** | **R³** |
|---|---|---|---|---|---|
| 2134, | 2190, | 2246, | 2302, | H | CH₃ |
| 2135, | 2191, | 2247, | 2303, | H | C₂H₅ |
| 2136, | 2192, | 2248, | 2304, | H | P-C₃H₇ |
| 2137, | 2193, | 2249, | 2305, | H | n-C₄H₄ |
| 2138, | 2194, | 2250, | 2306, | H | n-C₅H₁₁ |
| 2139, | 2195, | 2251, | 2307, | H | n-C₆H₁₃ |
| 2140, | 2196, | 2252, | 2308, | H | n-C₇H₁₅ |
| 2141, | 2197, | 2253, | 2309, | CH₃ | CH₃ |
| 2142, | 2198, | 2254, | 2310, | CH₃ | C₂H₅ |
| 2143, | 2199, | 2255, | 2311, | CH₃ | n-C₃H₇ |
| 2144, | 2200, | 2256, | 2312, | CH₃ | n-C₄H₉ |
| 2145, | 2201, | 2257, | 2313, | CH₃ | n-C₅H₁₁ |
| 2146, | 2202, | 2258, | 2314, | CH₃ | n-C₆H₁₃ |
| 2147, | 2203, | 2259, | 2315, | CH₃ | n-C₇H₁₅ |
| 2148, | 2204, | 2260, | 2316, | C₂H₅ | CH₃ |
| 2149, | 2205, | 2261, | 2317, | C₂H₅ | C₂H₅ |
| 2150, | 2206, | 2262, | 2318, | C₂H₅ | n-C₃H₇ |
| 2151, | 2207, | 2263, | 2319, | C₂H₅ | n-C₄H₉ |
| 2152, | 2208, | 2264, | 2320, | C₂H₅ | n-C₅H₁₁ |
| 2153, | 2209, | 2265, | 2321, | C₂H₅ | n-C₆H₁₃ |
| 2154, | 2210, | 2266, | 2322, | C₂H₅ | n-C₇H₁₅ |
| 2155, | 2211, | 2267, | 2323, | n-C₃H₇ | CH₃ |
| 2156, | 2212, | 2268, | 2324, | n-C₃H₇ | C₂H₅ |
| 2157, | 2213, | 2269, | 2325, | n-C₃H₇ | n-C₃H₇ |
| 2158, | 2214, | 2270, | 2326, | n-C₃H₇ | n-C₄H₉ |
| 2159, | 2215, | 2271, | 2327, | n-C₃H₇ | n-C₅H₁₁ |
| 2160, | 2216, | 2272, | 2328, | n-C₃H₇ | n-C₆H₁₃ |
| 2161, | 2217, | 2273, | 2329, | n-C₃H₇ | n-C₇H₁₅ |
| 2162, | 2218, | 2274, | 2330, | n-C₄H₉ | CH₃ |
| 2163, | 2219, | 2275, | 2331, | n-C₄H₉ | C₂H₅ |
| 2164, | 2220, | 2276, | 2332, | n-C₄H₉ | n-C₃H₇ |
| 2165, | 2221, | 2277, | 2333, | n-C₄H₉ | n-C₄H₉ |
| 2166, | 2222, | 2278, | 2334, | n-C₄H₉ | n-C₅H₁₁ |
| 2167, | 2223, | 2279, | 2335, | n-C₄H₉ | n-C₆H₁₃ |
| 2168, | 2224, | 2280, | 2336, | n-C₄H₉ | n-C₇H₁₅ |
| 2169, | 2225, | 2281, | 2337, | n-C₅H₁₁ | CH₃ |
| 2170, | 2226, | 2282, | 2338, | n-C₅H₁₁ | C₂H₅ |
| 2171, | 2227, | 2283, | 2339, | n-C₅H₁₁ | n-C₃H₇ |
| 2172, | 2228, | 2284, | 2340, | n-C₅H₁₁ | n-C₄H₉ |
| 2173, | 2229, | 2285, | 2341, | n-C₅H₁₁ | n-C₅H₁₁ |
| 2174, | 2230, | 2286, | 2342, | n-C₅H₁₁ | n-C₆H₁₃ |
| 2175, | 2231, | 2287, | 2343, | n-C₅H₁₁ | n-C₇H₁₅ |
| 2176, | 2232, | 2288, | 2344, | n-C₆H₁₃ | CH₃ |
| 2177, | 2233, | 2289, | 2345, | n-C₆H₁₃ | C₂H₅ |
| 2178, | 2234, | 2290, | 2346, | n-C₆H₁₃ | n-C₃H₇ |
| 2179, | 2235, | 2291, | 2347, | n-C₆H₁₃ | n-C₄H₉ |
| 2180, | 2236, | 2292, | 2348, | n-C₆H₁₃ | n-C₅H₁₁ |
| 2181, | 2237, | 2293, | 2349, | n-C₆H₁₃ | n-C₆H₁₃ |
| 2182, | 2238, | 2294, | 2350, | n-C₆H₁₃ | n-C₇H₁₅ |
| 2183, | 2239, | 2295, | 2351, | n-C₇H₁₅ | CH₃ |
| 2184, | 2240, | 2296, | 2352, | n-C₇H₁₅ | C₂H₅ |
| 2185, | 2241, | 2297, | 2353, | n-C₇H₁₅ | n-C₃H₇ |
| 2186, | 2242, | 2298, | 2354, | n-C₇H₁₅ | n-C₄H₉ |
| 2187, | 2243, | 2299, | 2355, | n-C₇H₁₅ | n-C₅H₁₁ |
| 2188, | 2244, | 2300, | 2356, | n-C₇H₁₅ | n-C₆H₁₃ |
| 2189, | 2245, | 2301, | 2357, | n-C₇H₁₅ | n-C₇H₁₅ |

### Beispiele 2358-2621

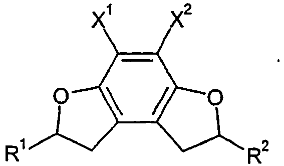

### Beispiele 2622-2885

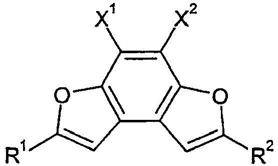

| **Beispiel-Nr.** | | **X¹** | **X²** | **R¹** | **R²** |
|---|---|---|---|---|---|
| 2358; | 2622, | H | H | H | H |
| 2359, | 2623, | H | H | H | CH₃ |
| 2360, | 2624, | H | H | H | C₂H₅ |
| 2361, | 2625, | H | H | H | n-C₃H₇ |
| 2362, | 2626, | H | H | H | n-C₄H₉ |
| 2363, | 2627, | H | H | H | n-C₅H₁₁ |
| 2364, | 2628, | H | H | H | n-C₆H₁₃ |
| 2365, | 2629, | H | H | H | n-C₇H₁₅ |
| 2366, | 2630, | F | H | H | H |
| 2367, | 2631, | F | H | H | CH₃ |
| 2368, | 2632, | F | H | H | C₂H₅ |
| 2369, | 2633, | F | H | H | n-C₃H₇ |
| 2370, | 2634, | F | H | H | n-C₄H₉ |
| 2371, | 2635, | F | H | H | n-C₅H₁₁ |
| 2372, | 2636, | F | H | H | n-C₆H₁₃ |
| 2373, | 2637, | F | H | H | n-C₇H₁₅ |
| 2374, | 2638, | F | F | H | H |
| 2375, | 2639, | F | F | H | CH₃ |
| 2376, | 2640, | F | F | H | C₂H₅ |
| 2377, | 2641, | F | F | H | n-C₃H₇ |
| 2378, | 2642, | F | F | H | n-C₄H₉ |
| 2379, | 2643, | F | F | H | n-C₅H₁₁ |
| 2380, | 2644, | F | F | H | n-C₆H₁₃ |
| 2381, | 2645, | F | F | H | n-C₇H₁₅ |
| 2382, | 2646, | CF₃ | H | H | H |
| 2383, | 2647, | CF₃ | H | H | CH₃ |
| 2384, | 2648, | CF₃ | H | H | C₂H₅ |
| 2385, | 2649, | CF₃ | H | H | n-C₃H₇ |
| 2386, | 2650, | CF₃ | H | H | n-C₄H₉ |
| 2387, | 2651, | CF₃ | H | H | n-C₅H₁₁ |
| 2388, | 2652, | CF₃ | H | H | n-C₆H₁₃ |
| 2389, | 2653, | CF₃ | H | H | n-C₇H₁₅ |
| 2390, | 2654, | -OCF₃ | H | H | H |
| 2391, | 2655, | -OCF₃ | H | H | CH₃ |
| 2392, | 2656, | -OCF₃ | H | H | C₂H₅ |
| 2393, | 2657, | -OCF₃ | H | H | n-C₃H₇ |
| 2394, | 2658, | -OCF₃ | H | H | n-C₄H₉ |
| 2395, | 2659, | -OCF₃ | H | H | n-C₅H₁₁ |
| 2396, | 2660, | -OCF₃ | H | H | n-C₆H₁₃ |
| 2397, | 2661, | -OCF₃ | H | H | n-C₇H₁₅ |
| 2398, | 2662, | H | H | CH₃ | CH₃ |
| 2399, | 2663, | H | H | CH₃ | C₂H₅ |
| 2400, | 2664, | H | H | CH₃ | n-C₃H₇ |
| 2401, | 2665, | H | H | CH₃ | n-C₄H₉ |
| 2402, | 2666, | H | H | CH₃ | n-C₅H₁₁ |
| 2403, | 2667, | H | H | CH₃ | n-C₆H₁₃ |
| 2404, | 2668, | H | H | CH₃ | n-C₇H₁₅ |
| 2405, | 2669, | F | H | CH₃ | H |
| 2406, | 2670, | F | H | CH₃ | CH₃ |
| 2407, | 2671, | F | H | CH₃ | C₂H₅ |
| 2408, | 2672, | F | H | CH₃ | n-C₃H₇ |
| 2409, | 2673, | F | H | CH₃ | n-C₄H₉ |
| 2410, | 2674, | F | H | CH₃ | n-C₅H₁₁ |
| 2411, | 2675, | F | H | CH₃ | n-C₆H₁₃ |
| 2412, | 2676, | F | H | CH₃ | n-C₇H₁₅ |
| 2413, | 2677, | F | F | CH₃ | CH₃ |
| 2414, | 2678, | F | F | CH₃ | C₂H₅ |
| 2415, | 2679, | F | F | CH₃ | n-C₃H₇ |
| 2416, | 2680, | F | F | CH₃ | n-C₄H₉ |
| 2417, | 2681, | F | F | CH₃ | n-C₅H₁₁ |
| 2418, | 2682, | F | F | CH₃ | n-C₆H₁₃ |
| 2419, | 2683, | F | F | CH₃ | n-C₇H₁₅ |
| 2420, | 2684, | CF₃ | H | CH₃ | H |
| 2421, | 2685, | CF₃ | H | CH₃ | CH₃ |
| 2422, | 2686, | CF₃ | H | CH₃ | C₂H₅ |
| 2423, | 2687, | CF₃ | H | CH₃ | n-C₃H₇ |
| 2424, | 2688, | CF₃ | H | CH₃ | n-C₄H₉ |
| 2425, | 2689, | CF₃ | H | CH₃ | n-C₅H₁₁ |
| 2426, | 2690, | CF₃ | H | CH₃ | n-C₆H₁₃ |
| 2427, | 2691, | CF₃ | H | CH₃ | n-C₇H₁₅ |
| 2428, | 2692, | -OCF₃ | H | CH₃ | H |
| 2429, | 2693, | -OCF₃ | H | CH₃ | CH₃ |
| 2430, | 2694, | -OCF₃ | H | CH₃ | C₂H₅ |
| 2431, | 2695, | -OCF₃ | H | CH₃ | n-C₃H₇ |
| 2432, | 2696, | -OCF₃ | H | CH₃ | n-C₄H₉ |
| 2433, | 2697, | -OCF₃ | H | CH₃ | n-C₅H₁₁ |
| 2434, | 2698, | -OCF₃ | H | CH₃ | n-C₆H₁₃ |
| 2435, | 2699, | -OCF₃ | H | CH₃ | n-C₇H₁₅ |
| 2436, | 2700, | H | H | C₂H₅ | C₂H₅ |
| 2437, | 2701, | H | H | C₂H₅ | n-C₃H₇ |
| 2438, | 2702, | H | H | C₂H₅ | n-C₄H₉ |
| 2439, | 2703, | H | H | C₂H₅ | n-C₅H₁₁ |
| 2440, | 2704, | H | H | C₂H₅ | n-C₆H₁₃ |
| 2441, | 2705, | H | H | C₂H₅ | n-C₇H₁₅ |
| 2442, | 2706, | F | H | C₂H₅ | H |
| 2443, | 2707, | F | H | C₂H₅ | CH₃ |
| 2444, | 2708, | F | H | C₂H₅ | C₂H₅ |
| 2445, | 2709, | F | H | C₂H₅ | n-C₃H₇ |
| 2446, | 2710, | F | H | C₂H₅ | n-C₄H₉ |
| 2447, | 2711, | F | H | C₂H₅ | n-C₅H₁₁ |
| 2448, | 2712, | F | H | C₂H₅ | n-C₆H₁₃ |
| 2449, | 2713, | F | H | C₂H₅ | n-C₇H₁₅ |
| 2450, | 2714, | F | F | C₂H₅ | C₂H₅ |
| 2451, | 2715, | F | F | C₂H₅ | n-C₃H₇ |
| 2452, | 2716, | F | F | C₂H₅ | n-C₄H₉ |
| 2453, | 2717, | F | F | C₂H₅ | n-C₅H₁₁ |
| 2454, | 2718, | F | F | C₂H₅ | n-C₆H₁₃ |
| 2455, | 2719, | F | F | C₂H₅ | n-C₇H₁₅ |
| 2456, | 2720, | CF₃ | H | C₂H₅ | H |
| 2457. | 2721, | CF₃ | H | C₂H₅ | CH₃ |
| 2458, | 2722, | CF₃ | H | C₂H₅ | C₂H₅ |
| 2459, | 2723, | CF₃ | H | C₂H₅ | n-C₃H₇ |
| 2460, | 2724, | CF₃ | H | C₂H₅ | n-C₄H₉ |
| 2461, | 2725, | CF₃ | H | C₂H₅ | n-C₅H₁₁ |
| 2462, | 2726, | CF₃ | H | C₂H₅ | n-C₆H₁₃ |
| 2463, | 2727, | CF₃ | H | C₂H₅ | n-C₇H₁₅ |
| 2464, | 2728, | -OCF₃ | H | C₂H₅ | H |
| 2465, | 2729, | -OCF₃ | H | C₂H₅ | CH₃ |
| 2466, | 2730, | -OCF₃ | H | C₂H₅ | C₂H₅ |
| 2467, | 2731, | -OCF₃ | H | C₂H₅ | n-C₃H₇ |
| 2468, | 2732, | -OCF₃ | H | C₂H₅ | n-C₄H₉ |
| 2469, | 2733, | -OCF₃ | H | C₂H₅ | n-C₅H₁₁ |
| 2470, | 2734, | -OCF₃ | H | C₂H₅ | n-C₆H₁₃ |
| 2471, | 2735, | -OCF₃ | H | C₂H₅ | n-C₇H₁₅ |
| 2472, | 2736, | H | H | n-C₃H₇ | n-C₃H₇ |
| 2473, | 2737, | H | H | n-C₃H₇ | n-C₄H₉ |
| 2474, | 2738, | H | H | n-C₃H₇ | n-C₅H₁₁ |
| 2475, | 2739, | H | H | n-C₃H₇ | n-C₆H₁₃ |
| 2476, | 2740, | H | H | n-C₃H₇ | n-C₇H₁₅ |
| 2477, | 2741, | F | H | n-C₃H₇ | H |
| 2478, | 2742, | F | H | n-C₃H₇ | CH₃ |
| 2479, | 2743, | F | H | n-C₃H₇ | C₂H₅ |
| 2480, | 2744, | F | H | n-C₃H₇ | n-C₃H₇ |
| 2481, | 2745, | F | H | n-C₃H₇ | n-C₄H₉ |
| 2482, | 2746, | F | H | n-C₃H₇ | n-C₅H₁₁ |
| 2483, | 2747, | F | H | n-C₃H₇ | n-C₆H₁₃ |
| 2484, | 2748, | F | H | n-C₃H₇ | n-C₇H₁₅ |
| 2485, | 2749, | F | F | n-C₃H₇ | n-C₃H₇ |
| 2486, | 2750, | F | F | n-C₃H₇ | n-C₄H₉ |
| 2487, | 2751, | F | F | n-C₃H₇ | n-C₅H₁₁ |
| 2488, | 2752, | F | F | n-C₃H₇ | n-C₆H₁₃ |
| 2489, | 2753, | F | F | n-C₃H₇ | n-C₇H₁₅ |
| 2490, | 2754, | CF₃ | H | n-C₃H₇ | H |
| 2491, | 2755, | CF₃ | H | n-C₃H₇ | CH₃ |
| 2492, | 2756, | CF₃ | H | n-C₃H₇ | C₂H₅ |
| 2493, | 2757, | CF₃ | H | n-C₃H₇ | n-C₃H₇ |
| 2494, | 2758, | CF₃ | H | n-C₃H₇ | n-C₄H₉ |
| 2495, | 2759, | CF₃ | H | n-C₃H₇ | n-C₅H₁₁ |
| 2496, | 2760, | CF₃ | H | n-C₃H₇ | n-C₆H₁₃ |
| 2497, | 2761, | CF₃ | H | n-C₃H₇ | n-C₇H₁₅ |
| 2498, | 2762, | -OCF₃ | H | n-C₃H₇ | H |
| 2499, | 2763, | -OCF₃ | H | n-C₃H₇ | CH₃ |
| 2500, | 2764, | -OCF₃ | H | n-C₃H₇ | C₂H₅ |
| 2501, | 2765, | -OCF₃ | H | n-C₃H₇ | n-C₃H₇ |
| 2502, | 2766, | -OCF₃ | H | n-C₃H₇ | n-C₄H₉ |
| 2503, | 2767, | -OCF₃ | H | n-C₃H₇ | n-C₅H₁₁ |
| 2504, | 2768, | -OCF₃ | H | n-C₃H₇ | n-C₆H₁₃ |
| 2505, | 2769, | -OCF₃ | H | n-C₃H₇ | n-C₇H₁₅ |
| 2506, | 2770, | H | H | n-C₄H₉ | n-C₄H₉ |
| 2507, | 2771, | H | H | n-C₄H₉ | n-C₅H₁₁ |
| 2508, | 2772, | H | H | n-C₄H₉ | n-C₅H₁₃ |
| 2509, | 2773, | H | H | n-C₄H₉ | n-C₇H₁₅ |
| 2510, | 2774, | F | H | n-C₄H₉ | H |
| 2511, | 2775, | F | H | n-C₄H₉ | CH₃ |
| 2512, | 2776, | F | H | n-C₄H₉ | C₂H₅ |
| 2513, | 2777, | F | H | n-C₄H₉ | n-C₃H₇ |
| 2514, | 2778, | F | H | n-C₄H₉ | n-C₄H₉ |
| 2515, | 2779, | F | H | n-C₄H₉ | n-C₅H₁₁ |
| 2516, | 2780, | F | H | n-C₄H₉ | n-C₆H₁₃ |
| 2517, | 2781, | F | H | n-C₄H₉ | n-C₇H₁₅ |
| 2518, | 2782, | F | F | n-C₄H₉ | n-C₄H₉ |
| 2519, | 2783, | F | F | n-C₄H₉ | n-C₅H₁₁ |
| 2520, | 2784, | F | F | n-C₄H₉ | n-C₆H₁₃ |
| 2521, | 2785, | F | F | n-C₄H₉ | n-C₇H₁₅ |
| 2522, | 2786, | CF₃ | H | n-C₄H₉ | H |
| 2523, | 2787, | CF₃ | H | n-C₄H₉ | CH₃ |
| 2524, | 2788, | CF₃ | H | n-C₄H₉ | C₂H₅ |
| 2525, | 2789, | CF₃ | H | n-C₄H₉ | n-C₃H₇ |
| 2526, | 2790, | CF₃ | H | n-C₄H₉ | n-C₄H₉ |
| 2527, | 2791, | CF₃ | H | n-C₄H₉ | n-C₅H₁₁ |
| 2528, | 2792, | CF₃ | H | n-C₄H₉ | n-C₆H₁₃ |
| 2529, | 2793, | CF₃ | H | n-C₄H₉ | n-C₇H₁₅ |
| 2530, | 2794, | -OCF₃ | H | n-C₄H₉ | H |
| 2531, | 2795, | -OCF₃ | H | n-C₄H₉ | CH₃ |
| 2532, | 2796, | -OCF₃ | H | n-C₄H₉ | C₂H₅ |
| 2533, | 2797, | -OCF₃ | H | n-C₄H₉ | n-C₃H₇ |
| 2534, | 2798, | -OCF₃ | H | n-C₄H₉ | n-C₄H₉ |
| 2535, | 2799, | -OCF₃ | H | n-C₄H₉ | n-C₅H₁₁ |
| 2536, | 2800, | -OCF₃ | H | n-C₄H₉ | n-C₆H₁₃ |
| 2537, | 2801, | -OCF₃ | H | n-C₄H₉ | n-C₇H₁₅ |
| 2538, | 2802, | H | H | n-C₅H₁₁ | n-C₅H₁₁ |
| 2539, | 2803, | H | H | n-C₅H₁₁ | n-C₆H₁₃ |
| 2540, | 2804, | H | H | n-C₅H₁₁ | n-C₇H₁₅ |
| 2541, | 2805, | F | H | n-C₅H₁₁ | H |
| 2542, | 2806, | F | H | n-C₅H₁₁ | CH₃ |
| 2543, | 2807, | F | H | n-C₅H₁₁ | C₂H₅ |
| 2544, | 2808, | F | H | n-C₅H₁₁ | n-C₃H₇ |
| 2545, | 2809, | F | H | n-C₅H₁₁ | n-C₄H₉ |
| 2546, | 2810, | F | H | n-C₅H₁₁ | n-C₅H₁₁ |
| 2547, | 2811, | F | H | n-C₅H₁₁ | n-C₆H₁₃ |
| 2548, | 2812, | F | H | n-C₅H₁₁ | n-C₇H₁₅ |
| 2549, | 2813, | F | F | n-C₅H₁₁ | n-C₅H₁₁ |
| 2550, | 2814, | F | F | n-C₅H₁₁ | n-C₆H₁₃ |
| 2551, | 2815, | F | F | n-C₅H₁₁ | n-C₇H₁₅ |
| 2552, | 2816, | CF₃ | H | n-C₅H₁₁ | H |
| 2553, | 2817, | CF₃ | H | n-C₅H₁₁ | CH₃ |
| 2554, | 2818, | CF₃ | H | n-C₅H₁₁ | C₂H₅ |
| 2555, | 2819, | CF₃ | H | n-C₅H₁₁ | n-C₃H₇ |
| 2556, | 2820, | CF₃ | H | n-C₅H₁₁ | n-C₄H₉ |
| 2557, | 2821, | CF₃ | H | n-C₅H₁₁ | n-C₅H₁₁ |
| 2558, | 2822, | CF₃ | H | n-C₅H₁₁ | n-C₆H₁₃ |
| 2559, | 2823, | CF₃ | H | n-C₅H₁₁ | n-C₇H₁₅ |
| 2560, | 2824, | -OCF₃ | H | n-C₅H₁₁ | H |
| 2561, | 2825, | -OCF₃ | H | n-C₅H₁₁ | CH₃ |
| 2562, | 2826, | -OCF₃ | H | n-C₅H₁₁ | C₂H₅ |
| 2563, | 2827, | -OCF₃ | H | n-C₅H₁₁ | n-C₃H₇ |
| 2564, | 2828, | -OCF₃ | H | n-C₅H₁₁ | n-C₄H₉ |
| 2565, | 2829, | -OCF₃ | H | n-C₅H₁₁ | n-C₅H₁₁ |
| 2566, | 2830, | -OCF₃ | H | n-C₅H₁₁ | n-C₆H₁₃ |
| 2567, | 2831, | -OCF₃ | H | n-C₅H₁₁ | n-C₇H₁₅ |
| 2568, | 2832, | H | H | n-C₆H₁₃ | n-C₆H₁₃ |
| 2569, | 2833, | H | H | n-C₆H₁₃ | n-C₇H₁₅ |
| 2570, | 2834, | F | H | n-C₆H₁₃ | H |
| 2571, | 2835, | F | H | n-C₆H₁₃ | CH₃ |
| 2572, | 2836, | F | H | n-C₆H₁₃ | C₂H₅ |
| 2573, | 2837, | F | H | n-C₆H₁₃ | n-C₃H₇ |
| 2574, | 2838, | F | H | n-C₆H₁₃ | n-C₄H₉ |
| 2575, | 2839, | F | H | n-C₆H₁₃ | n-C₅H₁₁ |
| 2576, | 2840, | F | H | n-C₆H₁₃ | n-C₆H₁₃ |
| 2577, | 2841, | F | H | n-C₆H₁₃ | n-C₇H₁₅ |
| 2578, | 2842, | F | F | n-C₆H₁₃ | n-C₆H₁₃ |
| 2579, | 2843, | F | F | n-C₆H₁₃ | n-C₇H₁₅ |
| 2580, | 2844, | CF₃ | H | n-C₆H₁₃ | H |
| 2581, | 2845, | CF₃ | H | n-C₅H₁₃ | CH₃ |
| 2582, | 2846, | CF₃ | H | n-C₆H₁₃ | C₂H₅ |
| 2583, | 2847, | CF₃ | H | n-C₆H₁₃ | n-C₃H₇ |
| 2584, | 2848, | CF₃ | H | n-C₆H₁₃ | n-C₄H₉ |
| 2585, | 2849, | CF₃ | H | n-C₆H₁₃ | n-C₅H₁₁ |
| 2586, | 2850, | CF₃ | H | n-C₆H₁₃ | n-C₆H₁₃ |
| 2587, | 2851, | CF₃ | H | n-C₆H₁₃ | n-C₇H₁₅ |
| 2588, | 2852, | -OCF₃ | H | n-C₆H₁₃ | H |
| 2589, | 2853, | -OCF₃ | H | n-C₆H₁₃ | CH₃ |
| 2590, | 2854, | -OCF₃ | H | n-C₆H₁₃ | C₂H₅ |
| 2591, | 2855, | -OCF₃ | H | n-C₆H₁₃ | n-C₃H₇ |
| 2592, | 2856, | -OCF₃ | H | n-C₆H₁₃ | n-C₄H₉ |
| 2593, | 2857, | -OCF₃ | H | n-C₆H₁₃ | n-C₅H₁₁ |
| 2594, | 2858, | -OCF₃ | H | n-C₆H₁₃ | n-C₆H₁₃ |
| 2595, | 2859, | -OCF₃ | H | n-C₆H₁₃ | n-C₇H₁₅ |
| 2596, | 2860, | H | H | n-C₇H₁₅ | n-C₇H₁₅ |
| 2597, | 2861, | F | H | n-C₇H₁₅ | H |
| 2598, | 2862, | F | H | n-C₇H₁₅ | CH₃ |
| 2599, | 2863, | F | H | n-C₇H₁₅ | C₂H₅ |
| 2600, | 2864, | F | H | n-C₇H₁₅ | n-C₃H₇ |
| 2601, | 2865, | F | H | n-C₇H₁₅ | n-C₄H₉ |
| 2602, | 2866, | F | H | n-C₇H₁₅ | n-C₅H₁₁ |
| 2603, | 2867, | F | H | n-C₇H₁₅ | n-C₆H₁₃ |
| 2604, | 22868, | F | H | n-C₇H₁₅ | n-C₇H₁₅ |
| 2605, | 2869, | F | F | n-C₇H₁₅ | n-C₇H₁₅ |
| 2606, | 2870, | CF₃ | H | n-C₇H₁₅ | H |
| 2607, | 2871, | CF₃ | H | n-C₇H₁₅ | CH₃ |
| 2608, | 2872, | CF₃ | H | n-C₇H₁₅ | C₂H₅ |
| 2609, | 2873, | CF₃ | H | n-C₇H₁₅ | n-C₃H₇ |
| 2610, | 2874, | CF₃ | H | n-C₇H₁₅ | n-C₄H₉ |
| 2611, | 2875, | CF₃ | H | n-C₇H₁₅ | n-C₅H₁₁ |
| 2612, | 2876, | CF₃ | H | n-C₇H₁₅ | n-C₆H₁₃ |
| 2613, | 2877, | CF₃ | H | n-C₇H₁₅ | n-C₇H₁₅ |
| 2614, | 2878, | -OCF₃ | H | n-C₇H₁₅ | H |
| 2615, | 2879, | -OCF₃ | H | n-C₇H₁₅ | CH₃ |
| 2616, | 2880, | -OCF₃ | H | n-C₇H₁₅ | C₂H₅ |
| 2617, | 2881, | -OCF₃ | H | n-C₇H₁₅ | n-C₃H₇ |
| 2618, | 2882, | -OCF₃ | H | n-C₇H₁₅ | n-C₄H₉ |
| 2619, | 2883, | -OCF₃ | H | n-C₇H₁₅ | n-C₅H₁₁ |
| 2620, | 2884, | -OCF₃ | H | n-C₇H₁₅ | n-C₆H₁₃ |
| 2621, | 2885, | -OCF₃ | H | n-C₇H₁₅ | n-C₇H₁₅ |

### Beispiele 2886-2949

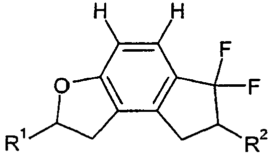

### Beispiele 2950-3013

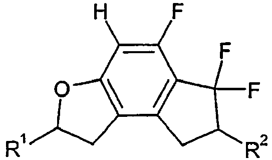

### Beispiele 3014-3077

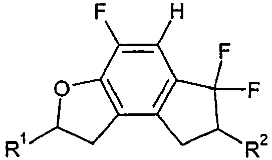

### Beispiele 3078-3141

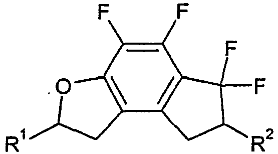

| **Beispiel-Nr.** | | | | **R¹** | **R²** |
|---|---|---|---|---|---|
| 2886, | 2950, | 3014, | 3078, | H | H |
| 2887, | 2951, | 3015, | 3079, | H | CH₃ |
| 2888, | 2952, | 3016, | 3080, | H | C₂H₅ |
| 2889, | 2953, | 3017, | 3081, | H | n-C₃H₇ |
| 2890, | 2954, | 3018, | 3082, | H | n-C₄H₉ |
| 2891, | 2955, | 3019, | 3083, | H | n-C₅H₁₁ |
| 2892, | 2956, | 3020, | 3084, | H | n-C₆H₁₃ |
| 2893, | 2957, | 3021, | 3085, | H | n-C₇H₁₅ |
| 2894, | 2958, | 3022, | 3086, | CH₃ | H |
| 2895, | 2959, | 3023, | 3087, | CH₃ | CH₃ |
| 2896, | 2960, | 3024, | 3088, | CH₃ | C₂H₅ |
| 2897, | 2961, | 3025, | 3089, | CH₃ | n-C₃H₇ |
| 2898, | 2962, | 3026, | 3090, | CH₃ | n-C₄H₉ |
| 2899, | 2963, | 3027, | 3091, | CH₃ | n-C₅H₁₁ |
| 2900, | 2964, | 3028, | 3092, | CH₃ | n-C₆H₁₃ |
| 2901, | 2965, | 3029, | 3093, | CH₃ | n-C₇H₁₅ |
| 2902, | 2966, | 3030, | 3094, | C₂H₅ | H |
| 2903, | 2967, | 3031, | 3095, | C₂H₅ | CH₃ |
| 2904, | 2968, | 3032, | 3096, | C₂H₅ | C₂H₅ |
| 2905, | 2969, | 3033, | 3097, | C₂H₅ | n-C₃H₇ |
| 2906, | 2970, | 3034, | 3098, | C₂H₅ | n-C₄H₉ |
| 2907, | 2971, | 3035, | 3099, | C₂H₅ | n-C₅H₁₁ |
| 2908, | 2972, | 3036, | 3100, | C₂H₅ | n-C₆H₁₃ |
| 2909, | 2973, | 3037, | 3101, | C₂H₅ | n-C₇H₁₅ |
| 2910, | 2974, | 3038, | 3102, | n-C₃H₇ | H |
| 2911, | 2975, | 3039, | 3103, | n-C₃H₇ | CH₃ |
| 2912, | 2976, | 3040, | 3104, | n-C₃H₇ | C₂H₅ |
| 2913, | 2977, | 3041, | 3105, | n-C₃H₇ | n-C₃H₇ |
| 2914, | 2978, | 3042, | 3106, | n-C₃H₇ | n-C₄H₉ |
| 2915, | 2979, | 3043, | 3107, | n-C₃H₇ | n-C₅H₁₁ |
| 2916, | 2980, | 3044, | 3108, | n-C₃H₇ | n-C₆H₁₃ |
| 2917, | 2981, | 3045, | 3109, | n-C₃H₇ | n-C₇H₁₅ |
| 2918, | 2982, | 3046, | 3110, | n-C₄H₉ | H |
| 2919, | 2983, | 3047, | 3111, | n-C₄H₉ | CH₃ |
| 2920, | 2984, | 3048, | 3112, | n-C₄H₉ | C₂H₅ |
| 2921, | 2985, | 3049, | 3113, | n-C₄H₉ | n-C₃H₇ |
| 2922, | 2986, | 3050, | 3114, | n-C₄H₉ | n-C₄H₉ |
| 2923, | 2987, | 3051, | 3115, | n-C₄H₉ | n-C₅H₁₁ |
| 2924, | 2988, | 3052, | 3116, | n-C₄H₉ | n-C₆H₁₃ |
| 2925, | 2989, | 3053, | 3117, | n-C₄H₉ | n-C₇H₁₅ |
| 2926, | 2990, | 3054, | 3118, | n-C₅H₁₁ | H |
| 2927, | 2991, | 3055, | 3119, | n-C₅H₁₁ | CH₃ |
| 2928, | 2992, | 3056, | 3120, | n-C₅H₁₁ | C₂H₅ |
| 2929, | 2993, | 3057, | 3121, | n-C₅H₁₁ | n-C₃H₇ |
| 2930, | 2994, | 3058, | 3122, | n-C₅H₁₁ | n-C₄H₉ |
| 2931, | 2995, | 3059, | 3123, | n-C₅H₁₁ | n-C₅H₁₁ |
| 2932, | 2996, | 3060, | 3124, | n-C₅H₁₁ | n-C₆H₁₃ |
| 2933, | 2997, | 3061, | 3125, | n-C₅H₁₁ | n-C₇H₁₅ |
| 2934, | 2998, | 3062, | 3126, | n-C₆H₁₃ | H |
| 2935, | 2999, | 3063, | 3127, | n-C₆H₁₃ | CH₃ |
| 2936, | 3000, | 3064, | 3128, | n-C₆H₁₃ | C₂H₅ |
| 2937, | 3001, | 3065, | 3129, | n-C₆H₁₃ | n-C₃H₇ |
| 2938, | 3002, | 3066, | 3130, | n-C₆H₁₃ | n-C₄H₉ |
| 2939, | 3003, | 3067, | 3131, | n-C₆H₁₃ | n-C₅H₁₁ |
| 2940, | 3004, | 3068, | 3132, | n-C₆H₁₃ | n-C₆H₁₃ |
| 2941, | 3005, | 3069, | 3133, | n-C₆H₁₃ | n-C₇H₁₅ |
| 2942, | 3006, | 3070, | 3134, | n-C₇H₁₅ | H |
| 2943, | 3007, | 3071, | 3135, | **n-C₇H₁₅** | CH₃ |
| 2944, | 3008, | 3072, | 3136, | n-C₇H₁₅ | C₂H₅ |
| 2945, | 3009, | 3073, | 3137, | n-C₇H₁₅ | n-C₃H₇ |
| 2946, | 3010, | 3074, | 3138, | n-C₇H₁₆ | n-C₄H₉ |
| 2947, | 3011, | 3075, | 3139, | n-C₇H₁₅ | n-C₅H₁₁ |
| 2948, | 3012, | 3076, | 3140, | n-C₇H₁₅ | n-C₆H₁₃ |
| 2949, | 3013, | 3077, | 3141, | n-C₇H₁₅ | n-C₇H₁₅ |

### Beispiele 3142-3205

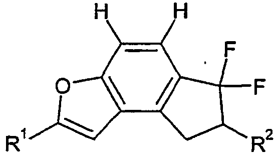

### Beispiele 3206-3269

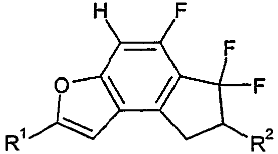

### Beispiele 3270-3333

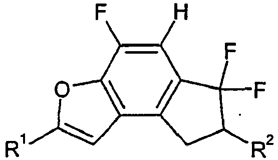

### Beispiele 3334-3397

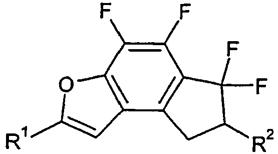

| **Beispiel-Nr.** | | | | **R¹** | **R²** |
|---|---|---|---|---|---|
| 3142, | 3206, | 3270, | 3334, | H | H |
| 3143, | 3207, | 3271, | 3335, | H | CH₃ |
| 3144, | 3208, | 3272, | 3336, | H | C₂H₅ |
| 3145, | 3209, | 3273, | 3337, | H | n-C₃H₇ |
| 3146, | 3210, | 3274, | 3338, | H | n-C₄H₉ |
| 3147, | 3211, | 3275, | 3339, | H | n-C₅H₁₁ |
| 3148, | 3212, | 3276, | 3340, | H | n-C₆H₁₃ |
| 3149, | 3213, | 3277, | 3341, | H | n-C₇H₁₅ |
| 3150, | 3214, | 3278, | 3342, | CH₃ | H |
| 3151, | 3215, | 3279, | 3343, | CH₃ | CH₃ |
| 3152, | 3216, | 3280, | 3344, | CH₃ | C₂H₅ |
| 3153, | 3217, | 3281, | 3345, | CH₃ | n-C₃H₇ |
| 3154, | 3218, | 3282, | 3346, | CH₃ | n-C₄H₉ |
| 3155, | 3219, | 3283, | 3347, | CH₃ | n-C₅H₁₁ |
| 3156, | 3220, | 3284, | 3348, | CH₃ | n-C₆H₁₃ |
| 3157, | 3221, | 3285, | 3349, | CH₃ | n-C₇H₁₅ |
| 3158, | 3222, | 3286, | 3350, | C₂H₅ | H |
| 3159, | 3223, | 3287, | 3351, | C₂H₅ | CH₃ |
| 3160, | 3224, | 3288, | 3352, | C₂H₅ | C₂H₅ |
| 3161, | 3225, | 3289, | 3353, | C₂H₅ | n-C₃H₇ |
| 3162, | 3226, | 3290, | 3354, | C₂H₅ | n-C₄H₉ |
| 3163, | 3227, | 3291, | 3355, | C₂H₅ | n-C₅H₁₁ |
| 3164, | 3228, | 3292, | 3356, | C₂H₅ | n-C₆H₁₃ |
| 3165, | 3229, | 3293, | 3357, | C₂H₅ | n-C₇H₁₅ |
| 3166, | 3230, | 3294, | 3358, | n-C₃H₇ | H |
| 3167, | 3231, | 3295, | 3359, | n-C₃H₇ | CH₃ |
| 3168, | 3232, | 3296, | 3360, | n-C₃H₇ | C₂H₅ |
| 3169, | 3233, | 3297, | 3361, | n-C₃H₇ | n-C₃H₇ |
| 3170, | 3234, | 3298, | 3362, | n-C₃H₇ | n-C₄H₉ |
| 3171, | 3235, | 3299, | 3363, | n-C₃H₇ | n-C₅H₁₁ |
| 3172, | 3236, | 3300, | 3364, | n-C₃H₇ | n-C₆H₁₃ |
| 3173, | 3237, | 3301, | 3365, | n-C₃H₇ | n-C₇H₁₅ |
| 3174, | 3238, | 3302, | 3366, | n-C₄H₉ | H |
| 3175, | 3239, | 3303, | 3367, | n-C₄H₉ | CH₃ |
| 3176, | 3240, | 3304, | 3368, | n-C₄H₉ | C₂H₅ |
| 3177, | 3241, | 3305, | 3369, | n-C₄H₉ | n-C₃H₇ |
| 3178, | 3242, | 3306, | 3370, | n-C₄H₉ | n-C₄H₉ |
| 3179, | 3243, | 3307, | 3371, | n-C₄H₉ | n-C₅H₁₁ |
| 3180, | 3244, | 3308, | 3372, | n-C₄H₉ | n-C₆H₁₃ |
| 3181, | 3245, | 3309, | 3373, | n-C₄H₉ | n-C₇H₁₅ |
| 3182, | 3246, | 3310, | 3374, | n-C₅H₁₁ | H |
| 3183, | 3247, | 3311, | 3375, | n-C₅H₁₁ | CH₃ |
| 3184, | 3248, | 3312, | 3376, | n-C₅H₁₁ | C₂H₅ |
| 3185, | 3249, | 3313, | 3377, | n-C₅H₁₁ | n-C₃H₇ |
| 3186, | 3250, | 3314, | 3378, | n-C₅H₁₁ | n-C₄H₉ |
| 3187, | 3251, | 3315, | 3379, | n-C₅H₁₁ | n-C₅H₁₁ |
| 3188, | 3252, | 3316, | 3380, | n-C₅H₁₁ | n-C₆H₁₃ |
| 3189, | 3253, | 3317, | 3381, | n-C₅H₁₁ | n-C₇H₁₅ |
| 3190, | 3254, | 3318, | 3382, | n-C₆H₁₃ | H |
| 3191, | 3255, | 3319, | 3383, | n-C₆H₁₃ | CH₃ |
| 3192, | 3256, | 3320, | 3384, | n-C₅H₁₃ | C₂H₅ |
| 3193, | 3257, | 3321, | 3385, | n-C₆H₁₃ | n-C₃H₇ |
| 3194, | 3258, | 3322, | 3386, | n-C₆H₁₃ | n-C₄H₉ |
| 3195, | 3259, | 3323, | 3387, | n-C₆H₁₃ | n-C₅H₁₁ |
| 3196, | 3260, | 3324, | 3388, | n-C₆H₁₃ | n-C₆H₁₃ |
| 3197, | 3261, | 3325, | 3389, | n-C₆H₁₃ | n-C₇H₁₅ |
| 3198, | 3262, | 3326, | 3390, | n-C₇H₁₅ | H |
| 3199, | 3263, | 3327, | 3391, | n-C₇H₁₅ | CH₃ |
| 3200, | 3264, | 3328, | 3392, | n-C₇H₁₅ | C₂H₅ |
| 3201, | 3265, | 3329, | 3393, | n-C₇H₁₅ | n-C₃H₇ |
| 3202, | 3266, | 3330, | 3394, | n-C₇H₁₅ | n-C₄H₉ |
| 3203, | 3267, | 3331, | 3395, | n-C₇H₁₅ | n-C₅H₁₁ |
| 3204, | 3268, | 3332, | 3396, | n-C₇H₁₅ | n-C₆H₁₃ |
| 3205, | 3269, | 3333, | 3397, | n-C₇H₁₅ | n-C₇H₁₅ |

### Beispiele 3398-3461

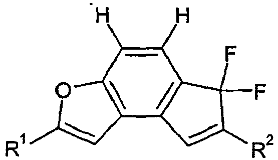

### Beispiele 3462-3525

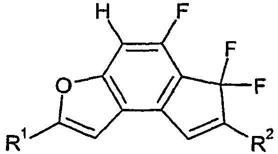

### Beispiele 3526-3589

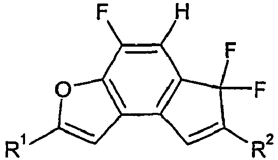

### Beispiele 3590-3653

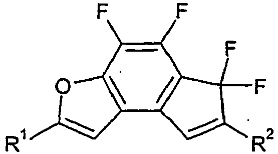

| **Beispiel-Nr.** | | | | **R¹** | **R²** |
|---|---|---|---|---|---|
| 3398, | 3462, | 3526, | 3590, | H | H |
| 3399, | 3463, | 3527, | 3591, | H | CH₃ |
| 3400, | 3464, | 3528, | 3592, | H | C₂H₅ |
| 3401, | 3465, | 3529, | 3593, | H | n-C₃H₇ |
| 3402, | 3466, | 3530, | 3594, | H | n-C₄H₉ |
| 3403, | 3467, | 3531, | 3595, | H | n-C₅H₁₁ |
| 3404, | 3468, | 3532, | 3596, | H | n-C₆H₁₃ |
| 3405, | 3469, | 3533, | 3597, | H | n-C₇H₁₅ |
| 3406, | 3470, | 3534, | 3598, | CH₃ | H |
| 3407, | 3471, | 3535, | 3599, | CH₃ | CH₃ |
| 3408, | 3472, | 3536, | 3600, | CH₃ | C₂H₅ |
| 3409, | 3473, | 3537, | 3601, | CH₃ | n-C₃H₇ |
| 3410, | 3474, | 3538, | 3602, | CH₃ | n-C₄H₉ |
| 3411, | 3475, | 3539, | 3603, | CH₃ | n-C₅H₁₁ |
| 3412, | 3476, | 3540, | 3604, | CH₃ | n-C₆H₁₃ |
| 3413, | 3477, | 3541, | 3605, | CH₃ | n-C₇H₁₅ |
| 3414, | 3478, | 3542, | 3606, | C₂H₅ | H |
| 3415, | 3479, | 3543, | 3607, | C₂H₅ | CH₃ |
| 3416, | 3480, | 3544, | 3608, | C₂H₅ | C₂H₅ |
| 3417, | 3481, | 3545, | 3609, | C₂H₅ | n-C₃H₇ |
| 3418, | 3482, | 3546, | 3610, | C₂H₅ | n-C₄H₉ |
| 3419, | 3483, | 3547, | 3611, | C₂H₅ | n-C₅H₁₁ |
| 3420, | 3484, | 3548, | 3612, | C₂H₅ | n-C₆H₁₃ |
| 3421, | 3485, | 3549, | 3613, | C₂H₅ | n-C₇H₁₅ |
| 3422, | 3486, | 3550, | 3614, | n-C₃H₇ | H |
| 3423, | 3487, | 3551, | 3615, | n-C₃H₇ | CH₃ |
| 3424, | 3488, | 3552, | 3616, | n-C₃H₇ | C₂H₅ |
| 3425, | 3489, | 3553, | 3617, | n-C₃H₇ | n-C₃H₇ |
| 3426, | 3490, | 3554, | 3618, | n-C₃H₇ | n-C₄H₉ |
| 3427, | 3491, | 3555, | 3619, | n-C₃H₇ | n-C₅H₁₁ |
| 3428, | 3492, | 3556, | 3620, | n-C₃H₇ | n-C₆H₁₃ |
| 3429, | 3493, | 3557, | 3621, | n-C₃H₇ | n-C₇H₁₅ |
| 3430, | 3494, | 3558, | 3622, | n-C₄H₉ | H |
| 3431, | 3495, | 3559, | 3623, | n-C₄H₉ | CH₃ |
| 3432, | 3496, | 3560, | 3624, | n-C₄H₉ | C₂H₅ |
| 3433, | 3497, | 3561, | 3625, | n-C₄H₉ | n-C₃H₇ |
| 3434, | 3498, | 3562, | 3626, | n-C₄H₉ | n-C₄H₉ |
| 3435, | 3499, | 3563, | 3627, | n-C₄H₉ | n-C₅H₁₁ |
| 3436, | 3500, | 3564, | 3628, | n-C₄H₉ | n-C₆H₁₃ |
| 3437, | 3501, | 3565, | 3629, | n-C₄H₉ | n-C₇H₁₅ |
| 3438, | 3502, | 3566, | 3630, | n-C₅H₁₁ | H |
| 3439, | 3503, | 3567, | 3631, | n-C₅H₁₁ | CH₃ |
| 3440, | 3504, | 3568, | 3632, | n-C₅H₁₁ | C₂H₅ |
| 3441, | 3505, | 3569, | 3633, | n-C₅H₁₁ | n-C₃H₇ |
| 3442, | 3506, | 3570, | 3634, | n-C₅H₁₁ | n-C₄H₉ |
| 3443, | 3507, | 3571, | 3635, | n-C₅H₁₁ | n-C₅H₁₁ |
| 3444, | 3508, | 3572, | 3636, | n-C₅H₁₁ | n-C₆H₁₃ |
| 3445, | 3509, | 3573, | 3637, | n-C₅H₁₁ | n-C₇H₁₅ |
| 3446, | 3510, | 3574, | 3638, | n-C₆H₁₃ | H |
| 3447, | 3511, | 3575, | 3639, | n-C₆H₁₃ | CH₃ |
| 3448, | 3512, | 3576, | 3640, | n-C₆H₁₃ | C₂H₅ |
| 3449, | 3513, | 3577, | 3641, | n-C₆H₁₃ | n-C₃H₇ |
| 3450, | 3514, | 3578, | 3642, | n-C₆H₁₃ | n-C₄H₉ |
| 3451, | 3515, | 3579, | 3643, | n-C₆H₁₃ | n-C₅H₁₁ |
| 3452, | 3516, | 3580, | 3644, | n-C₆H₁₃ | n-C₆H₁₃ |
| 3453, | 3517, | 3581, | 3645, | n-C₆H₁₃ | n-C₇H₁₅ |
| 3454, | 3518, | 3582, | 3646, | n-C₇H₁₅ | H |
| 3455, | 3519, | 3583, | 3647, | n-C₇H₁₅ | CH₃ |
| 3456, | 3520, | 3584, | 3648, | n-C₇H₁₅ | C₂H₅ |
| 3457, | 3521, | 3585, | 3649, | n-C₇H₁₅ | n-C₃H₇ |
| 3458, | 3522, | 3586, | 3650, | n-C₇H₁₅ | n-C₄H₉ |
| 3459, | 3523, | 3587, | 3651, | n-C₇H₁₅ | n-C₅H₁₁ |
| 3460, | 3524, | 3588, | 3652, | n-C₇H₁₅ | n-C₆H₁₃ |
| 3461, | 3525, | 3589, | 3653, | n-C₇H₁₅ | n-C₇H₁₅ |

### Beispiele 3654-3912

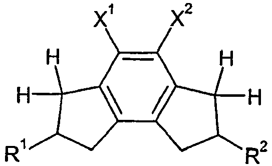

| **Beispiel-Nr.** | **X¹** | **X²** | **R¹** | **R²** |
|---|---|---|---|---|
| 3654, | H | H | H | CH₃ |
| 3655, | H | H | H | C₂H₅ |
| 3656, | H | H | H | n-C₃H₇ |
| 3657, | H | H | H | n-C₄H₉ |
| 3658, | H | H | H | n-C₅H₁₁ |
| 3659, | H | H | H | n-C₆H₁₃ |
| 3660, | H | H | H | n-C₇H₁₅ |
| 3661, | F | H | H | CH₃ |
| 3662, | F | H | H | C₂H₅ |
| 3663, | F | H | H | n-C₃H₇ |
| 3664, | F | H | H | n-C₄H₉ |
| 3665, | F | H | H | n-C₅H₁₁ |
| 3666, | F | H | H | n-C₆H₁₃ |
| 3667, | F | H | H | n-C₇H₁₅ |
| 3668, | H | F | H | CH₃ |
| 3669, | H | F | H | C₂H₅ |
| 3670, | H | F | H | n-C₃H₇ |
| 3671, | H | F | H | n-C₄H₉ |
| 3672, | H | F | H | n-C₅H₁₁ |
| 3673, | H | F | H | n-C₆H₁₃ |
| 3674, | H | F | H | n-C₇H₁₅ |
| 3675, | F | F | H | CH₃ |
| 3676, | F | F | H | C₂H₅ |
| 3677, | F | F | H | n-C₃H₇ |
| 3678, | F | F | H | n-C₄H₉ |
| 3679, | F | F | H | n-C₅H₁₁ |
| 3680, | F | F | H | n-C₆H₁₃ |
| 3681, | F | F | H | n-C₇H₁₅ |
| 3682, | CF₃ | H | H | CH₃ |
| 3683, | CF₃ | H | H | C₂H₅ |
| 3684, | CF₃ | H | H | n-C₃H₇ |
| 3685, | CF₃ | H | H | n-C₄H₉ |
| 3686, | CF₃ | H | H | n-C₅H₁₁ |
| 3687, | CF₃ | H | H | n-C₆H₁₃ |
| 3688, | CF₃ | H | H | n-C₇H₁₅ |
| 3689, | H | CF₃ | H | CH₃ |
| 3690, | H | CF₃ | H | C₂H₅ |
| 3691, | H | CF₃ | H | n-C₃H₇ |
| 3692, | H | CF₃ | H | n-C₄H₉ |
| 3693, | H | CF₃ | H | n-C₅H₁₁ |
| 3694, | H | CF₃ | H | n-C₆H₁₃ |
| 3695, | H | CF₃ | H | n-C₇H₁₅ |
| 3696, | -OCF₃ | H | H | CH₃ |
| 3697, | -OCF₃ | H | H | C₂H₅ |
| 3698, | -OCF₃ | H | H | n-C₃H₇ |
| 3699, | -OCF₃ | H | H | n-C₄H₉ |
| 3700, | -OCF₃ | H | H | n-C₅H₁₁ |
| 3701, | -OCF₃ | H | H | n-C₆H₁₃ |
| 3702, | -OCF₃ | H | H | n-C₇H₁₅ |
| 3703, | H | -OCF₃ | H | CH₃ |
| 3704, | H | -OCF₃ | H | C₂H₅ |
| 3705, | H | -OCF₃ | H | n-C₃H₇ |
| 3706, | H | -OCF₃ | H | n-C₄H₉ |
| 3707, | H | -OCF₃ | H | n-C₅H₁₁ |
| 3708, | H | -OCF₃ | H | n-C₆H₁₃ |
| 3709, | H | -OCF₃ | H | n-C₇H₁₅ |
| 3710, | H | H | CH₃ | CH₃ |
| 3711, | H | H | CH₃ | C₂H₅ |
| 3712, | H | H | CH₃ | n-C₃H₇ |
| 3713, | H | H | CH₃ | n-C₄H₉ |
| 3714, | H | H | CH₃ | n-C₅H₁₁ |
| 3715, | H | H | CH₃ | n-C₆H₁₃ |
| 3716, | H | H | CH₃ | n-C₇H₁₅ |
| 3717, | F | H | CH₃ | CH₃ |
| 3718, | F | H | CH₃ | C₂H₅ |
| 3719, | F | H | CH₃ | n-C₃H₇ |
| 3720, | F | H | CH₃ | n-C₄H₉ |
| 3721, | F | H | CH₃ | n-C₅H₁₁ |
| 3722, | F | H | CH₃ | n-C₆H₁₃ |
| 3723, | F | H | CH₃ | n-C₇H₁₆ |
| 3724, | F | F | CH₃ | CH₃ |
| 3725, | F | F | CH₃ | C₂H₅ |
| 3726, | F | F | CH₃ | n-C₃H₇ |
| 3727, | F | F | CH₃ | n-C₄H₉ |
| 3728, | F | F | CH₃ | n-C₅H₁₁ |
| 3729, | F | F | CH₃ | n-C₆H₁₃ |
| 3730, | F | F | CH₃ | n-C₇H₁₅ |
| 3731, | CF₃ | H | CH₃ | CH₃ |
| 3732, | CF₃ | H | CH₃ | C₂H₅ |
| 3733, | CF₃ | H | CH₃ | n-C₃H₇ |
| 3734, | CF₃ | H | CH₃ | n-C₄H₉ |
| 3735, | CF₃ | H | CH₃ | n-C₅H₁₁ |
| 3736, | CF₃ | H | CH₃ | n-C₆H₁₃ |
| 3737, | CF₃ | H | CH₃ | n-C₇H₁₅ |
| 3738, | -OCF₃ | H | CH₃ | CH₃ |
| 3739, | -OCF₃ | H | CH₃ | C₂H₅ |
| 3740, | -OCF₃ | H | CH₃ | n-C₃H₇ |
| 3741, | -OCF₃ | H | CH₃ | n-C₄H₉ |
| 3742, | -OCF₃ | H | CH₃ | n-C₅H₁₁ |
| 3743, | -OCF₃ | H | CH₃ | n-C₆H₁₃ |
| 3744, | -OCF₃ | H | CH₃ | n-C₇H₁₅ |
| 3745, | H | H | C₂H₅ | C₂H₅ |
| 3746, | H | H | C₂H₅ | n-C₃H₇ |
| 3747, | H | H | C₂H₅ | n-C₄H₉ |
| 3748, | H | H | C₂H₅ | n-C₅H₁₁ |
| 3749, | H | H | C₂H₅ | n-C₆H₁₃ |
| 3750, | H | H | C₂H₅ | n-C₇H₁₅ |
| 3751, | F | H | C₂H₅ | CH₃ |
| 3752, | F | H | C₂H₅ | C₂H₅ |
| 3753, | F | H | C₂H₅ | n-C₃H₇ |
| 3754, | F | H | C₂H₅ | n-C₄H₉ |
| 3755, | F | H | C₂H₅ | n-C₅H₁₁ |
| 3756, | F | H | C₂H₅ | n-C₆H₁₃ |
| 3757, | F | H | C₂H₅ | n-C₇H₁₅ |
| 3758, | F | F | C₂H₅ | C₂H₅ |
| 3759, | F | F | C₂H₅ | n-C₃H₇ |
| 3760, | F | F | C₂H₅ | n-C₄H₉ |
| 3761, | F | F | C₂H₅ | n-C₅H₁₁ |
| 3762, | F | F | C₂H₅ | n-C₆H₁₃ |
| 3763, | F | F | C₂H₅ | n-C₇H₁₅ |
| 3764, | CF₃ | H | C₂H₅ | CH₃ |
| 3765, | CF₃ | H | C₂H₅ | C₂H₅ |
| 3766, | CF₃ | H | C₂H₅ | n-C₃H₇ |
| 3767, | CF₃ | H | C₂H₅ | n-C₄H₉ |
| 3768, | CF₃ | -H | C₂H₅ | n-C₅H₁₁ |
| 3769, | CF₃ | H | C₂H₅ | n-C₆H₁₃ |
| 3770, | CF₃ | H | C₂H₅ | n-C₇H₁₅ |
| 3771, | -OCF₃ | H | C₂H₅ | CH₃ |
| 3772, | -OCF₃ | H | C₂H₅ | C₂H₅ |
| 3773, | -OCF₃ | H | C₂H₅ | n-C₃H₇ |
| 3774, | -OCF₃ | H | C₂H₅ | n-C₄H₉ |
| 3775, | -OCF₃ | H | C₂H₅ | n-C₅H₁₁ |
| 3776, | -OCF₃ | H | C₂H₅ | n-C₆H₁₃ |
| 3777, | -OCF₃ | H | C₂H₅ | n-C₇H₁₅ |
| 3778, | H | H | n-C₃H₇ | n-C₃H₇ |
| 3779, | H | H | n-C₃H₇ | n-C₄H₉ |
| 3780, | H | H | n-C₃H₇ | n-C₅H₁₁ |
| 3781, | H | H | n-C₃H₇ | n-C₆H₁₃ |
| 3782, | H | H | n-C₃H₇ | n-C₇H₁₅ |
| 3783, | F | H | n-C₃H₇ | CH₃ |
| 3784, | F | H | n-C₃H₇ | C₂H₅ |
| 3785, | F | H | n-C₃H₇ | n-C₃H₇ |
| 3786, | F | H | n-C₃H₇ | n-C₄H₉ |
| 3787, | F | H | n-C₃H₇ | n-C₅H₁₁ |
| 3788, | F | H | n-C₃H₇ | n-C₆H₁₃ |
| 3789, | F | H | n-C₃H₇ | n-C₇H₁₅ |
| 3790, | F | F | n-C₃H₇ | n-C₃H₇ |
| 3791, | F | F | n-C₃H₇ | n-C₄H₉ |
| 3792, | F | F | n-C₃H₇ | n-C₅H₁₁ |
| 3793, | F | F | n-C₃H₇ | n-C₆H₁₃ |
| 3794, | F | F | n-C₃H₇ | n-C₇H₁₅ |
| 3795, | CF₃ | H | n-C₃H₇ | CH₃ |
| 3796, | CF₃ | H | n-C₃H₇ | C₂H₅ |
| 3797, | CF₃ | H | n-C₃H₇ | n-C₃H₇ |
| 3798, | CF₃ | H | n-C₃H₇ | n-C₄H₉ |
| 3799, | CF₃ | H | n-C₃H₇ | n-C₅H₁₁ |
| 3800, | CF₃ | H | n-C₃H₇ | n-C₆H₁₃ |
| 3801, | CF₃ | H | n-C₃H₇ | n-C₇H₁₅ |
| 3802, | -OCF₃ | H | n-C₃H₇ | CH₃ |
| 3803, | -OCF₃ | H | n-C₃H₇ | C₂H₅ |
| 3804, | -OCF₃ | H | n-C₃H₇ | n-C₃H₇ |
| 3805, | -OCF₃ | H | n-C₃H₇ | n-C₄H₉ |
| 3806, | -OCF₃ | H | n-C₃H₇ | n-C₅H₁₁ |
| 3807, | -OCF₃ | H | n-C₃H₇ | n-C₆H₁₃ |
| 3808, | -OCF₃ | H | n-C₃H₇ | n-C₇H₁₅ |
| 3809, | H | H | n-C₄H₉ | n-C₄H₉ |
| 3810, | H | H | n-C₄H₉ | n-C₆H₁₁ |
| 3811, | H | H | n-C₄H₉ | n-C₆H₁₃ |
| 3812, | H | H | n-C₄H₉ | n-C₇H₁₅ |
| 3813, | F | H | n-C₄H₉ | CH₃ |
| 3814, | F | H | n-C₄H₉ | C₂H₅ |
| 3815, | F | H | n-C₄H₉ | n-C₃H₇ |
| 3816, | F | H | n-C₄H₉ | n-C₄H₉ |
| 3817, | F | H | n-C₄H₉ | n-C₅H₁₁ |
| 3818, | F | H | n-C₄H₉ | n-C₆H₁₃ |
| 3819, | F | H | n-C₄H₉ | n-C₇H₁₅ |
| 3820, | F | F | n-C₄H₉ | n-C₄H₉ |
| 3821, | F | F | n-C₄H₉ | n-C₅H₁₁ |
| 3822, | F | F | n-C₄H₉ | n-C₆H₁₃ |
| 3823, | F | F | n-C₄H₉ | n-C₇H₁₅ |
| 3824, | CF₃ | H | n-C₄H₉ | CH₃ |
| 3825, | CF₃ | H | n-C₄H₉ | C₂H₅ |
| 3826, | CF₃ | H | n-C₄H₉ | n-C₃H₇ |
| 3827, | CF₃ | H | n-C₄H₉ | n-C₄H₉ |
| 3828, | CF₃ | H | n-C₄H₉ | n-C₅H₁₁ |
| 3829, | CF₃ | H | n-C₄H₉ | n-C₆H₁₃ |
| 3830, | CF₃ | H | n-C₄H₉ | n-C₇H₁₅ |
| 3831, | -OCF₃ | H | n-C₄H₉ | CH₃ |
| 3832, | -OCF₃ | H | n-C₄H₉ | C₂H₅ |
| 3833, | -OCF₃ | H | n-C₄H₉ | n-C₃H₇ |
| 3834, | -OCF₃ | H | n-C₄H₉ | n-C₄H₉ |
| 3835, | -OCF₃ | H | n-C₄H₉ | n-C₅H₁₁ |
| 3836, | -OCF₃ | H | n-C₄H₉ | n-C₈H₁₃ |
| 3837, | -OCF₃ | H | n-C₄H₉ | n-C₇H₁₅ |
| 3838, | H | H | n-C₅H₁₁ | n-C₅H₁₁ |
| 3839, | H | H | n-C₅H₁₁ | n-C₆H₁₃ |
| 3840, | H | H | n-C₅H₁₁ | n-C₇H₁₅ |
| 3841, | F | H | n-C₅H₁₁ | CH₃ |
| 3842, | F | H | n-C₅H₁₁ | C₂H₅ |
| 3843, | F | H | n-C₅H₁₁ | n-C₃H₇ |
| 3844, | F | H | n-C₅H₁₁ | n-C₄H₉ |
| 3845, | F | H | n-C₅H₁₁ | n-C₅H₁₁ |
| 3846, | F | H | n-C₅H₁₁ | n-C₆H₁₃ |
| 3847, | F | H | n-C₅H₁₁ | n-C₇H₁₅ |
| 3848, | F | F | n-C₅H₁₁ | n-C₅H₁₁ |
| 3849, | F | F | n-C₅H₁₁ | n-C₆H₁₃ |
| 3850, | F | F | n-C₅H₁₁ | n-C₇H₁₅ |
| 3851, | CF₃ | H | n-C₅H₁₁ | CH₃ |
| 3852, | CF₃ | H | n-C₅H₁₁ | C₂H₅ |
| 3853, | CF₃ | H | n-C₅H₁₁ | n-C₃H₇ |
| 3854, | CF₃ | H | n-C₅H₁₁ | n-C₄H₉ |
| 3855, | CF₃ | H | n-C₅H₁₁ | n-C₅H₁₁ |
| 3856, | CF₃ | H | n-C₅H₁₁ | n-C₆H₁₃ |
| 3857, | CF₃ | H | n-C₅H₁₁ | n-C₇H₁₅ |
| 3858, | -OCF₃ | H | n-C₅H₁₁ | CH₃ |
| 3859, | -OCF₃ | H | n-C₅H₁₁ | C₂H₅ |
| 3860, | -OCF₃ | H | n-C₅H₁₁ | n-C₃H₇ |
| 3861, | -OCF₃ | H | n-C₅H₁₁ | n-C₄H₉ |
| 3862, | -OCF₃ | H | n-C₅H₁₁ | n-C₅H₁₁ |
| 3863, | -OCF₃ | H | n-C₅H₁₁ | n-C₆H₁₃ |
| 3864, | -OCF₃ | H | n-C₅H₁₁ | n-C₇H₁₅ |
| 3865, | H | H | n-C₆H₁₃ | n-C₆H₁₃ |
| 3866, | H | H | n-C₆H₁₃ | n-C₇H₁₅ |
| 3867, | F | H | n-C₆H₁₃ | CH₃ |
| 3868, | F | H | n-C₆H₁₃ | C₂H₅ |
| 3869, | F | H | n-C₆H₁₃ | n-C₃H₇ |
| 3870, | F | H | n-C₆H₁₃ | n-C₄H₉ |
| 3871, | F | H | n-C₆H₁₃ | n-C₅H₁₁ |
| 3872, | F | H | n-C₆H₁₃ | n-C₆H₁₃ |
| 3873, | F | H | n-C₆H₁₃ | n-C₇H₁₅ |
| 3874, | F | F | n-C₆H₁₃ | n-C₆H₁₃ |
| 3875, | F | F | n-C₆H₁₃ | n-C₇H₁₅ |
| 3876, | CF₃ | H | n-C₆H₁₃ | CH₃ |
| 3877, | CF₃ | H | n-C₆H₁₃ | C₂H₅ |
| 3878, | CF₃ | H | n-C₆H₁₃ | n-C₃H₇ |
| 3879, | CF₃ | H | n-C₆H₁₃ | n-C₄H₉ |
| 3880, | CF₃ | H | n-C₆H₁₃ | n-C₅H₁₁ |
| 3881, | CF₃ | H | n-C₆H₁₃ | n-C₆H₁₃ |
| 3882, | CF₃ | H | n-C₆H₁₃ | n-C₇H₁₅ |
| 3883, | -OCF₃ | H | n-C₆H₁₃ | CH₃ |
| 3884, | -OCF₃ | H | n-C₆H₁₃ | C₂H₅ |
| 3885, | -OCF₃ | H | n-C₆H₁₃ | n-C₃H₇ |
| 3886, | -OCF₃ | H | n-C₆H₁₃ | n-C₄H₉ |
| 3887, | -OCF₃ | H | n-C₆H₁₃ | n-C₅H₁₁ |
| 3888, | -OCF₃ | H | n-C₆H₁₃ | n-C₆H₁₃ |
| 3889, | -OCF₃ | .H | n-C₆H₁₃ | n-C₇H₁₅ |
| 3890, | H | H | n-C₇H₁₅ | n-C₇H₁₅ |
| 3891, | F | H | n-C₇H₁₅ | CH₃ |
| 3892, | F | H | n-C₇H₁₅ | C₂H₅ |
| 3893, | F | H | n-C₇H₁₅ | n-C₃H₇ |
| 3894, | F | H | n-C₇H₁₅ | n-C₄H₉ |
| 3895, | F | H | n-C₇H₁₅ | n-C₅H₁₁ |
| 3896, | F | H | n-C₇H₁₅ | n-C₆H₁₃ |
| 3897, | F | H | n-C₇H₁₅ | n-C₇H₁₅ |
| 3898, | F | F | n-C₇H₁₅ | n-C₇H₁₅ |
| 3899, | CF₃ | H | n-C₇H₁₅ | CH₃ |
| 3900, | CF₃ | H | n-C₇H₁₅ | C₂H₅ |
| 3901, | CF₃ | H | n-C₇H₁₅ | n-C₃H₇ |
| 3902, | CF₃ | H | n-C₇H₁₅ | n-C₄H₉ |
| 3903, | CF₃ | H | n-C₇H₁₅ | n-C₅H₁₁ |
| 3904, | CF₃ | H | n-C₇H₁₅ | n-C₆H₁₃ |
| 3905, | CF₃ | H | n-C₇H₁₅ | **n-C₇H₁₅** |
| 3906, | -OCF₃ | H | n-C₇H,₅ | CH₃ |
| 3907, | -OCF₃ | H | n-C₇H₁₅ | C₂H₅ |
| 3908, | -OCF₃ | H | n-C₇H₁₅ | n-C₃H₇ |
| 3909, | -OCF₃ | H | n-C₇H₁₅ | n-C₄H₉ |
| 3910, | -OCF₃ | H | n-C₇H₁₅ | n-C₅H₁₁ |
| 3911, | -OCF₃ | H | n-C₇H₁₅ | n-C₆H₁₃ |
| 3912, | -OCF₃ | H | n-C₇H₁₅ | n-CᵣH₁₅ |

**Tabelle 1**

| Δε- und Δn-Werte für Substanzen einzelner Beispiele | | |
|---|---|---|
| Beispiel Nr. | Δε | Δn |
| 73 | -10,2 | 0,006 |
| 106 | -10,3 | 0,068 |
| 137 | -10,0 | 0,069 |
| 1401 | -6,8 | 0,074 |
| 1402 | -7,4 | 0,072 |
| 2325 | -8,7 | 0,078 |
| 3726 | -4,4 | 0,087 |
| 3758 | -5,0 | 0,088 |
| 3759 | -4,5 | 0,088 |
| 3790 | -4,4 | 0,088 |

## Patentansprüche

1. Verbindung der allgemeinen Formel I: 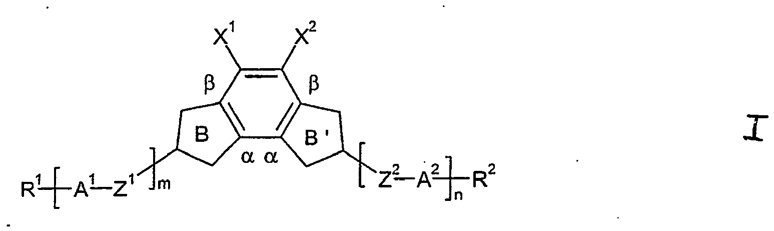 worin: 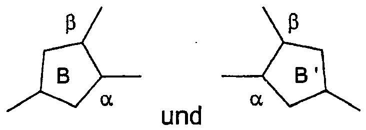 unabhängig voneinander für 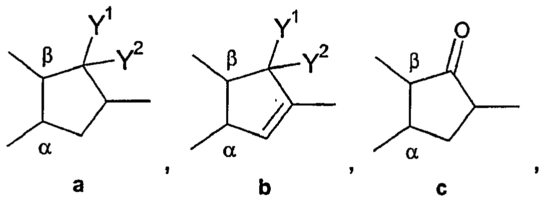 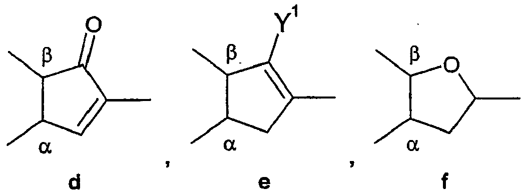 oder 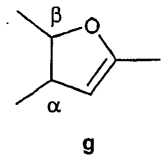 stehen;
A¹ und A² jeweils unabhängig voneinander 1,4-Phenylen, worin =CH- ein- oder zweimal durch =N- ersetzt sein kann und das ünsubstituiert oder ein- bis viermal unabhängig voneinander mit -CN, F, CI, Br und/oder I, unsubstituiertem oder mit Fluor und/oder Chlor ein- oder mehrfach substituiertem C₁-C₆-Alkanyl, unsubstituiertem oder mit Fluor und/oder Chlor ein- oder mehrfach substituiertem C₁-C₆-Alkoxy substituiert sein kann, 1,4-Cyclohexylen, 1,4-Cyclohexenylen oder 1,4-Cyclohexadienylen, worin -CH₂- ein- oder zweimal unabhängig voneinander durch -O- oder -S- so ersetzt sein kann, daß Heteroatome nicht direkt verknüpft sind, und die unsubstituiert oder ein- oder mehrfach durch F, CI, Br und/oder I substituiert sein können, bedeuten;
Z¹ und Z² jeweils unabhängig voneinander eine Einfachbindung, eine Doppelbindung, -CF₂O- -OCF₂-, -CH₂CH₂-, -CF₂CF₂-, -CF₂CH₂-,- -CH₂CF₂-, -CHF-CHF-, -C(O)O-, -OC(O)-, -CH₂O-, -OCH₂-, -CF=CH-, -CH=CF-, -CF=CF-, -CH=CH- oder -C≡Cbedeuten;
R¹ und R² Wasserstoff, einen unsubstituierten, einen einfach mit -CN oder -CF₃ oder einen einfach oder mehrfach mit F, Cl, Br und/oder I substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂- oder -OCO-O-so ersetzt sein können, daß Heteroatome in der Kette nicht direkt verknüpft sind, F, Cl, Br, I, -CN, -SCN, -NCS oder -SF₅ bedeuten;
m und n unabhängig voneinander 0, 1, 2 oder 3 sind;
Y² Wasserstoff, einen unsubstituierten oder einfach oder mehrfach durch F, Cl, Br und/oder I substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂-, -CO-, -COO-, -OCO- oder -OCO-O- so ersetzt sein können, daß Heteroatome in der Kette nicht direkt verknüpft sind, F, Cl, Br, I, -CN, -SF₅, -SCN oder -NCS bedeuten;
Y¹ wie Y² definiert ist oder für -[-Z³-A³-]ₚ-R³ steht, wobei Z³ wie Z¹ und Z², A³ wie A¹ und A², R³ wie R¹ und R² sowie p wie n und m definiert sind;
wobei
A¹, A², A³_{;} Z¹, Z², Z³, R¹, R², R³ jeweils die gleiche oder unterschiedliche Bedeutungen haben können, wenn m, n beziehungsweise p größer als 1 sind;
Y¹ und Y² für die Ringe B und B' jeweils die gleichen oder verschiedene Bedeutungen haben können;
**dadurch gekennzeichnet, dass**
X¹ und X², unabhängig voneinander F, Cl, CF₃ oder OCF₃ bedeuten.

2. Verbindung gemäß dem vorangehenden Anspruch, **dadurch gekennzeichnet, daß**
die Ringe B und B' jeweils unabhängig voneinander ausgewählt sind aus

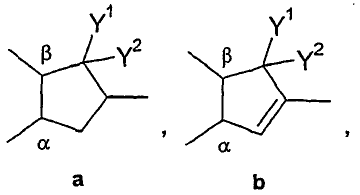

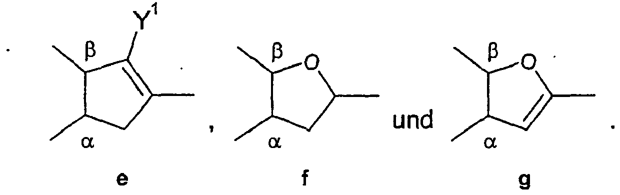

3. Verbindung gemäß wenigstens einem der vorangehenden Ansprüche, **dadurch gekennzeichnet, daß**
die Ringe B und B' gleich sind, wobei der Rest Y¹ in beiden Ringen die gleiche oder unterschiedliche Bedeutungen und der Rest Y² in beiden Ringen gleiche oder unterschiedliche Bedeutungen haben kann.

4. Verbindung gemäß wenigstens einem der vorangehenden Ansprüche, **dadurch gekennzeichnet, daß**
die Verbindung der Formel I ausgewählt ist aus der Gruppe bestehend aus Verbindungen der Formeln laa, Ibb, lee, Iff, Igg: 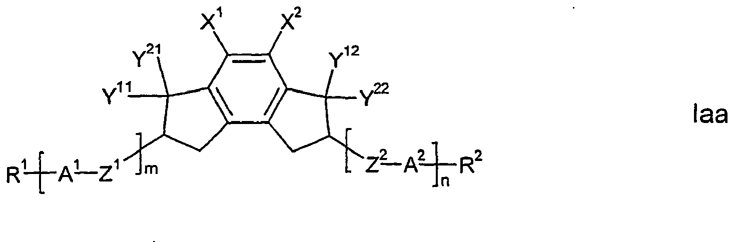 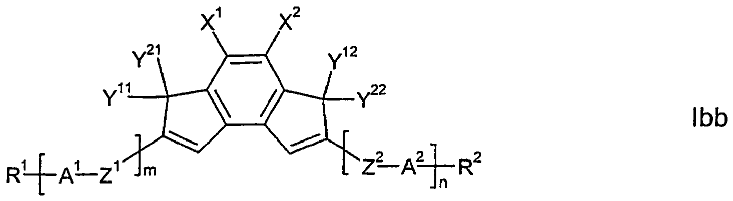 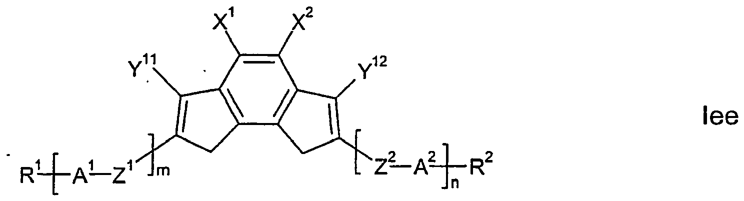 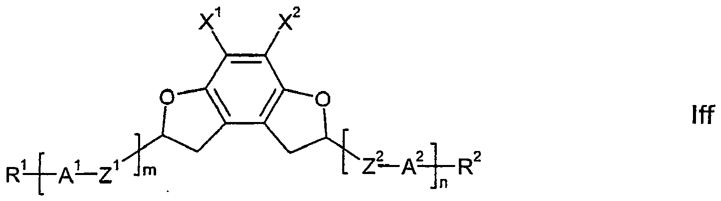 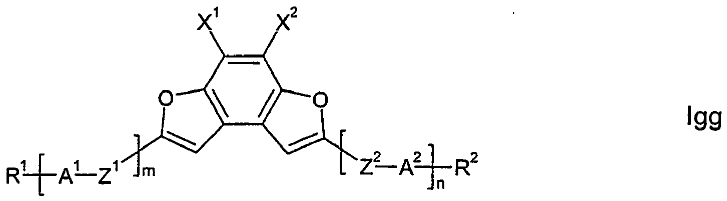 wobei
X¹, X², R¹, R², A¹, A², Z¹, Z², m und n wie in Anspruch 1 definiert sind;
Y¹¹ und Y¹² wie Y¹ in Anspruch 1 definiert sind und die gleiche oder verschiedene Bedeutungen haben können; und
Y²¹ und Y²² wie Y² in Anspruch 1 definiert sind und die gleiche oder verschiedene Bedeutungen haben können.

5. Verbindung gemäß wenigstens einem der vorangehenden Ansprüche, **dadurch gekennzeichnet, daß** Y¹ und Y² bzw. Y¹¹, Y¹², Y²¹ und Y²² gleichzeitig F sind.

6. Verbindung gemäß wenigstens einem der vorangehenden Ansprüche, **dadurch gekennzeichnet, daß**
Z¹ und Z² sowie Z³ unabhängig voneinander eine Einfachbindung, -CF₂O- -OCF₂-, -CF₂CF₂-, -CH=CH-, -CF=CH-, -CH=CF- oder -CF=CF- sind.

7. Verbindung gemäß wenigstens einem der vorangehenden Ansprüche, **dadurch gekennzeichnet, daß**
A¹ und A² sowie A³ unabhängig voneinander 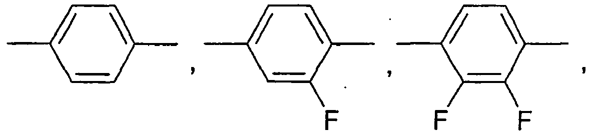 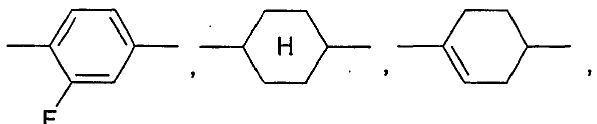 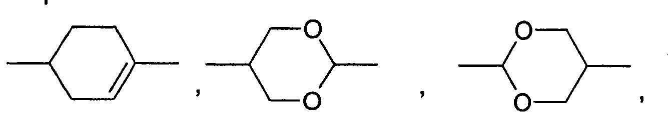 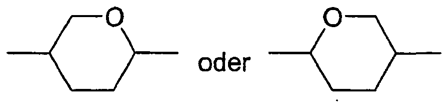
ist.

8. Verbindung gemäß wenigstens einem der vorangehenden Ansprüche, **dadurch gekennzeichnet, daß**
R¹ und R² sowie R³ unabhängig voneinander jeweils ein Alkanylrest, Alkoxyrest oder Alkenylrest mit 1 bis 7 bzw. 2 bis 7 C-Atomen sind.

9. Verbindung gemäß wenigstens einem der vorangehenden Ansprüche, **dadurch gekennzeichnet, daß**
m 0, 1 oder 2 ist;
n 0, 1 oder 2 ist;
m+n 0, 1, 2 oder 3 ist; und
p 0 ist.

10. Verbindung gemäß wenigstens einem der vorangehenden Ansprüche, **dadurch gekennzeichnet, daß**
wenigstens eines von X¹ und X² F, Cl, CF₃ oder OCF₃ ist.

11. Verbindung gemäß wenigstens einem der vorangehenden Ansprüche, **dadurch gekennzeichnet, daß**
X¹ und X² F sind.

12. Verwendung einer Verbindung gemäß wenigstens einem der vorangehenden Ansprüche in flüssigkristallinen Medien.

13. Flüssigkristallines Medium mit mindestens zwei flüssigkristallinen Verbindungen, **dadurch gekennzeichnet, daß** es mindestens eine Verbindung gemäß wenigstens einem der Ansprüche 1 bis 11 enthält.

14. Elektrooptisches Anzeigeelement, enthaltend ein flüssigkristallines Medium gemäß Anspruch 13.

## Claims

1. Compound of the general formula I: 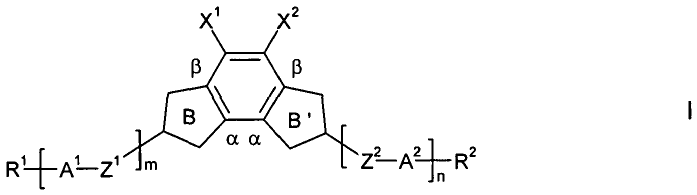 in which: 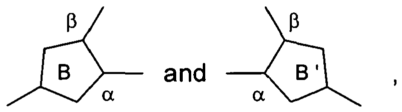 independently of one another, stand for 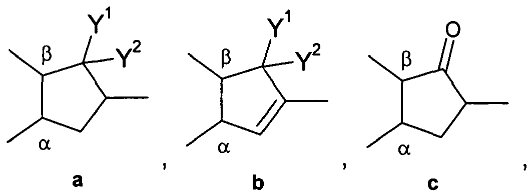 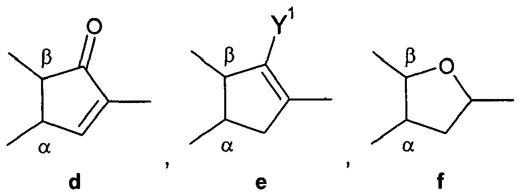 or 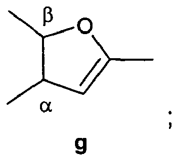
A¹ and A² each, independently of one another, denote 1,4-phenylene, in which =CH- may be replaced once or twice by =N- and which may be unsubstituted or mono- to tetrasubstituted, independently of one another, by -CN, F, Cl, Br and/or I, C₁-C₆-alkanyl which is unsubstituted or mono- or polysubstituted by fluorine and/or chlorine, C₁-C₆-alkoxy which is unsubstituted or mono- or polysubstituted by fluorine and/or chlorine, 1,4-cyclohexylene, 1,4-cyclohexenylene or 1,4-cyclohexadienylene, in which -CH₂- may be replaced once or twice, independently of one another, by -O- or -S- in such a way that heteroatoms are not linked directly and which may be unsubstituted or mono- or polysubstituted by F, Cl, Br and/or I;
Z¹ and Z² each, independently of one another, denote a single bond, a double bond, -CF₂O-, -OCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CF=CH-, -CH=CF-, -CF=CF-, -CH=CH- or -C≡C-;
R¹ and R² denote hydrogen, an alkanyl, alkoxy, alkenyl or alkynyl radical having 1 to 15 or 2 to 15 C atoms respectively, each of which is unsubstituted, monosubstituted by -CN or -CF₃ or monosubstituted or polysubstituted by F, Cl, Br and/or I, where, in addition, one or more CH₂ groups in these radicals may each be replaced, independently of one another, by -O-, -S-, -SO₂- or -OCO-O- in such a way that heteroatoms in the chain are not linked directly, F, Cl, Br, I, -CN, -SCN, -NCS or -SF₅;
m and n, independently of one another, are 0, 1, 2 or 3;
Y² denotes hydrogen, an alkanyl, alkoxy, alkenyl or alkynyl radical having 1 to 15 or 2 to 15 C atoms respectively, each of which is unsubstituted or monosubstituted or polysubstituted by F, Cl, Br and/or I, where, in addition, one or more CH₂ groups in these radicals may each be replaced, independently of one another, by -O-, -S-, -SO₂-, -CO-, -COO-, -OCO- or -OCO-O- in such a way that heteroatoms in the chain are not linked directly, F, Cl, Br, I, -CN, -SF₅, -SCN or -NCS;
Y¹ is as defined for Y² or stands for -[-Z³-A³-]ₚ-R³, where Z³ is as defined for Z¹ and Z² , A³ is as defined for A¹ and A², R³ is as defined for R¹ and R², and p is as defined for n and m;
where
A¹, A², A³, Z¹, Z², Z³, R¹, R², R³ can each have identical or different meanings if m, n and p respectively are greater than 1;
Y¹ and Y² can each have identical or different meanings for rings B and B';
**characterised in that**
X¹ and X², independently of one another, denote F, Cl, CF₃ or OCF₃.

2. Compound according to the preceding claim, **characterised in that** rings B and B' are each, independently of one another, selected from

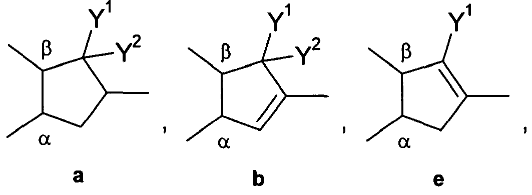

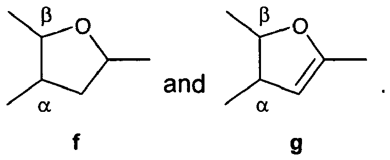

3. Compound according to at least one of the preceding claims, **characterised in that** rings B and B' are identical, where the radical Y¹ in both rings can have identical or different meanings and the radical Y² in both rings can have identical or different meanings.

4. Compound according to at least one of the preceding claims, **characterised in that** the compound of the formula I is selected from the group consisting of compounds of the formulae laa, Ibb, lee, Iff, Igg: 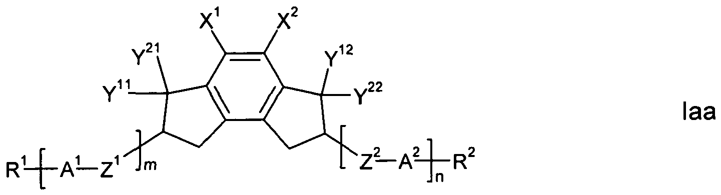 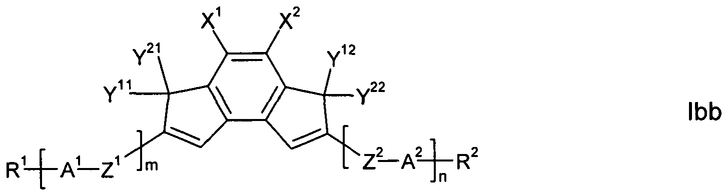 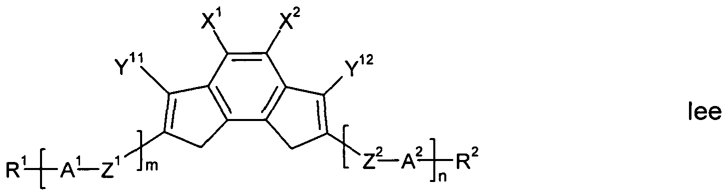 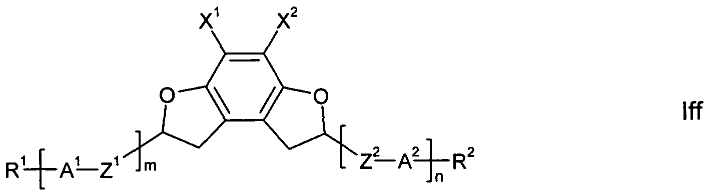 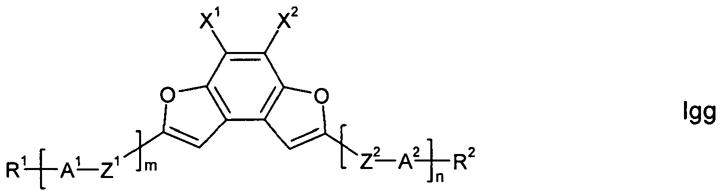 where
X¹, X², R¹, R², A¹, A², Z¹, Z², m and n are as defined in Claim 1;
Y¹¹ and Y¹² are as defined for Y¹ in Claim 1 and can have identical or different meanings; and
Y²¹ and Y²² are as defined for Y² in Claim 1 and can have identical or different meanings.

5. Compound according to at least one of the preceding claims, **characterised in that**
Y¹ and Y² or Y¹¹, Y¹², Y²¹ and Y²² are simultaneously F.

6. Compound according to at least one of the preceding claims, **characterised in that**
Z¹ and Z² as well as Z³, independently of one another, are a single bond, -CF₂O-, -OCF₂-, -CF₂CF₂-, -CH=CH-, -CF=CH-, -CH=CF- or -CF=CF-.

7. Compound according to at least one of the preceding claims, **characterised in that**
A¹ and A² as well as A³, independently of one another, are

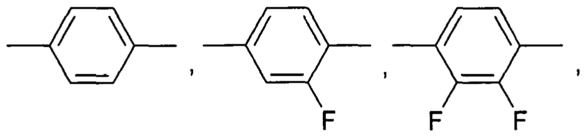

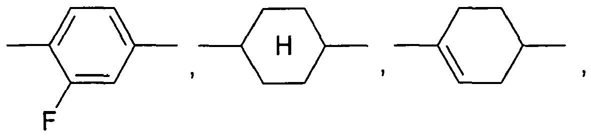

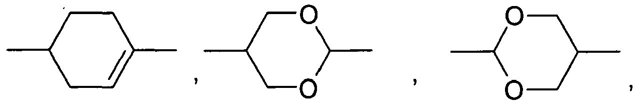

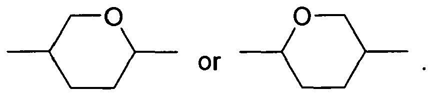

8. Compound according to at least one of the preceding claims, **characterised in that**
R¹ and R² as well as R³, independently of one another, are each an alkanyl radical, alkoxy radical or alkenyl radical having 1 to 7 or 2 to 7 C atoms respectively.

9. Compound according to at least one of the preceding claims, **characterised in that**
m is 0, 1 or 2;
n is 0, 1 or 2;
m+n is 0, 1, 2 or 3; and
p is 0.

10. Compound according to at least one of the preceding claims, **characterised in that**
at least one of X¹ and X² is F, Cl, CF₃ or OCF₃.

11. Compound according to at least one of the preceding claims, **characterised in that**
X¹ and X² are F.

12. Use of a compound according to at least one of the preceding claims in liquid-crystalline media.

13. Liquid-crystalline medium comprising at least two liquid-crystalline compounds, **characterised in that** it comprises at least one compound according to at least one of Claims 1 to 11.

14. Electro-optical display element containing a liquid-crystalline medium according to Claim 13.

## Revendications

1. Composé de la formule générale I : 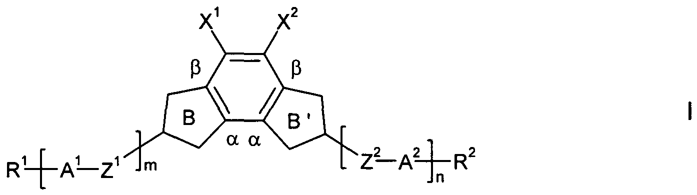 dans laquelle : 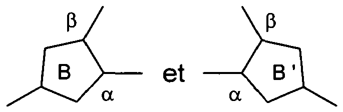 représentent, indépendamment l'un de l'autre, 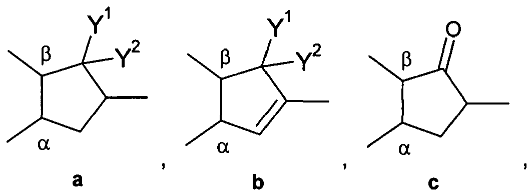 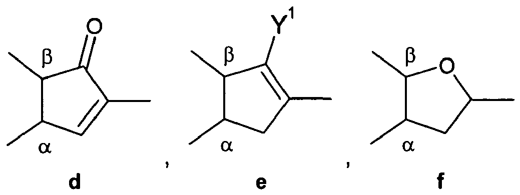 ou 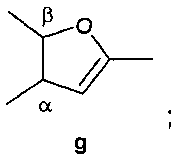
A¹ et A² représentent, chacun indépendamment de l'autre, 1,4-phénylène, où =CH- peut être remplacé une fois ou deux fois par =N- et lesquels peuvent être non substitués ou mono- à tétrasubstitués, indépendamment l'un de l'autre, par -CN, F, Cl, Br et/ou I, C₁-C₆-alkanyle, lequel est non substitué ou mono- ou polysubstitué par fluor et/ou chlore, C₁-C₆-alcoxy, lequel est non substitué ou mono- ou polysubstitué par fluor et/ou chlore, 1,4-cyclohexylène, 1,4-cyclohexénylène ou 1,4-cyclohexadiénylène, où -CH₂- peut être remplacé une fois ou deux fois, indépendamment l'une de l'autre, par -O- ou -S- de telle sorte que des hétéroatomes ne soient pas liés directement et lequel peut être non substitué ou mono- ou polysubstitué par F, Cl, Br et/ou I ;
Z¹ et Z² représentent, chacun indépendamment l'un de l'autre, une liaison simple, une liaison double, -CF₂O- -OCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CF=CH-, -CH=CF-, -CF=CF-, -CH=CH- ou -C≡C- ;
R¹ et R² représentent hydrogène, un radical alkanyle, alcoxy, alkényle ou alkynyle comportant respectivement de 1 à 15 ou de 2 à 15 atomes de C, dont chacun est non substitué, monosubstitué par -CN ou -CF₃ ou monosubstitué ou polysubstitué par F, Cl, Br et/ou I, où, en outre, un ou plusieurs groupes CH₂ dans ces radicaux peuvent chacun être remplacés, indépendamment les uns des autres, par -O-, -S-, -SO₂- ou -OCO-O- de telle sorte que des hétéroatomes dans la chaîne ne soient pas liés directement, F, Cl, Br, I, -CN, -SCN, -NCS ou -SF₅ ;
m et n, sont, indépendamment l'un de l'autre, 0, 1, 2 ou 3 ;
Y² représente hydrogène, un radical alkanyle, alcoxy, alkényle ou alkynyle comportant respectivement de 1 à 15 ou de 2 à 15 atomes de C, dont chacun est non substitué ou monosubstitué ou polysubstitué par F, Cl, Br et/ou I, où, en outre, un ou plusieurs groupes CH₂ dans ces radicaux peuvent être remplacés, indépendamment les uns des autres, par -O-, Y¹ -S-, -SO₂-, -CO-, -COO-, -OCO- ou -OCO-O- de telle sorte que des hétéroatomes dans la chaîne ne soient pas liés directement, F, CI, Br, I, -CN, -SF₅, -SCN ou -NCS ; est comme défini pour Y² ou représente -[-Z³-A³-]ₚ-R³, où Z³ est comme défini pour Z¹ et Z², A³ est comme défini pour A¹ et A², R³ est comme défini pour R¹ et R², et p est comme défini pour n et m ;
où
A¹, A², A³, A¹, Z², Z³, R¹, R², R³ peuvent chacun présenter des significations identiques ou différentes si m, n et p sont respectivement supérieurs à 1 ;
Y¹ et Y² peuvent chacun présenter des significations identiques ou différentes pour des cycles B et B' ;
**caractérisé en ce que**
X¹ et X² représentent, indépendamment l'un de l'autre, F, Cl, CF₃ ou OCF₃.

2. Composé selon la revendication précédente, **caractérisé en ce que** des cycles B et B' sont chacun, indépendamment l'un de l'autre, choisi parmi

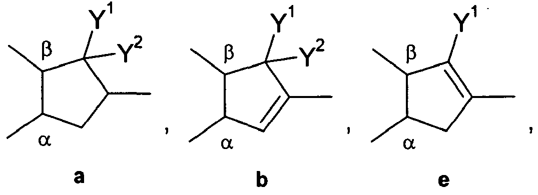

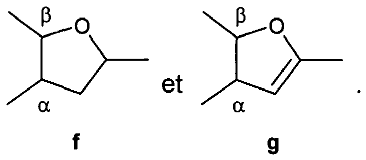

3. Composé selon au moins l'une des revendications précédentes, **caractérisé en ce que** des cycles B et B' sont identiques, où le radical Y¹ dans les deux cycles peut présenter des significations identiques ou différentes et le radical Y² dans les deux cycles peut présenter des significations identiques ou différentes.

4. Composé selon au moins l'une des revendications précédentes, **caractérisé en ce que** le composé de la formule I est choisi parmi le groupe constitué par les composés des formules laa, Ibb, lee, Iff, Igg 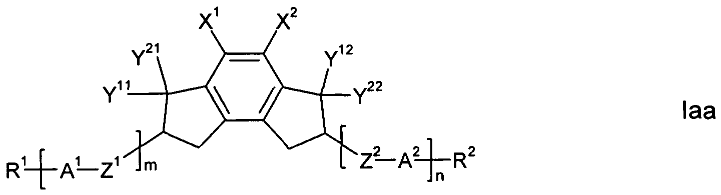 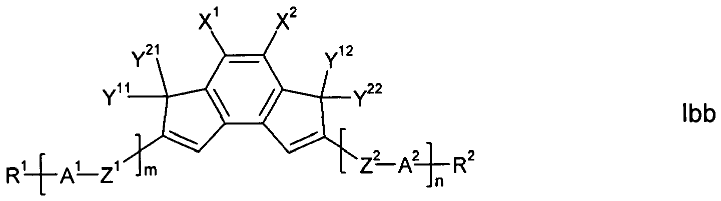 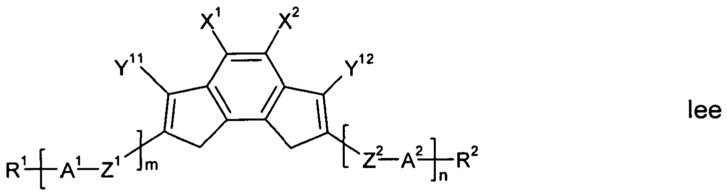 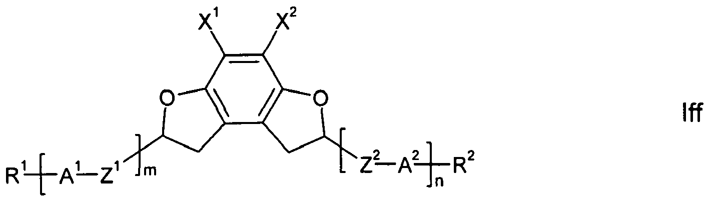 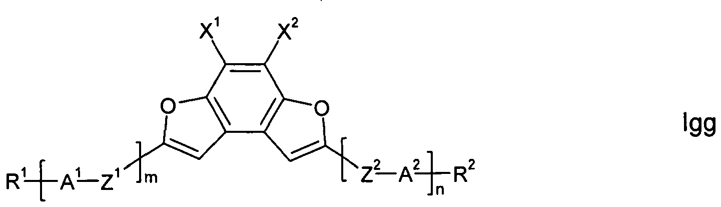 dans lesquelles
X¹, X², R¹, R², A¹, A², Z¹, Z², m et n sont comme défini selon la revendication 1 ;
Y¹¹ et Y¹² sont comme défini pour Y¹ selon la revendication 1 et peuvent présenter des significations identiques ou différentes ; et
Y²¹ et Y²² sont comme défini pour Y² selon la revendication 1 et peuvent présenter des significations identiques ou différentes.

5. Composé selon au moins l'une des revendications précédentes, **caractérisé en ce que**
Y¹ et Y² ou Y¹¹, Y¹², Y²¹ et Y²² sont simultanément F.

6. Composé selon au moins l'une des revendications précédentes, **caractérisé en ce que**
Z¹ et Z² de même que Z³ sont, indépendamment les uns des autres, une liaison simple, -CF₂O-, -OCF₂-, -CF₂CF₂-, -CH=CH-, -CF=CH-, -CH=CF- ou -CF=CF-.

7. Composé selon au moins l'une des revendications précédentes, **caractérisé en ce que**
A¹ et A² de même que A³ sont, indépendamment les uns des autres,

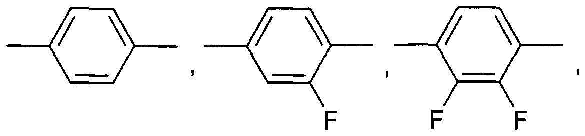

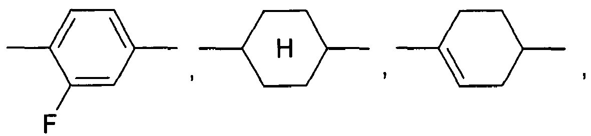

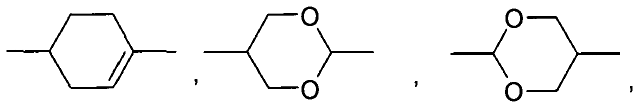

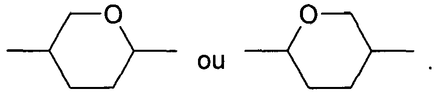

8. Composé selon au moins l'une des revendications précédentes, **caractérisé en ce que**
R¹ et R² de même que R³ sont chacun, indépendamment les uns des autres, un radical alkanyle, un radical alcoxy ou un radical alkényle comportant respectivement de 1 à 7 ou de 2 à 7 atomes de C.

9. Composé selon au moins l'une des revendications précédentes, **caractérisé en ce que**
| | |
|---|---|
| m | est 0, 1 ou 2 ; |
| n | est 0, 1 ou 2 ; |
| m+n | est 0, 1, 2 ou 3 ; et |
| p | est 0. |

10. Composé selon au moins l'une des revendications précédentes, **caractérisé en ce que**
au moins l'un de X¹ et X² est F, Cl, CF₃ ou OCF₃.

11. Composé selon au moins l'une des revendications précédentes, **caractérisé en ce que**
X¹ et X² sont F.

12. Utilisation d'un composé selon au moins l'une des revendications précédentes dans des milieux cristallins liquides.

13. Milieu cristallin liquide comprenant au moins deux composés cristallins liquides, **caractérisé en ce qu'**il comprend au moins un composé selon au moins l'une des revendications 1 à 11.

14. Elément d'affichage électro-optique contenant un milieu cristallin liquide selon la revendication 13.
